# EUROPEAN PATENT SPECIFICATION

(11) **EP 4 138 881 B1**
(45) Date of publication and mention of the grant of the patent: **13.11.2024**
(21) Application number: 21725964.7
(22) Date of filing: 23.04.2021
(51) Int. Cl.: A61K 38/46, A61P 37/04

(54) **COMPOSITION COMPRISING DNASE I FOR USE IN A METHOD OF PREVENTING THE ACTIVATION OF THE AIM2 INFLAMMASOME**
DNASE I ENTHALTENDE ZUSAMMENSETZUNG ZUR VERWENDUNG IN EINEM VERFAHREN ZUR VERHINDERUNG DER AKTIVIERUNG DES AIM2-INFLAMMASOMS
COMPOSITION COMPRENANT DE LA DNASE I À UTILISER DANS UN PROCÉDÉ POUR LA PRÉVENTION DE L'ACTIVATION DE L'INFLAMMASOME AIM2

(30) Priority: 24.04.2020 EP 20171271
(43) Date of publication of application: 01.03.2023
(73) Proprietor: Klinikum der Universität München (KUM), 81377 München (DE)
(72) Inventor: LIESZ, Arthur, 86929 Penzing (DE); ROTH, Stefan, 82284 Grafrath (DE)
(74) Representative: Schiweck Weinzierl Koch Patentanwälte Partnerschaft mbB
(86) International application number: PCT/EP2021/060664
(87) International publication number: WO 2021/214293

(56) References cited:
- WO-A1-2014/160284
- WO-A1-2015/104656
- US-A1- 2017 100 463
- HAEUSLER KARL GEORG ET AL: "Immune responses after acute ischemic stroke or myocardial infarction", INTERNATIONAL JOURNAL OF CARDIOLOGY, vol. 155, no. 3, 13 November 2010 (2010-11-13), pages 372 - 377, XP028899047, ISSN: 0167-5273, DOI: 10.1016/J.IJCARD.2010.10.053
- BARLOW ET AL: "T lymphocytes and immunosuppression in the burned patient: a review", BURNS, BUTTERWORTH HEINEMANN, GB, vol. 20, no. 6, 1 December 1994 (1994-12-01), pages 487 - 490, XP027254892, ISSN: 0305-4179, [retrieved on 19941201]

## Description

### TECHNICAL FIELD OF THE INVENTION

The present invention is in the field of treatment of immunosuppression, in particular in the field of treatment of post sterile traumatic immunosuppression, more particularly wherein said immunosuppression occurs after an immunoactivation. Particularly, the present invention relates to a composition comprising a DNA-degrading enzyme for use in a method of reducing the activation of a cell-free (cf) DNA sensing AIM2-inflammasome in myeloid cells of a subject in need thereof, wherein said DNA-degrading enzyme is DNAse I. Further, the present invention relates to the composition for the use of the present invention, wherein the DNAse I is administered after acute tissue injury and/or in the course of the treatment of acute tissue injury.

The invention is defined by the appended claims.

### BACKGROUND ART

Acute tissue injuries such as stroke (Vogelgesang *et al.*, 2008), myocardial infarction (Kohsaka *et al.*, 2005) and burn injury (Xu *et al.*, 2016) induce both local and systemic inflammatory responses. These immune perturbations are characterized by an acute proinflammatory response, followed by an immunosuppressive phase, which can be acute and which predisposes patients to infections. Secondary immune-mediated complications such as acute cytokine-induced comorbidities and infections reportedly cause more patient deaths than the primary injury (Dantzer *et al.,* 2008; Vermeij *et al.,* 2009; D'Avignon *et al.,* 2010). The actual mediators and underlying mechanism of the brain-immune communication are so far unknown.

For example, De Meyer et al. (Arterioscler. Thromb. Vase. Biol., 2012) describes extracellular chromatin as an important mediator of ischemic stroke in mice.

McIlroy et al. (J. Trauma Acute Care Surg., 2018) suggests that reduced deoxyribonuclease enzyme activity might provide a therapeutic target for Systemic Inflammatory Response Syndrome.

Though it has been demonstrated that soluble mediators derived from the brain are responsible for the development and progression of the systemic immune response after stroke (Roth *et al.*, 2018), wherein similar findings have been reported for burns and traumatic injuries (Hazeldine *et al.,* 2015; Manson *et al.,* 2012), the identity of these mediators as well as the mechanism linking acute immune activation and subsequent immunosuppression were unclear so far.

Consequently, the inventors of the present invention have established possibilities for treating a new clinical scenario, namely reducing the activation of a cf DNA sensing AIM2-inflammasome in myeloid cells of a subject in need thereof. It was not able to provide a composition for the use of the treatment thereof so far as the causal reasons, namely how the immunosuppression after acute tissue injury was triggered, where unidentified. Those have not been known so far in the prior art.

Thus, there has been a drastical need to provide such a composition for the use in the treatment for the described clinical scenario.

### SUMMARY OF THE INVENTION

The present invention is defined in the claims.

According to the present invention, the inventors have found that a DNA-degrading enzyme, for example an enzyme which degrades nuclear DNA and possibly additionally mtDNA, specifically DNase I, can be used in a method of reducing the activation of a cf DNA sensing AIM2-inflammasome in myeloid cells of a subject in need thereof. Disclosed herein is also the treatment of the clinical scenario post sterile traumatic immunosuppression. In other words, the inventors demonstrate that different tissue injuries induce a uniform and systemic activation of the inflammasome by sensing cell-free nucleic acids released from injured tissues. In this context, the inflammasome is a multi-protein complex in peripheral monocytes which accumulates and orchestrates caspase-1 cleavage upon activation of a wide range of danger signals sensed by the inflammasome. Inflammasome activation is primarily described as an innate response to bacterial and viral non-self molecules. Yet, the inventors observed that cell-free self-DNA activates the inflammasome in peripheral monocytes via the nucleic acid-sensing AIM2-inflammasome. In other words, it was shown that the inflammasome was triggered by AIM2 in myeloid cells, which sense cell-free DNA released after an acute tissue injury / damage. It was further demonstrated that monocytic inflammasome activation then drives overexpression of FasL on monocytes, subsequently inducing caspase-8-dependent apoptosis in T cells. The induction of FasL-expressing monocytes and preferably consecutive lymphopenia is in a more detailed embodiment of the invention driven by inflammasome-dependent IL-1 secretion. Consequently, the inventors provide a mechanistic understanding for a common, yet thus far elusive, clinical observation: the biphasic systemic immune response to sterile tissue injuries. With these findings the inventors provide further studies involving novel therapeutic strategies against post-injury immune alterations, thereby preventing the medical burden of inflammatory comorbidities in a wide range of acute tissue injuries. In sum, it has been demonstrated that inflammasome-dependent monocyte activation is the cause of T cell death after injury, and challenges the current paradigms of post-injury lymphopenia. Thus, the present invention provides new therapeutic targets for the pathway identified here along the events of increased cf-dsDNA concentration after acute tissue injury, inflammasome activation, IL-1β secretion, and Fas-mediated T cell death. This reduces the medical burden of postinjury immunosuppression and secondary infections. For the majority of the experiments in the present invention an experimental stroke model as a prototypic tissue injury model was applied. Key findings from the stroke model were also generalizable to a second tissue injury model of burn lesions. Thus, the extension to other tissue injuries / damages has been plausibly presented by the inventors.

Thus, the present invention provides a composition comprising a DNAse I for use in a method of reducing the activation of a cf DNA sensing AIM2-inflammasome in myeloid cells of a subject in need thereof.

Disclosed herein is also a composition comprising a DNAse-degrading enzyme for use in a method for the treatment of post sterile traumatic immunosuppression.

Also disclosed herein is the composition for such disclosed use, wherein an immunoactivation occurs before the immunosuppression.

Also disclosed herein is the composition for such disclosed use, wherein the post sterile traumatic immunosuppression is characterized by an early systemic immune response syndrome and subsequent lymphocyte death.

Also disclosed herein is the composition for such disclosed use, wherein the lymphocyte death is caused by apoptosis.

Also disclosed herein is the composition for such disclosed use, wherein the post sterile traumatic immunosuppression is associated with systemic immune response syndrome (SIRS).

Also disclosed herein is the composition for such disclosed use, wherein the post sterile traumatic immunosuppression is triggered by acute tissue injury.

Also disclosed herein is the composition for such disclosed use, wherein the acute tissue injury is triggered by a physical, chemical, or metabolic noxious stimulus.

Also disclosed herein is the composition for such disclosed use, wherein the acute tissue injury is selected from stroke, myocardial infection, haemorrhagic shock, ischemia, ischemia reperfusion injury, chronic inhalation of irritants (e.g. asbestos, silica), atherosclerosis, gout, pseudogout, trauma, non-penetrating polytrauma (multiple bone fractures), and thermal trauma.

Also disclosed herein is the composition for such disclosed use, wherein the post sterile traumatic immunosuppression is associated with a secondary infectious disease.

Also disclosed herein is the composition for such disclosed use, wherein the DNA-degrading enzyme is a nuclease. Also disclosed herein is the composition for such disclosed use, wherein the nuclease is an exonuclease or endonuclease. Also disclosed herein is the composition for such disclosed use, wherein the endonuclease is a deoxyribonuclease. Also disclosed herein is the composition for such disclosed use, wherein the deoxyribonuclease is DNAse !.

In one further embodiment of the composition for the use of the present invention, the DNAse I is administered after the acute tissue injury and/or in the course of the treatment of the acute tissue injury.

According to one further embodiment of the composition for the use of the present invention, the DNAse I is administered parenterally, preferably intravenously or by inhalation.

### BRIEF DESCRIPTION OF THE FIGURES

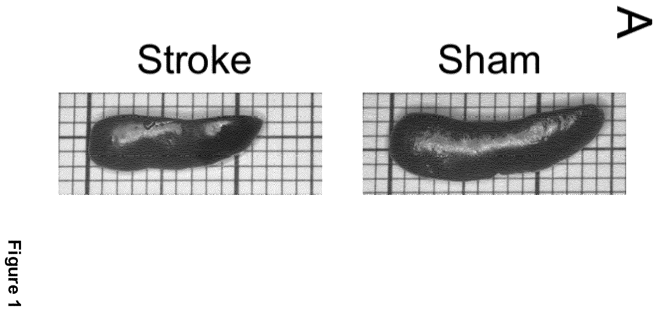

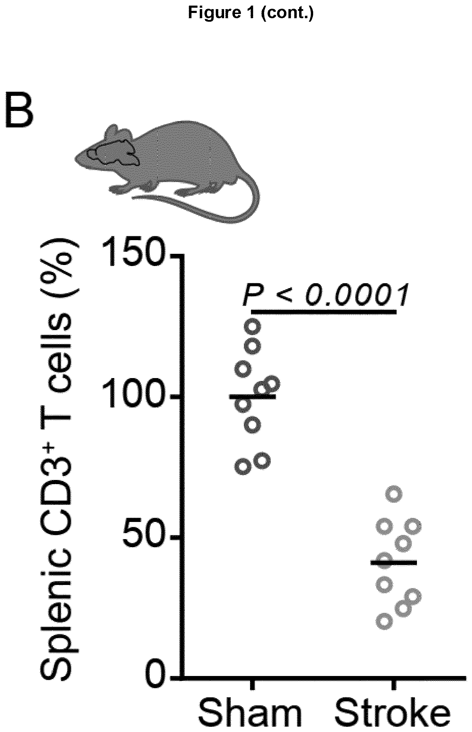

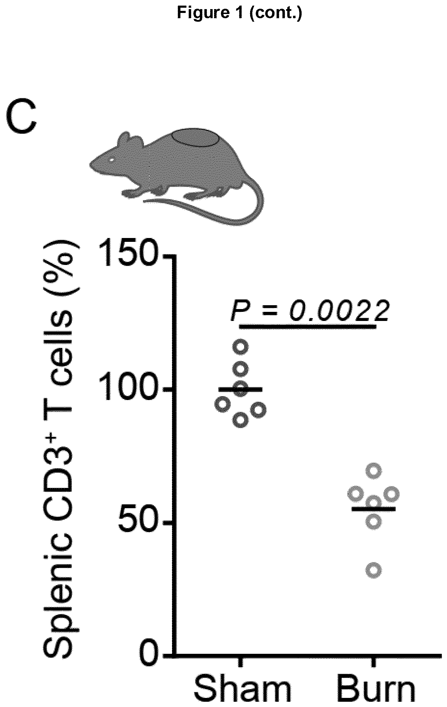

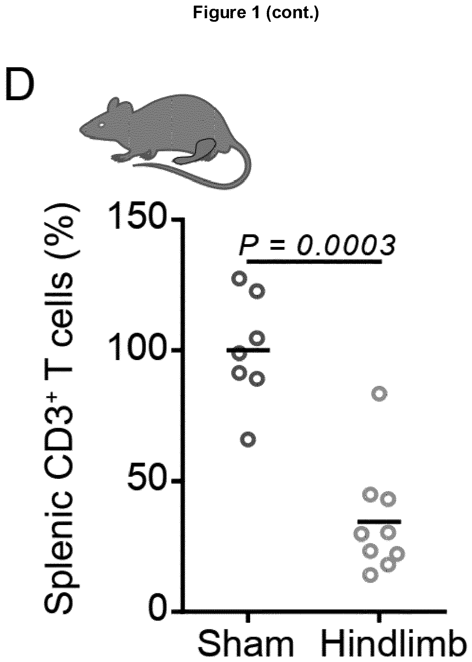

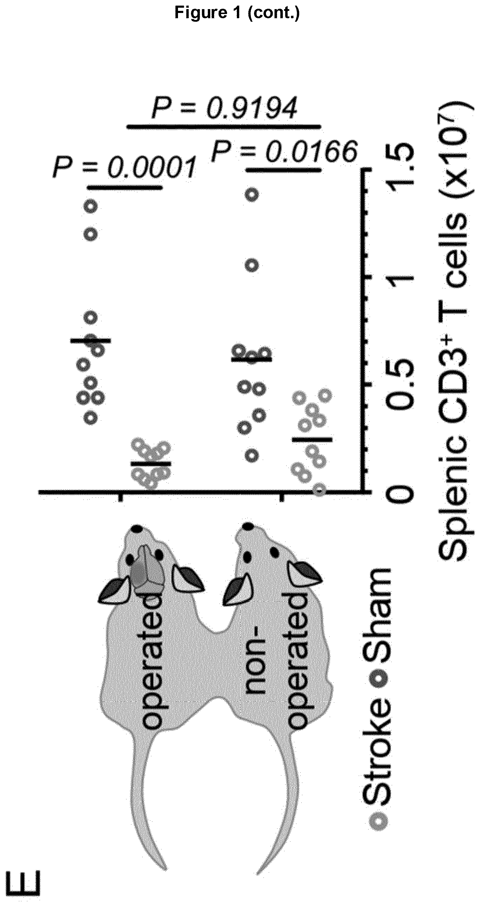

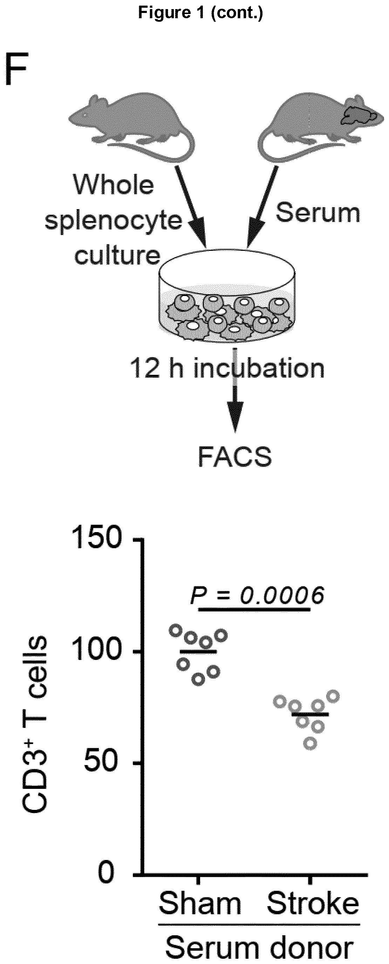

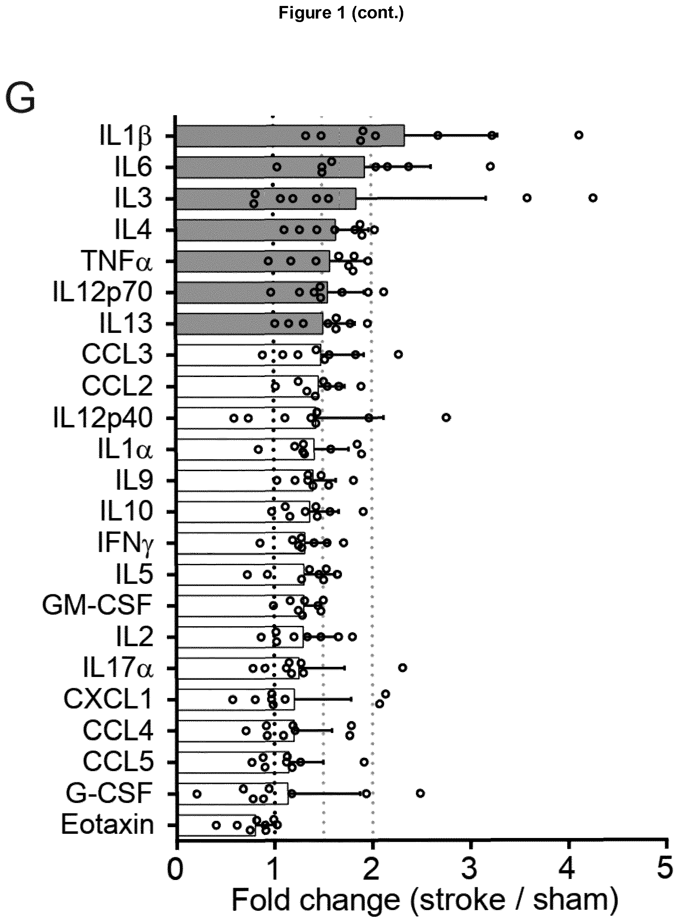

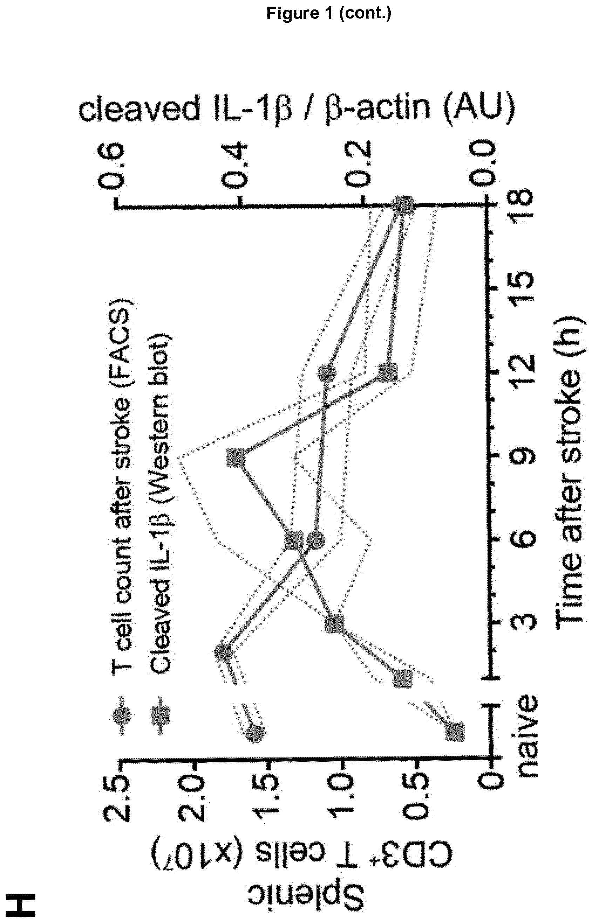

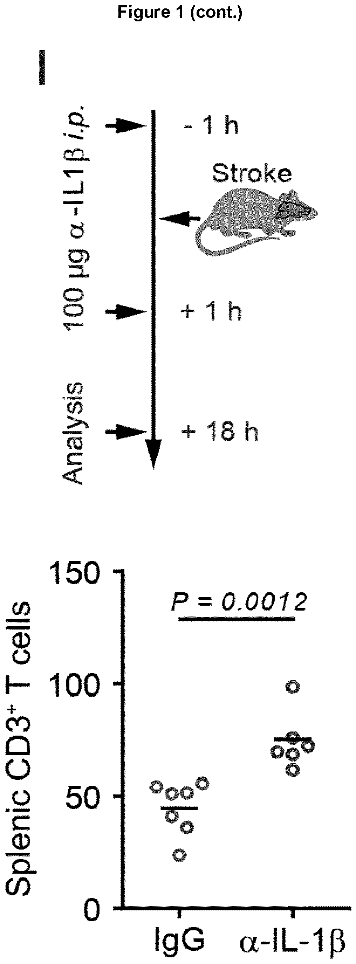

**Figure 1** shows soluble mediators inducing systemic lymphopenia after sterile injury via IL1β cleavage. **Figure 1A** shows representative photographs of whole spleens 18 h after sham or stroke surgery in mice. Figures 1B-D show flow cytometric (FACS) quantification of splenic T cells at 18 h after experimental stroke (**1B**), burn injury (**1C**) and transient hindlimb ischemia (**1D**) (n = 6-9 per group; U-test). Data for the intervention group was normalized to the respective sham group. **Figure 1E** shows parabionts, which underwent stroke or sham surgery and were sacrificed 18 h after surgical procedure for FACS quantification of CD45⁺CD3⁺ splenic T cells of both the operated and non-operated parabionts (n = 10 per group; H-test). **Figure 1E** shows the following experimental design: Whole splenocytes were isolated from naïve animals and then incubated with serum from stroke or sham operated mice for 12 h. Absolute cell count quantification of T cells was performed by flow cytometry (n = 7 per group; U-test). **Figure 1G** shows multiplex ELISA for cytokines and chemokines in serum of sham or stroke operated mice (normalized to sham group, n = 8 per group). Vertical lines indicate 2-fold (first vertical line from the right) and 1.5-fold (middle or second vertical line from the right) increase in stroke compared to sham. **Figure 1H** shows the time course of splenic T cell death and cleavage of IL-1β (n = 6-10 per group and time point, mean ± SEM). **Figure 1I** shows mice, which received two boluses of either neutralizing anti-IL1-β antibodies or isotype control antibodies 1 h before and 1 h after sham or stroke surgery. 18 h after stroke or sham surgery animals were sacrificed for FACS quantification of splenic T cells (n = 6-7 per group, U-test). All data (except in Figure 1I) are shown as mean ± s.d.

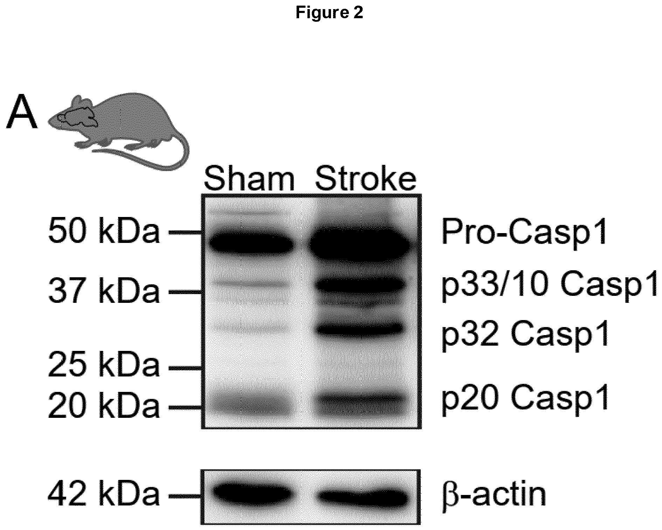

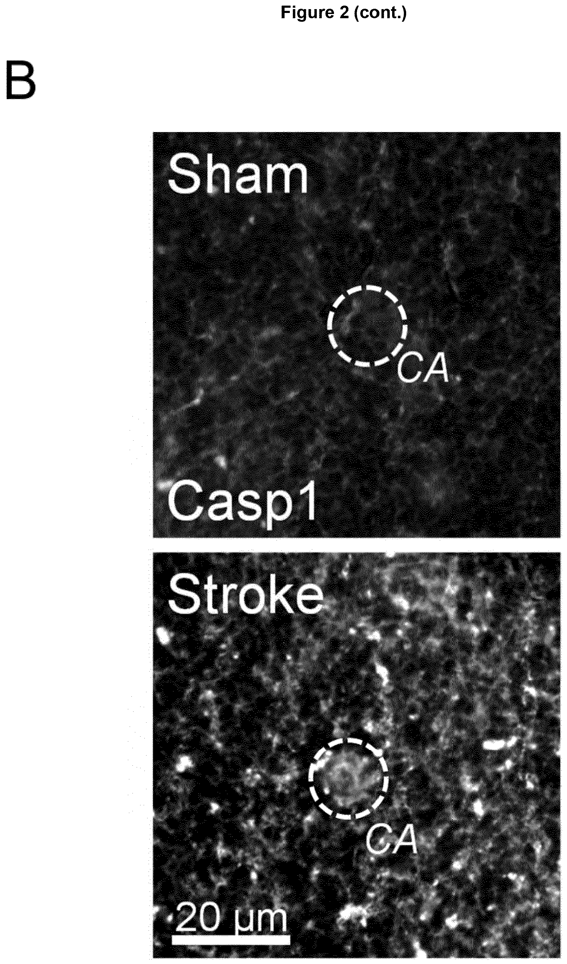

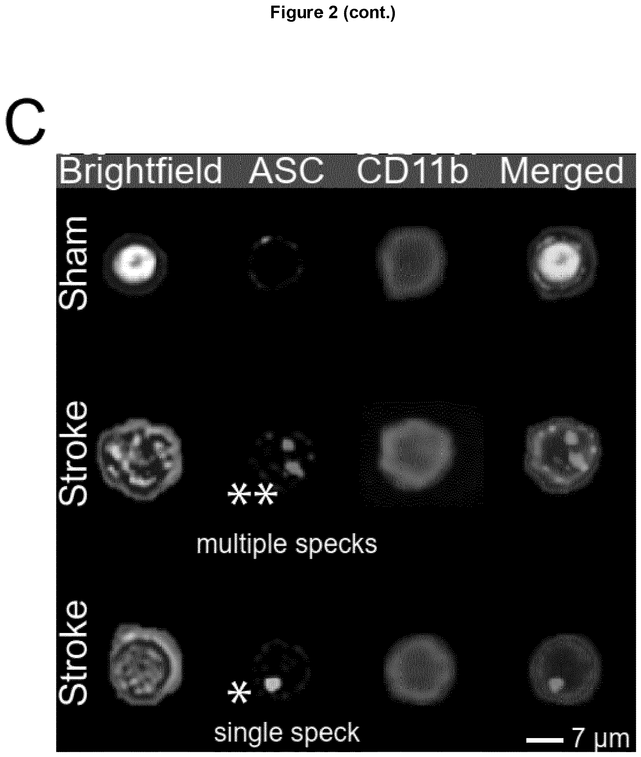

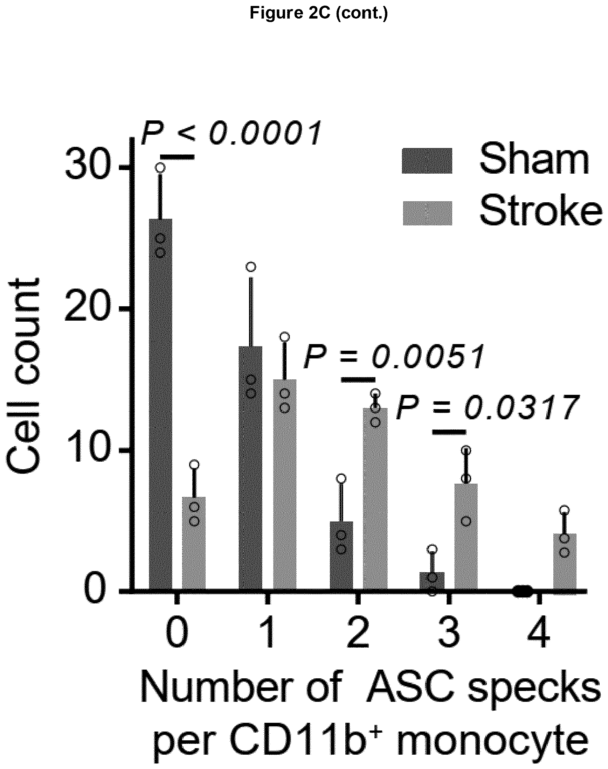

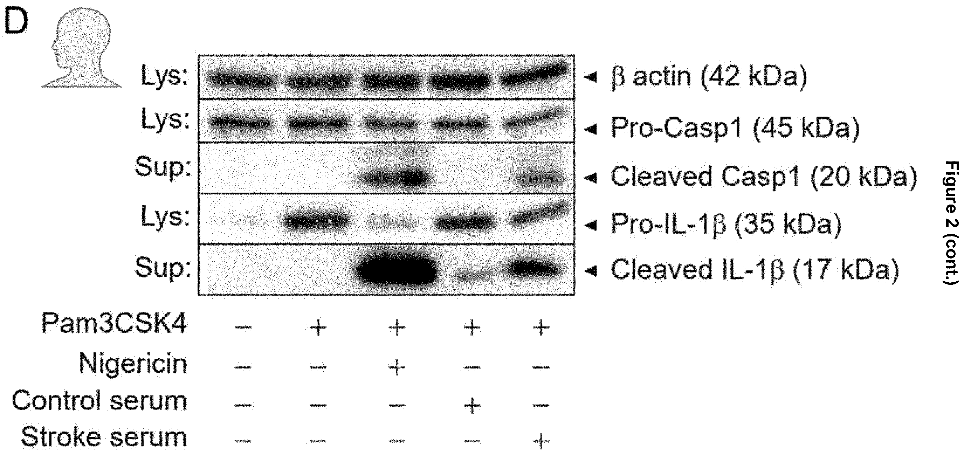

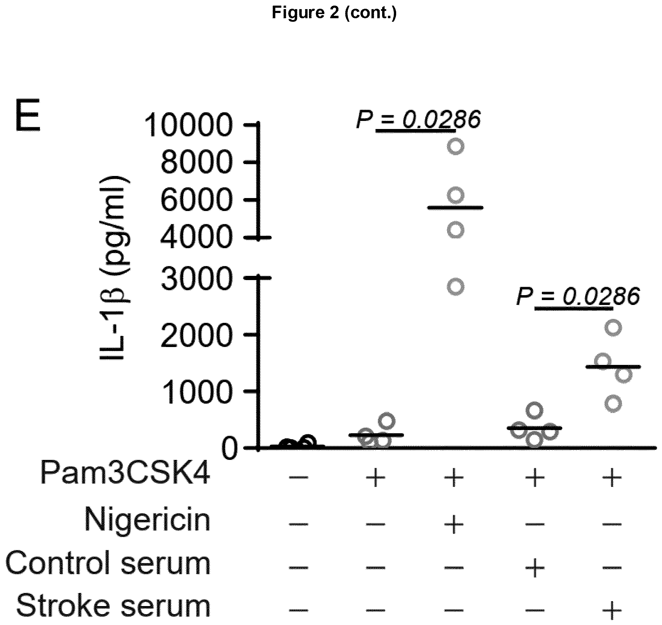

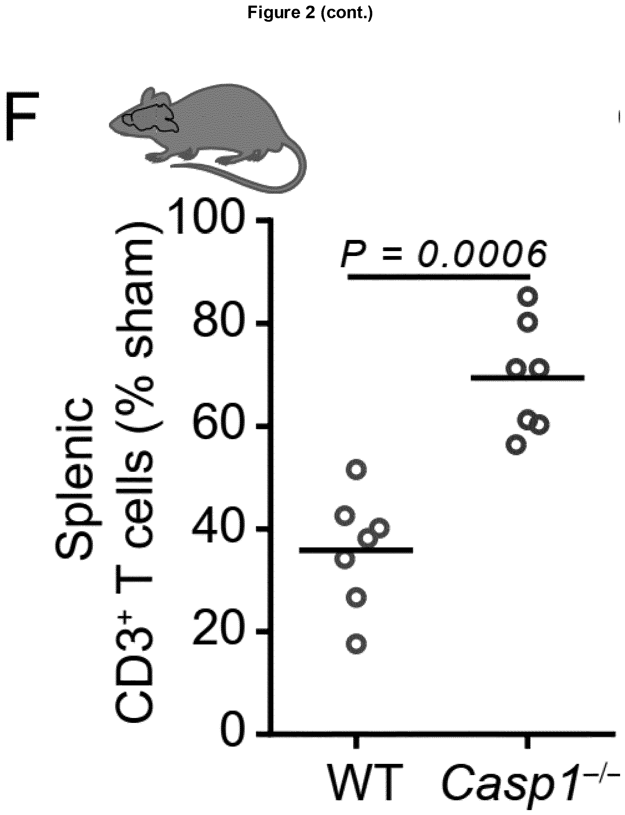

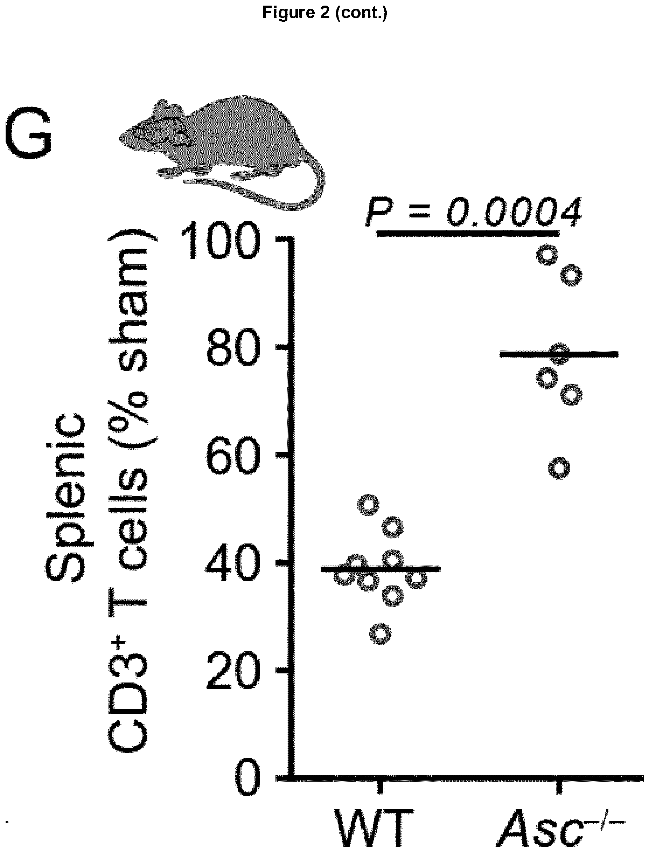

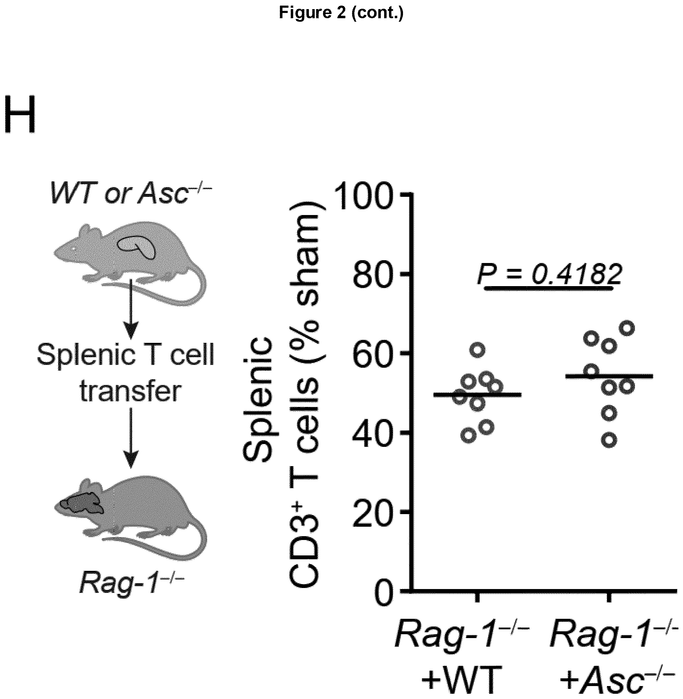

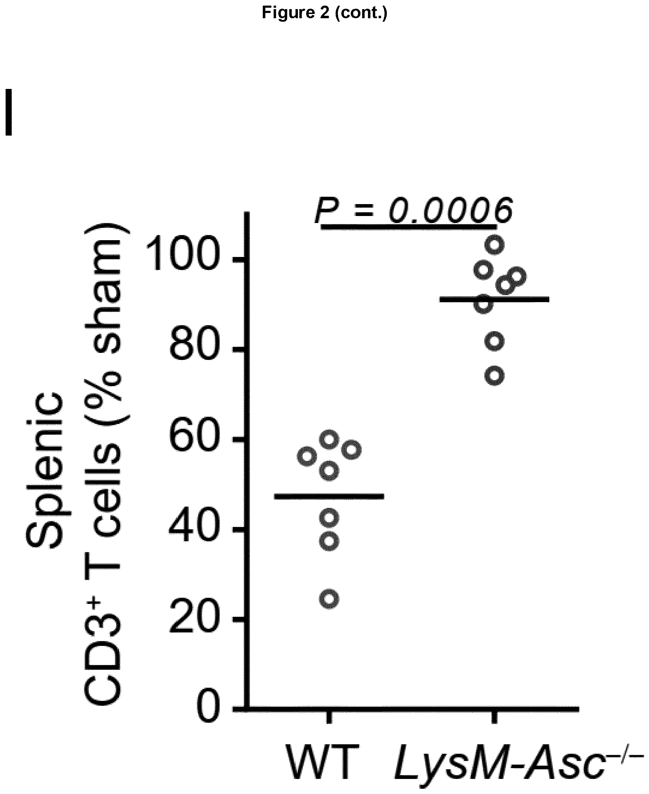

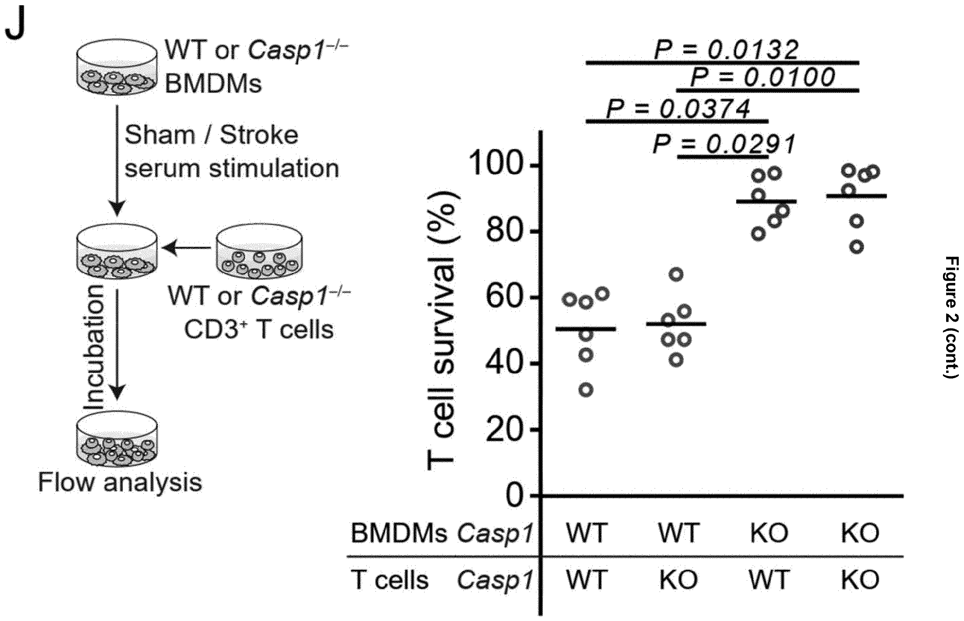

**Figure 2** shows systemic inflammasome activation caused by lymphopenia after stroke. **Figure 2A** shows a representative western blot micrograph of the different cleavage forms of caspase-1 (Casp1) in splenocyte lysates 12 h after stroke or sham surgery. **Figure 2B** shows representative images of a white pulp from murine spleen after sham or stroke surgery. Caspase-1⁺ (FAM FLICA) areas are labeled in the spleen 6 h after stroke (*CA* = central artery). **Figure 2C** shows ASC speck formations (*, **) and number of ASC specks per cell were analyzed by FACS-imaging of monocytes in ASC-citrine reporter mice 6 h after stroke and sham surgery. Cell count analysis for monocytes was done with the indicated number of ASC⁺ specks. In total, 300 monocytes were analyzed by FACS-imaging (50 randomly selected cells per mouse, 3 mice per group, H-test). **Figure 2D** and **Figure 2E** shows monocytes from human blood that were isolated and stimulated with either healthy control or stroke patient serum (Pam3CSK4 for priming, Nigericin as positive control). Lysates were harvested for western blot analysis and supernatants were used for caspase-1 and IL-1β cleavage analysis and IL-1β ELISA. **Figure 2D** shows representative western blot photographs of the different cleavage forms of caspase-1 and IL-1β detected in stimulated human monocyte lysates (Lys) and culture supernatants (Sup). **Figure 2E** shows monocyte culture supernatant levels of IL-1β that were measured via ELISA (n = 4 different monocyte donors; H-test). **Figure 2F** shows splenic T cell counts that were analyzed in caspase-1 deficient (*Casp1*^{-/-}) and wildtype (WT) mice 18 h after sham and stroke surgery (n = 7 per group; U-test), revealing significantly improved T cell survival in *Casp1*^{-/-} mice. **Figure 2G** shows ASC-deficient (*Asc*^{-/-}) and WT mice that underwent stroke or sham surgery and were sacrificed 18 h after the surgical procedure. Splenic T cells were analyzed by flow cytometry (n = 7 per group; U-test). **Figure 2H** shows splenic T cells from WT or *Asc*^{-/-} mice that were isolated and transferred to lymphocyte-deficient *Rag-1*^{-/-} mice for reconstitution of the T cell population. 4 weeks later these mice received either a stroke or sham operation and 18 h later splenic T cell numbers were analyzed by flow cytometry (n = 7-8 per group; U-test). **Figure 2I** shows quantification of splenic T cell counts in myeloid cell-specific ASC-deficiency (*LysM-Asc*^{*-*/*-*}) and WT mice 18 h after stroke or sham surgery (n = 7 per group; U-test). **Figure 2J** shows WT or *Casp1*^{-/-} BMDMs that were stimulated with serum of mice after sham or stroke surgery for 15 min. T cells (WT or *Casp1*^{-/-}) were added to the BMDMs and their survival was quantified 3 h later by flow cytometry (n = 6 per group; H-test). All data are presented as mean (± s.d.). Data in **Figure 2F** to **2I** are presented as stroke-operated mice normalized to the mean of sham operated within the respective treatment or genotype group.

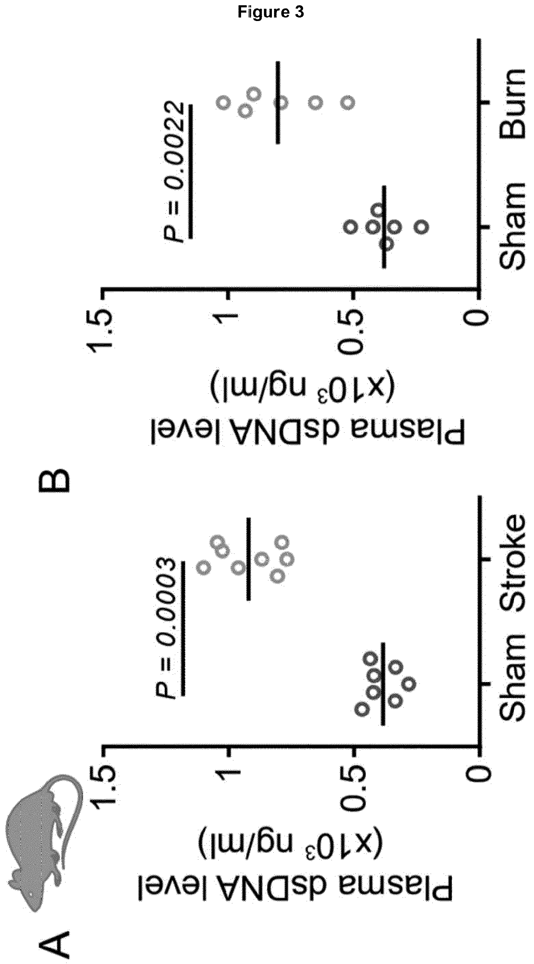

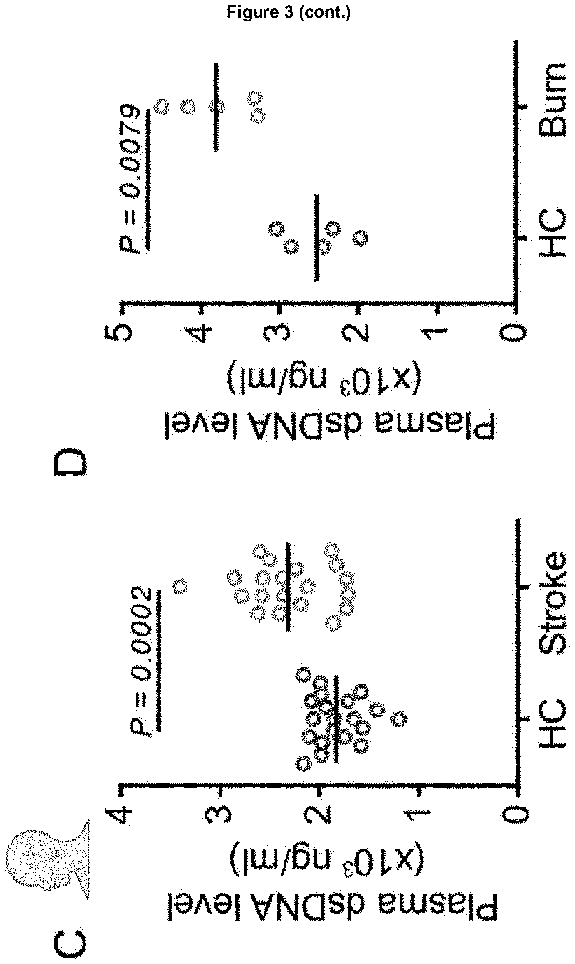

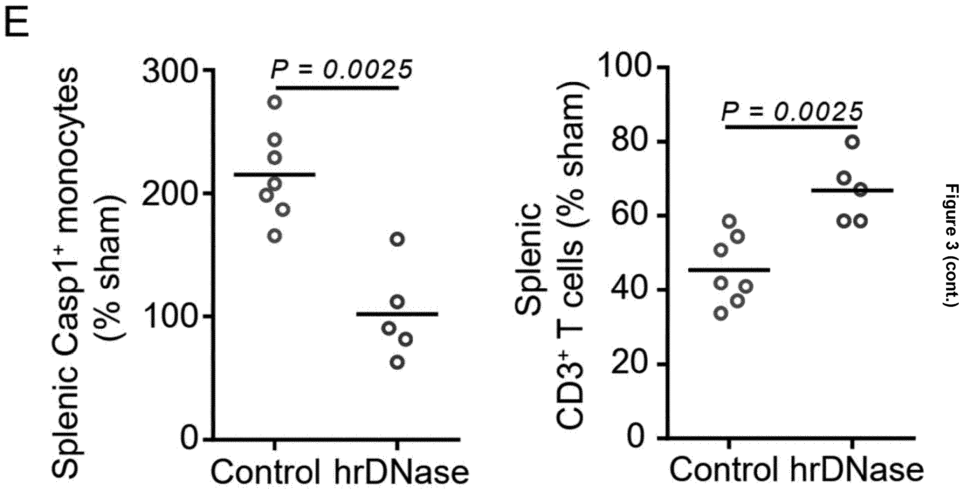

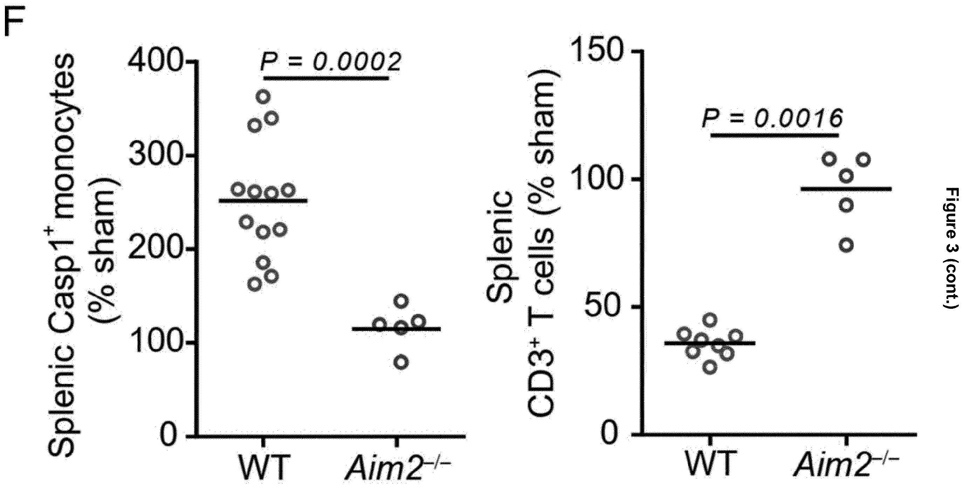

**Figure 3** shows free nucleic acids after tissue injury that induce the systemic inflammasome response. **Figure 3A** and **3B** show double strand (ds) DNA concentrations that were measured in mouse serum 6 h after stroke (**3A**) or burn injury (**3B**) and respective sham surgeries (n = 6-8 per group; U-test). Correspondingly, **Figure 3C** and **3D** show dsDNA levels that were analyzed in serum of stroke (**3C**) and burn injury patients (**3D**) in comparison to matched healthy control patients (n = 5-20 per group; U-test). **Figure 3E** shows cell-free dsDNA that was therapeutically degraded by *i.p.* administration of hrDNase after stroke. hrDNase treatment significantly reduced monocyte inflammasome activation and increased splenic T cell counts (n = 5-6 per group; U-test). **Figure 3F** shows comparison of WT and AIM2-deficient (Aim2 ") mice revealed a similar pattern of reduced inflammasome activation in monocytes and improved T cell survival in spleens of *Aim2*^{-/-} mice after stroke (n = 5-13 per group; U-test). Inflammasome activation in **Figures 3E** and **3F** was determined flow cytometrically using the caspase-1 probe FAM-FLICA.

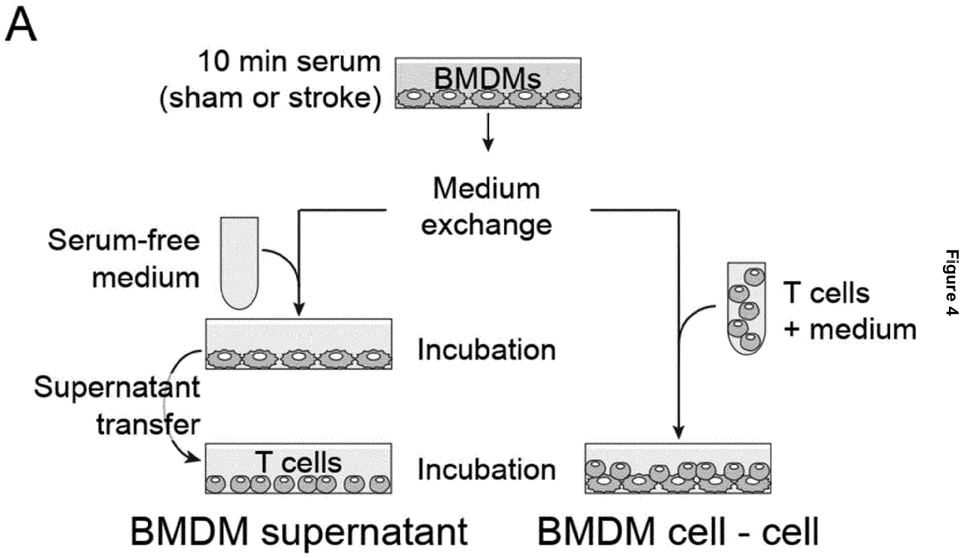

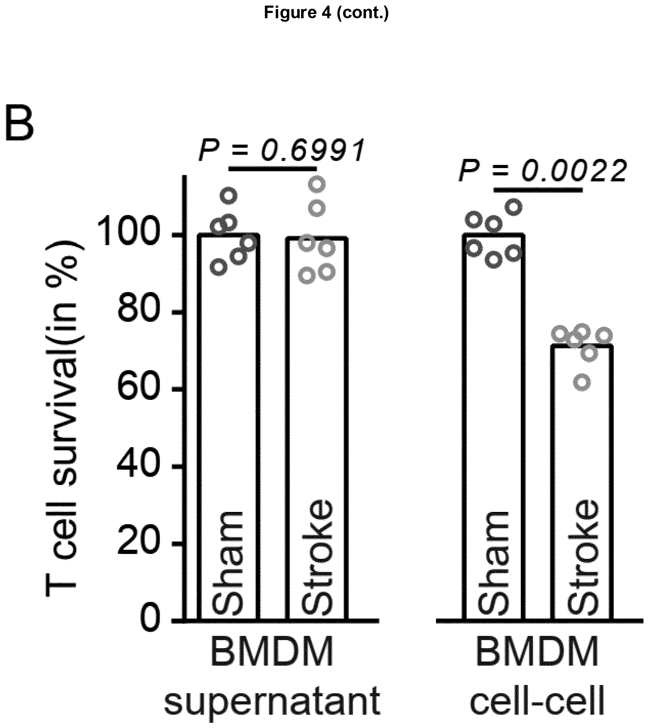

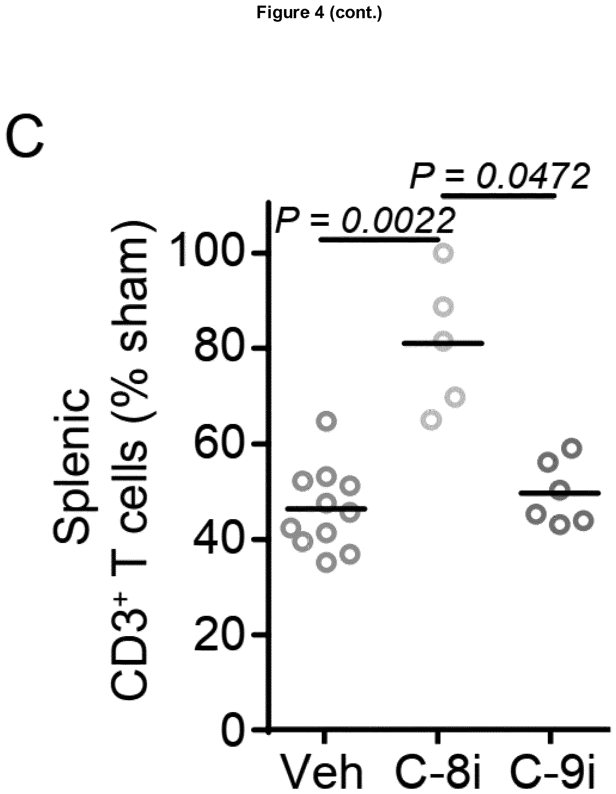

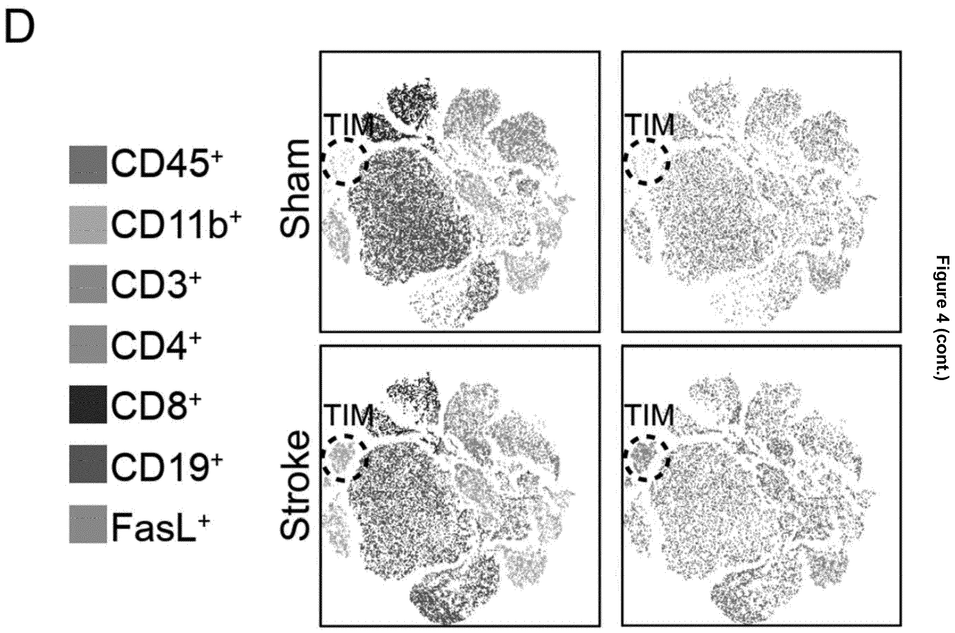

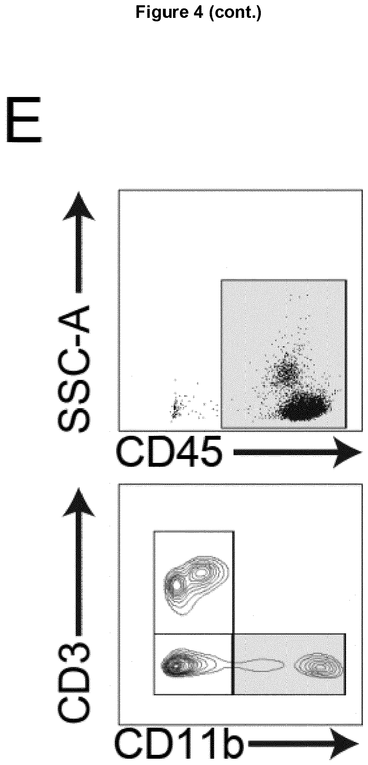

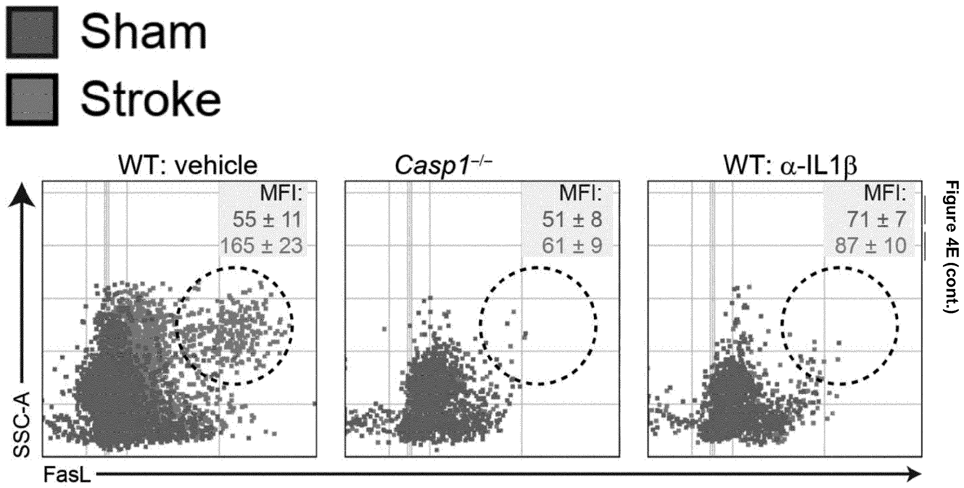

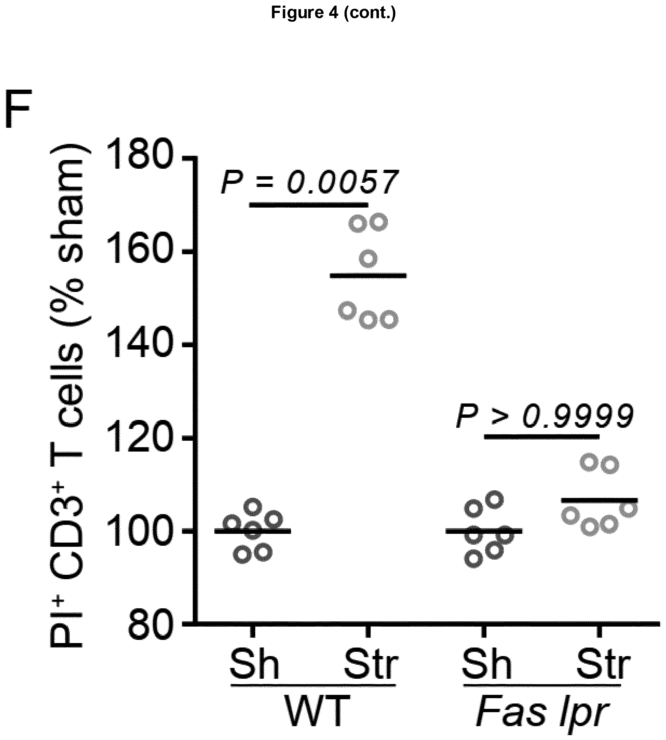

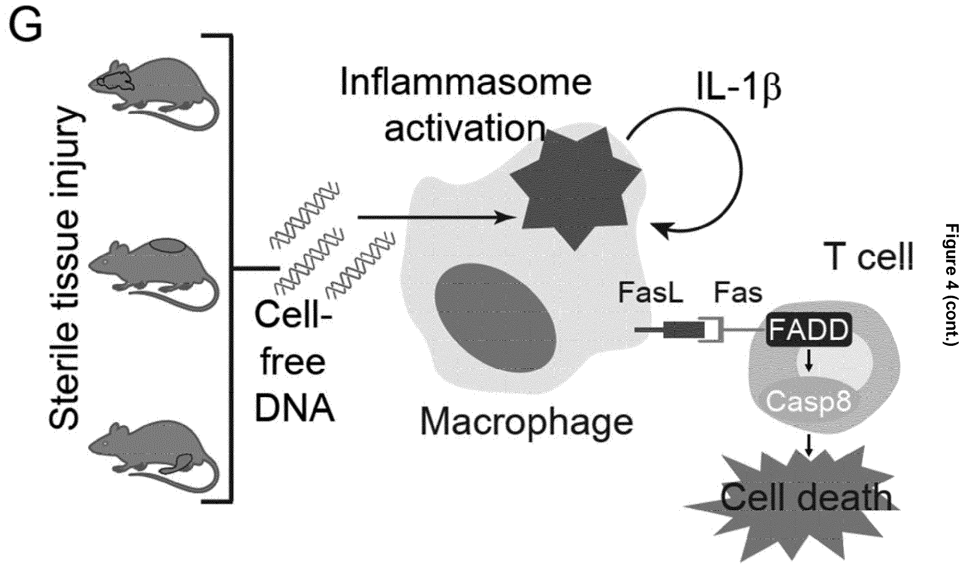

**Figure 4** shows Fas-FasL interaction that induces T cell death after sterile tissue injury. **Figure 4A** shows BMDMs that were stimulated with stroke or sham serum. Then, BMDM supernatant was used to stimulated T cells or T cells were directly added to the BMDMs enabling cell-cell contact. **Figure 4B** shows the co-culture of BMDMs and T cells that allowed cell-cell contact that resulted in stroke serum-induced T cell death (n = 6 per group, H-test). **Figure 4C** shows the role of intrinsic versus extrinsic apoptosis pathways for T cell death, which was tested by i.p. administration of either caspase-8 (C-8i) or caspase-9 (C-9i) inhibitor after sham or stroke surgery (n = 5-11 per group; H-test). T cell death was analyzed by flow cytometry and presented for stroke-operated mice normalized to the mean of sham operated mice. **Figure 4D** shows t-SNE plots of flow cytometry data from whole murine spleen color-coded by the epitope markers for T cells (CD4⁺ Tₕₑₗₚₑᵣ and CD8⁺ T_{cytotoxic}), CD19⁺ B cells and CD11b⁺ monocytes (left panels) and FasL expression in leukocytes (right panels) 18 h after sham or stroke surgery. Comparison of sham and stroke conditions reveals a population of tissue injury-induced monocytes (TIM) expressing high levels of FasL (n = 8 mice per group; 3,000 CD45⁺ cells per mouse). **Figure 4E** shows left a representative gating strategy for analysis of FasL-expressing monocytes in WT mice treated with vehicle or anti-IL-1β and *Casp1*^{-/-} mice after sham or stroke surgery (n = 6-8 per group). Grey shaded boxes depict the mean fluorescence intensity (MFI) for FasL in the respective Sham and Stroke group. **Figure 4F** shows T cell death that was analyzed by PI uptake in a co-culture approach of WT or Fas-deficient (*Fas Ipr*) T cells with WT BMDMs enabling cell-cell contact as depicted in Figure 4A. PI uptake has been microscopically quantified and is presented as percentage of the respective Sham group (n = 6 per group; U-test per genotype). **Figure 4G** shows a schematic overview of proposed mechanism of inflammasome-induced T cell death after sterile tissue injury.

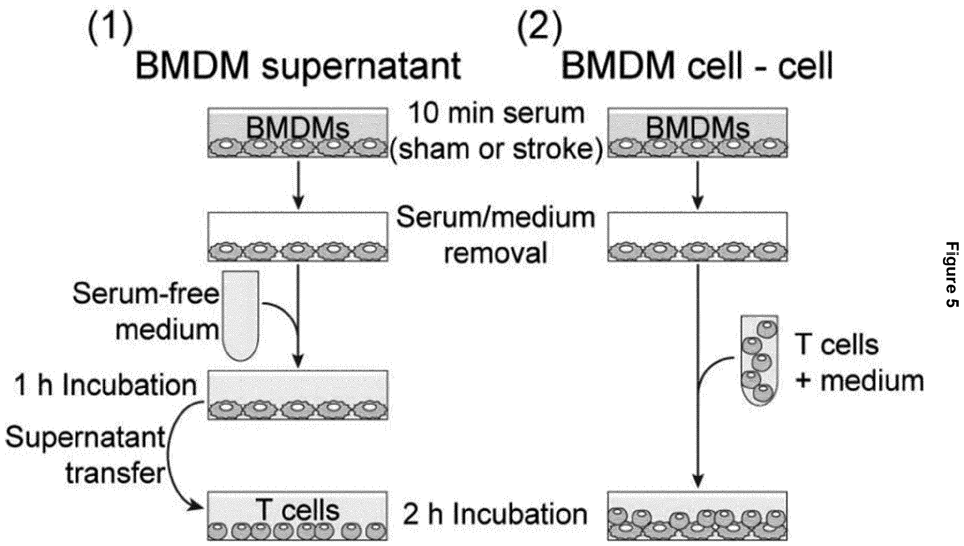

**Figure 5** shows differentiated BMDMs that were cultured for 8-10 days, then harvested, washed, counted, and seeded in flat-bottom tissue-culture treated 96-well plates at a density of 100,000 cells per well in a total volume of 200 µl, and then cultured overnight for 16 h. BMDMs were stimulated for 4 h with LPS (100 ng/ml) and by 10 minute incubation with serum from either stroke or sham operated wild type mice at a concentration of 25 % total volume. Control-treated BMDMs received only FBS-containing culture media. After stimulation, the culture medium was removed, and the cells were washed with sterile PBS to ensure no leftover serum in the medium. BMDM-T cell interaction was then assessed with two approaches: 1. Stimulation by secreted factors (left), and 2. Cell-cell contact (right). 1: Serum-free RPMI was added to the BMDMs, which were then incubated for 1 hour at 37 °C with 5 % CO₂. The BMDM-conditioned supernatant was then transferred onto purified, cultured T cells and incubated for 2 hours at 37 °C with 5 % CO₂. 2: T cells were added to the serum-stimulated BMDMs at a density of 200,000 cells per well in a total volume of 200 µl complete RPMI medium (10 % FBS, 1 % penicillin/ streptomycin and 10 µM β-mercaptoethanol), and then incubated for 2 hours at 37 °C with 5 % CO₂. T cell counts and survival rate were assessed by flow cytometry.

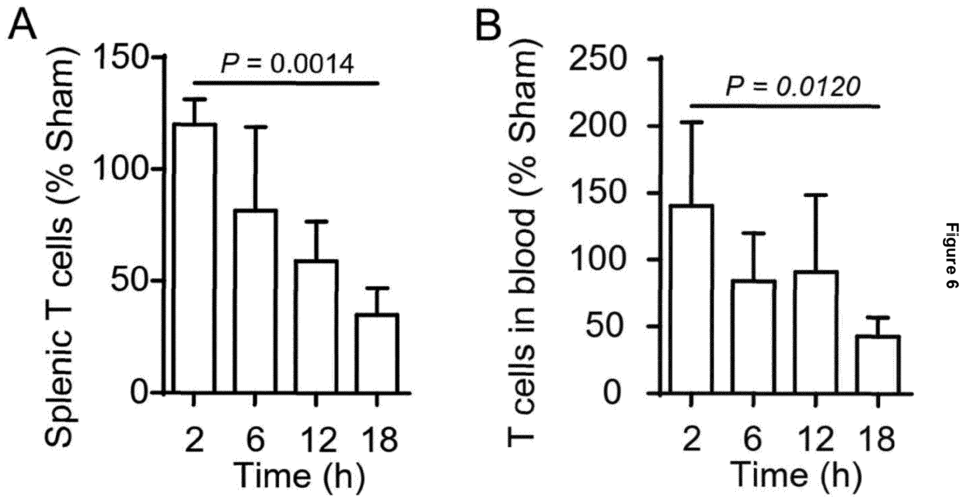

**Figure 6** shows the kinetics of T cell death in spleens and blood after experimental stroke. WT mice received a stroke or sham surgery and were sacrificed 2, 6, 12 and 18 h after operation. Blood and spleens were collected and analyzed by flow cytometry (FACS) for CD3+ T cell counts. **Figure 6A** shows FACS quantification of splenic T cells 2, 6, 12 and 18 h after stroke (n = 6-9 per group; H-test). **Figure 6B** shows FACS quantification of T cells in blood 2, 6, 12 and 18 h after stroke (n = 6-9 per group; H-test). Data for the stroke group was normalized to the sham group. All data are presented as mean ± s.d.

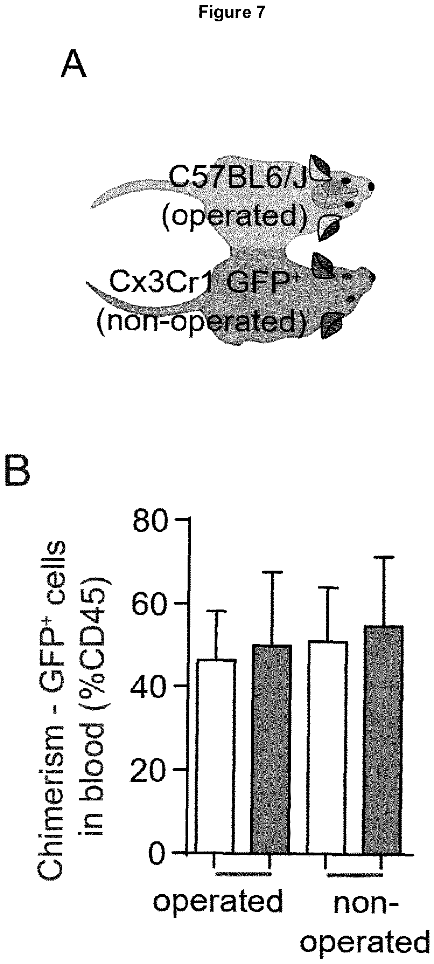

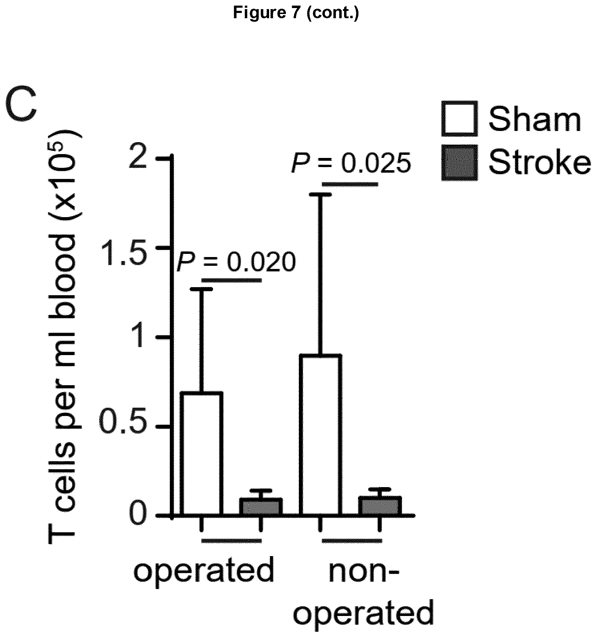

**Figure 7** shows that parabiosis reveals soluble mediators as the initiators for T cell death after stroke. **Figure 7A** shows the schematic of the parabionts showing the operated (C57BI6/J) and non-operated (Cx3Cr1 GFP+) mice sharing a common circulation. **Figure 7B** shows FACS analysis for the percentage of GFP+ cells within the CD45+ leukocyte population, which reveals a shared circulation and chimerism close to 50 % of Cx3Cr1 GFP+ mouse-derived leukocytes of all groups (n = 10 per group, H-test). **Figure 7C** shows FACS quantification of T cells in blood of the operated and non-operated parabionts 18 h after stroke or sham surgery (n = 10 per group; H-test). All data are presented as mean ± s.d.

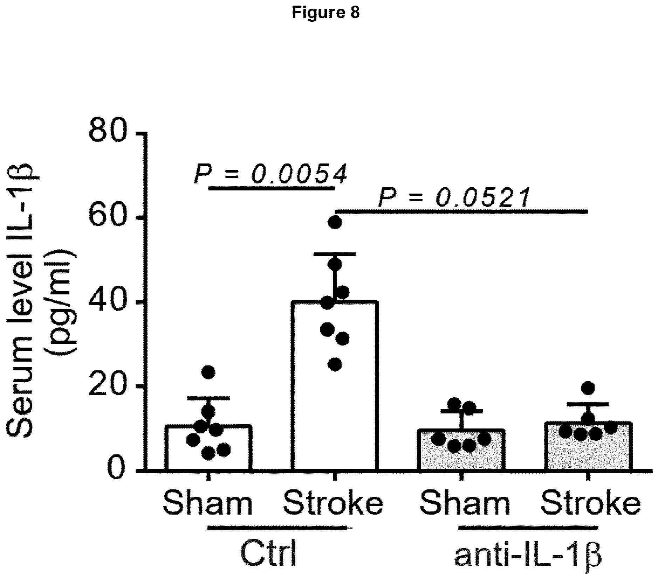

**Figure 8** shows that IL-1β-specific antibodies reduce IL-1β serum concentrations. Mice were treated i.p. with either IgG isotype control or IL-1β-specific neutralizing monoclonal antibodies and sacrificed for serum collection 18 h after stroke or sham surgery. ELISA for IL-1β revealed decreased IL-1β serum concentrations in stroke mice treated with the neutralizing antibody to levels of Sham-operated mice (n = 6-7 per group; H-test). All data are presented as mean ± s.d.

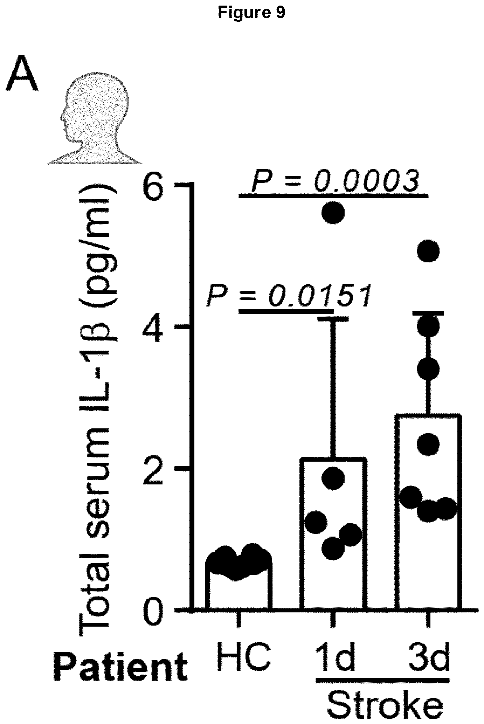

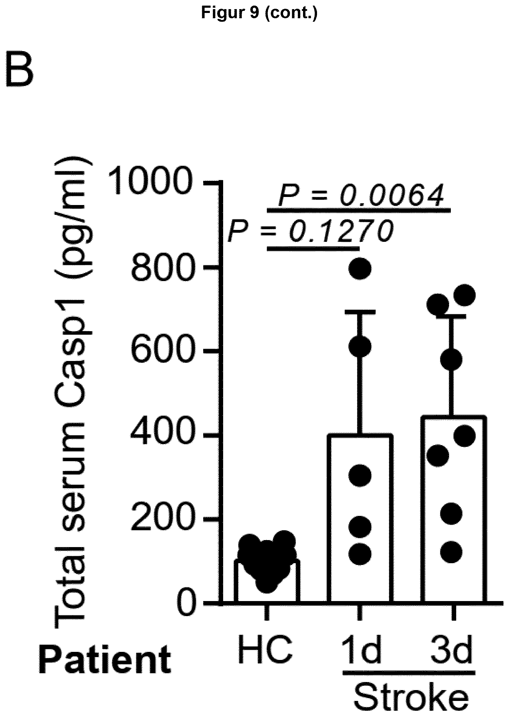

**Figure 9** shows increased serum levels of IL-1β and caspase-1 in stroke patients. **Figure 9A** and **9B** show serum from stroke patients (Stroke) and age-matched healthy controls (HC) were analyzed via ELISA for IL-1β (**Figure 9A**) and caspase-1 (**Figure 9B**). Both markers of inflammasome activation were significantly increased in patients at 1 d and 3 d after stroke compared to age-matched healthy controls (n = 5-10 per group; H-test). All data are presented as mean (± s.d.).

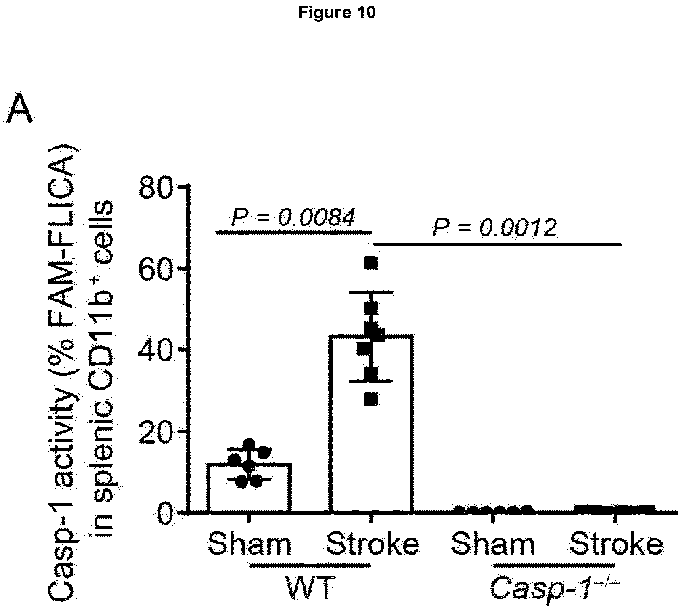

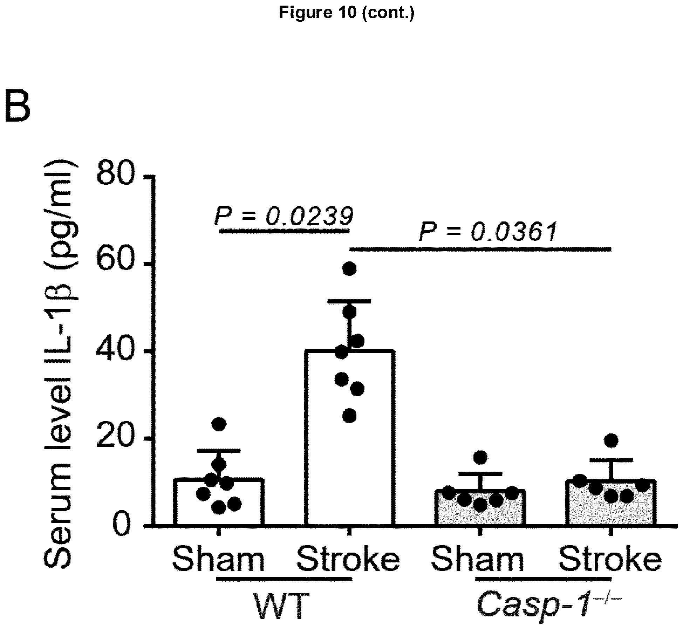

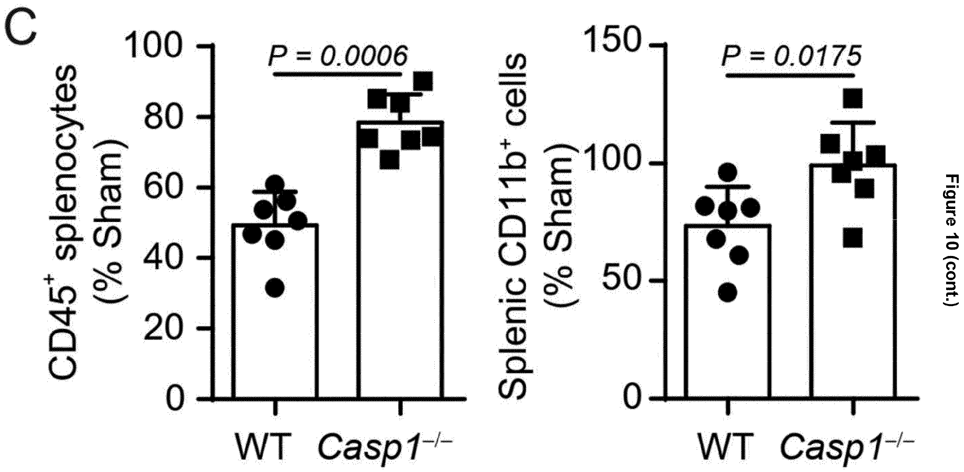

**Figure 10** shows that genetic caspase-1 deficiency decreases circulating IL-1β and restores splenic cellularity. **Figure 10A** shows FAM FLICA flow cytometry analysis of caspase-1 activity in splenic CD11b+ monocytes of WT or *Casp1* mice 6 h after sham or stroke surgery (n = 7 per group; H-test). **Figure 10B** shows serum IL-1β concentrations of WT or *Casp1* mice 6 h after sham or stroke surgery (n = 6 per group; U-test). **Figure 10C** shows that *Casp1* mice have significantly restored cell counts for CD45+ splenocytes and CD11b+ monocytes 18 h after stroke (normalized to respective sham group) compared to WT animals (n = 7 per group; U-test). All data are presented as mean ± s.d.

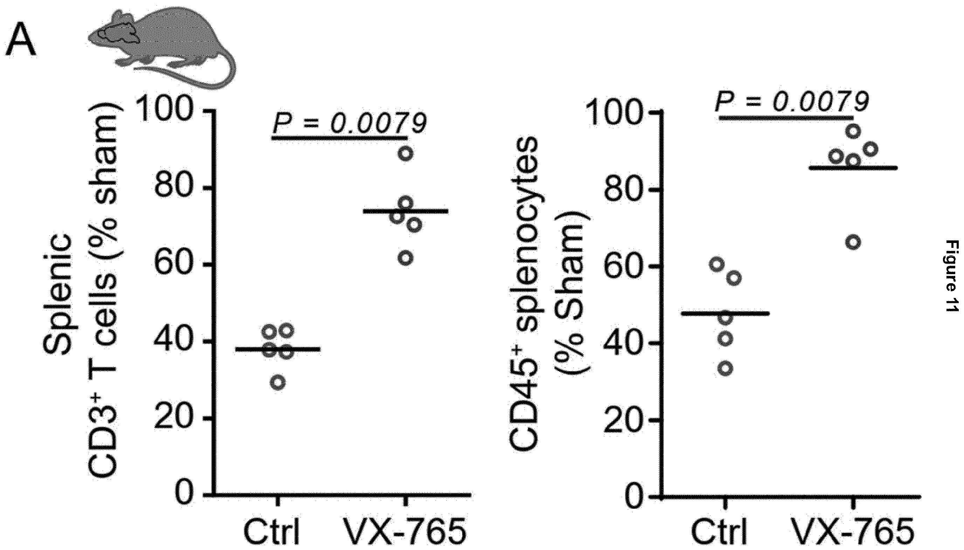

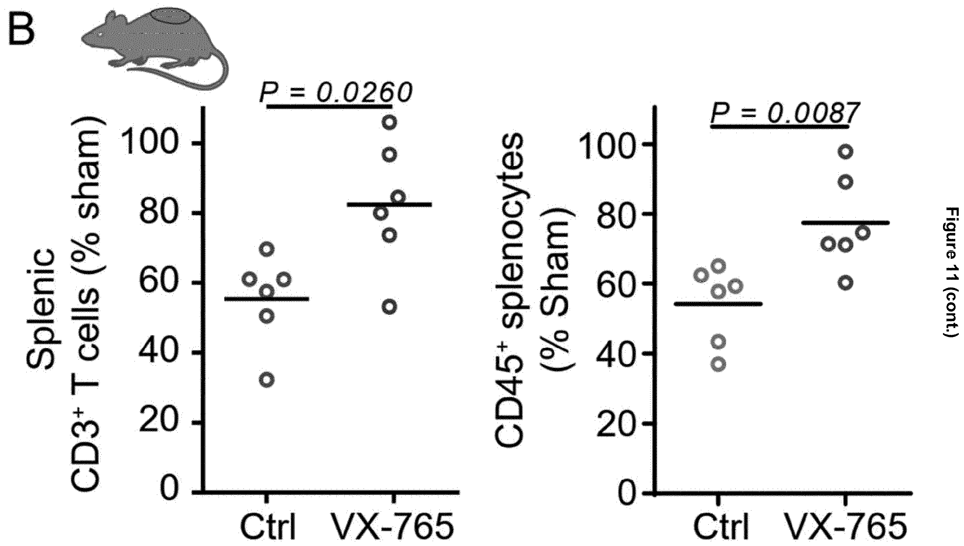

**Figure 11** shows that pharmacological caspase-1 inhibition reduced T cell death after acute tissue injury. **Figure 11A** shows that mice received either a single bolus of the caspase-1 inhibitor VX-765 or vehicle (control) 1 h prior sham or stroke surgery (n = 5 per group; U-test). **Figure 11B** shows that mice received VX-765 or vehicle treatment (control) 1 h prior to burn injury or sham surgery (n = 5 per group; U-test). In both tissue injury models, FACS quantification of splenic CD3⁺ T cell and overall CD45⁺ splenocyte counts revealed an improved survival after VX-765 treatment compared to control (n = 5 per group; U-test). Data for intervention group was normalized to the respective sham group. All data are presented as mean ± s.d.

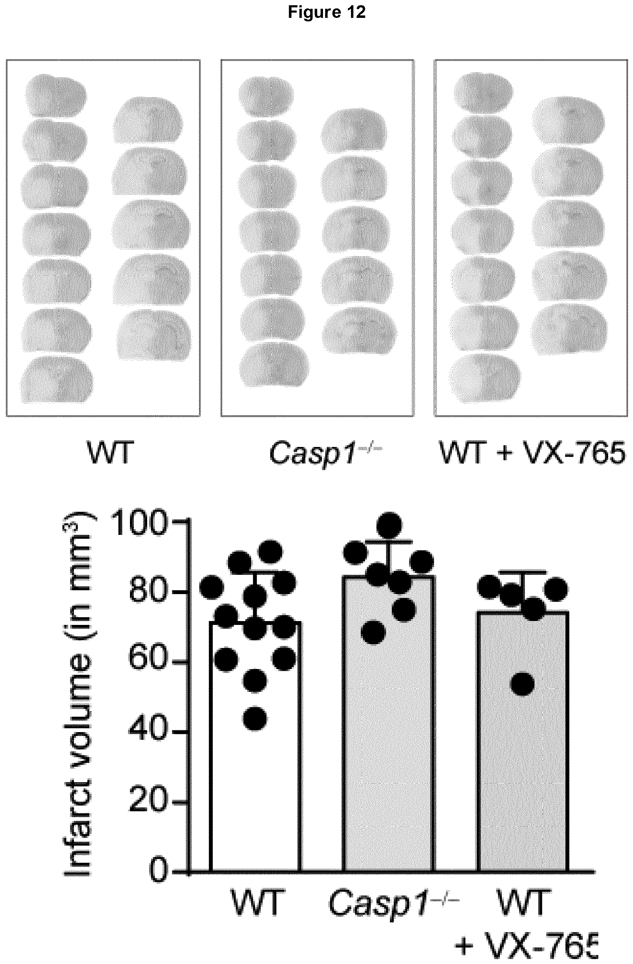

**Figure 12** shows that caspase-1 deficiency or inhibition does not affect the primary lesion size. Brains of WT mice receiving either VX-765 or control treatment and Casp-1 mice were analyzed 18 h after stroke for infarct volume. No significant difference in infarct volume between groups (n = 5-12 per group; H-test). All data are presented as mean ± s.d.

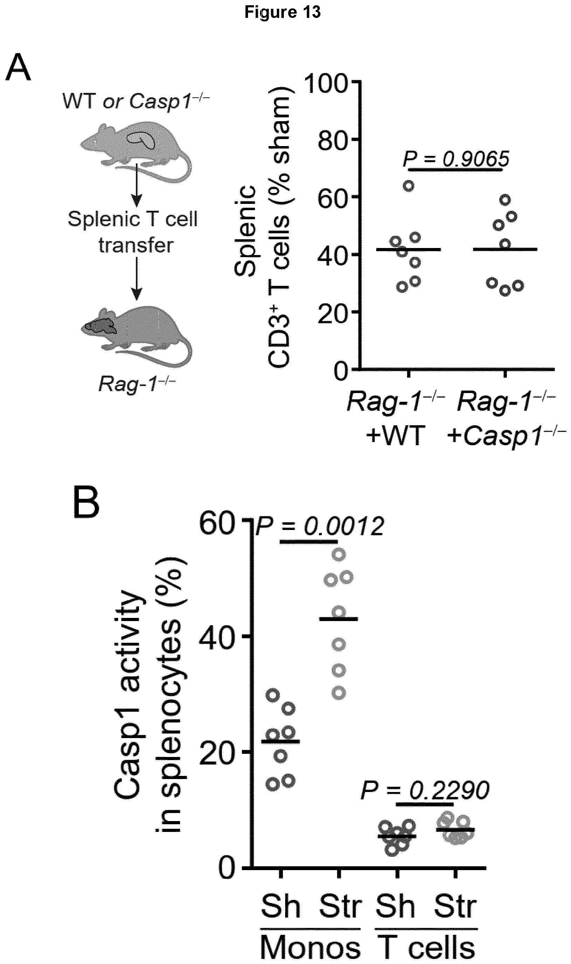

**Figure 13** shows that inflammasome activation in T cells does not contribute to their cell death after tissue injury. **Figure 13A** shows that adoptive transfer of splenic WT or *Casp1* T cells to lymphocyte-deficient *Rag1* was performed and 4 weeks later these mice received either a stroke or sham surgery. FACS quantification of splenic CD3⁺ T cells 18 h after surgery revealed no differences in T cell death between transferred WT and *Casp1* splenic T cells (n = 7 per group; U-test). Data for stroke groups were normalized to the corresponding sham-operated groups. Panel labels indicate the genotype of transferred T cells (i.e. WT or *Casp1*). **Figure 13B** shows FAM FLICA flow cytometry analysis of caspase-1 activity in splenic CD11b⁺ monocytes and CD3⁺ T cells 6 h after sham (Sh) or stroke (Str) surgery, which showed an increase of caspase-1 activity in monocytes, but not in T cells. Data in **Figure 13A** and **Figure 13B** are presented as mean ± s.d.

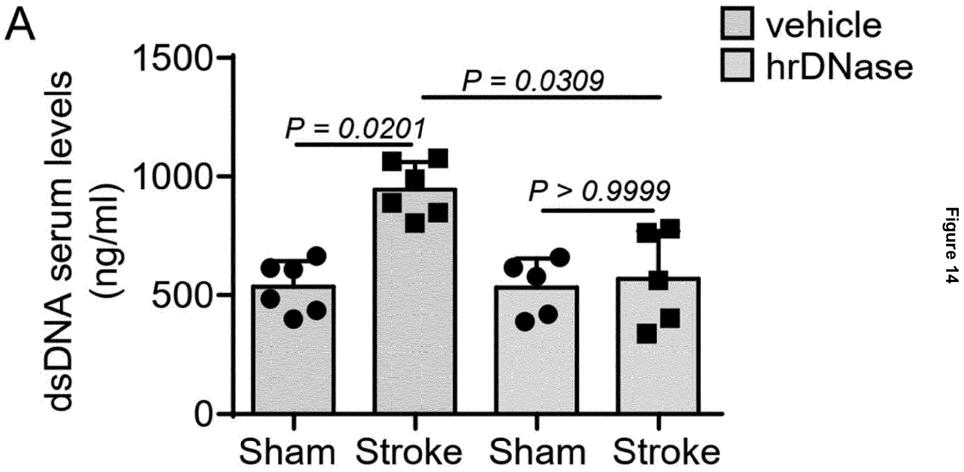

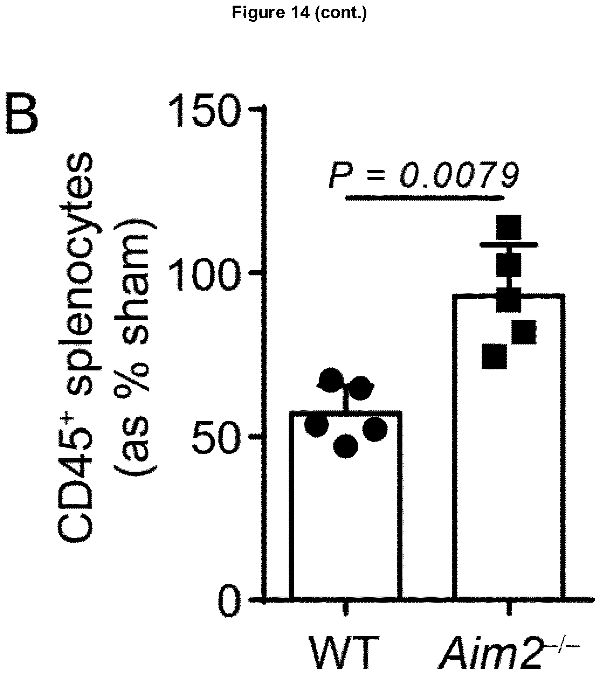

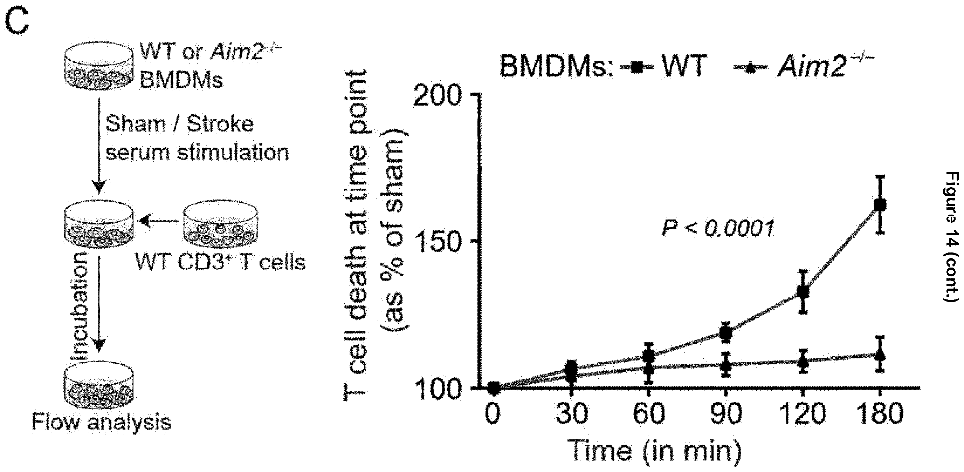

**Figure 14** shows that cf-dsDNA is sensed by the AIM2 inflammasome and can be degraded by hrDNAse. **Figure 14A** shows that double strand (ds) DNA concentrations were measured in the serum of hrDNase- or vehicle-treated mice 6 h after stroke or sham surgery. Fluorescence-based quantification revealed a decrease of serum dsDNA concentration in mice receiving hrDNase treatment after stroke to levels of sham-operated mice (n = 6 per group; H-test). **Figure 14B** shows FACS quantification of CD45⁺ splenocytes from WT and *Aim2* mice after stroke showing restored spleen cellularity in *Aim2* mice. Data is presented for stroke groups normalized to the sham group of the same genotype. **Figure 14C** shows that BMDMs were stimulated with stroke or sham serum, the serum was removed and eGFP+ T cells were added for subsequent live imaging of eGFP⁺ T cell survival in co-culture with WT or *Aim2*BMDMs. Results are shown as T cell death rate for T cells in the stroke-serum normalized to sham serum-treated BMDMs culture conditions (n = 4 per group, 2-way ANOVA). Data in (A) and (B) are shown as mean ± s.d., results in (C) are shown as mean ± s.e.m.

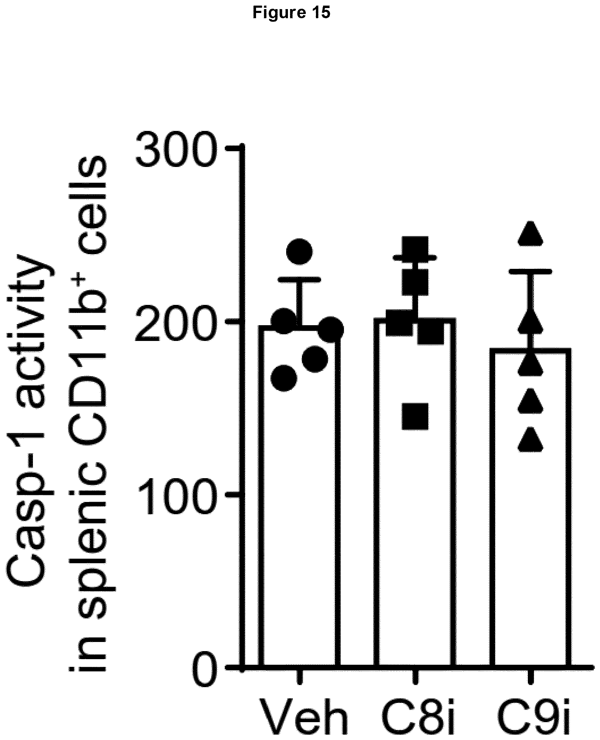

**Figure 15** shows that pharmacological inhibition of caspase-8 and-9 does not affect caspase-1 activity. WT mice were either treated with a caspase-8 inhibitor (C8i), caspase-9 inhibitor (C9i) or control vehicle (Veh) immediately after sham or stroke surgery. 18 h after the surgery, mice were sacrificed and FAM FLICA FACS was used to quantify caspase-1 activity in splenic CD11b⁺ monocytes. Neither caspase-8 nor caspase-9 inhibitor showed differences in caspase-1 activity compared to the vehicle-treated mice. Results are shown for monocytes from stroke-operated mice normalized to the respective sham-operated group receiving the same treatment. All data are presented as mean ± s.d.

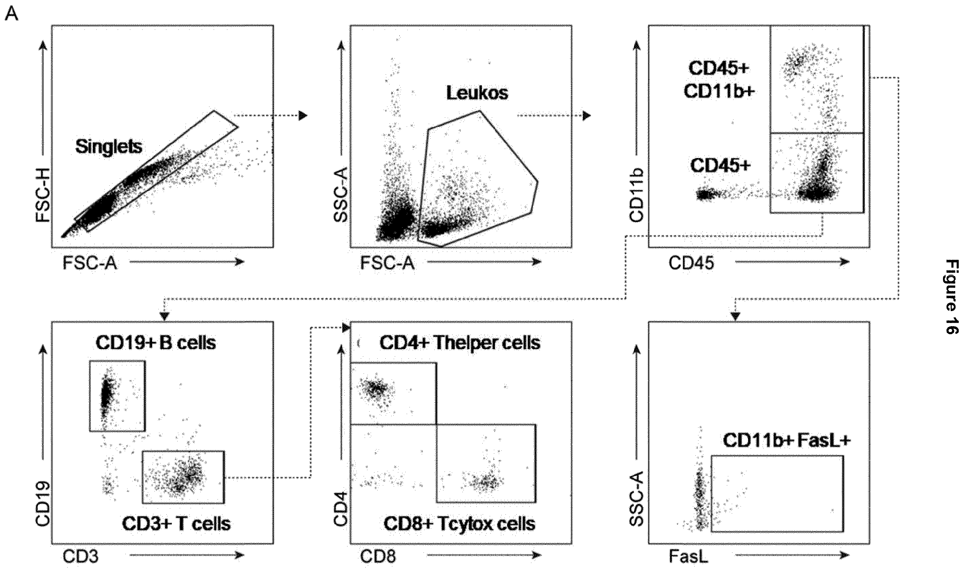

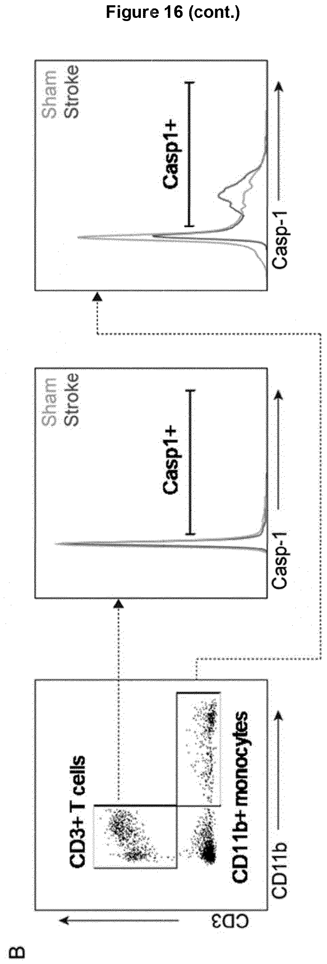

**Figure 16** shows representative FACS gating strategies. **Figure 16A** shows whole splenocytes stained for CD45, CD3, CD4, CD8, CD19, CD11b and FasL that were acquired and gated for CD45⁺CD11b⁺(FasL⁺) monocytes, CD3⁺ T cells (CD3⁺CD4⁺ T helper cells and CD3⁺ CD8⁺ T cytotoxic cells) and CD3⁻CD19⁺ B cells. **Figure 16B** shows that for the FAM FLICA flow cytometry whole splenocytes were acquired and pre-gated for CD45⁺ expression. T cells were defined as CD45⁺ CD3⁺ and monocytes as CD45⁺CD11b⁺. FAM FLICA expression was analyzed in both populations, T cells and monocytes.

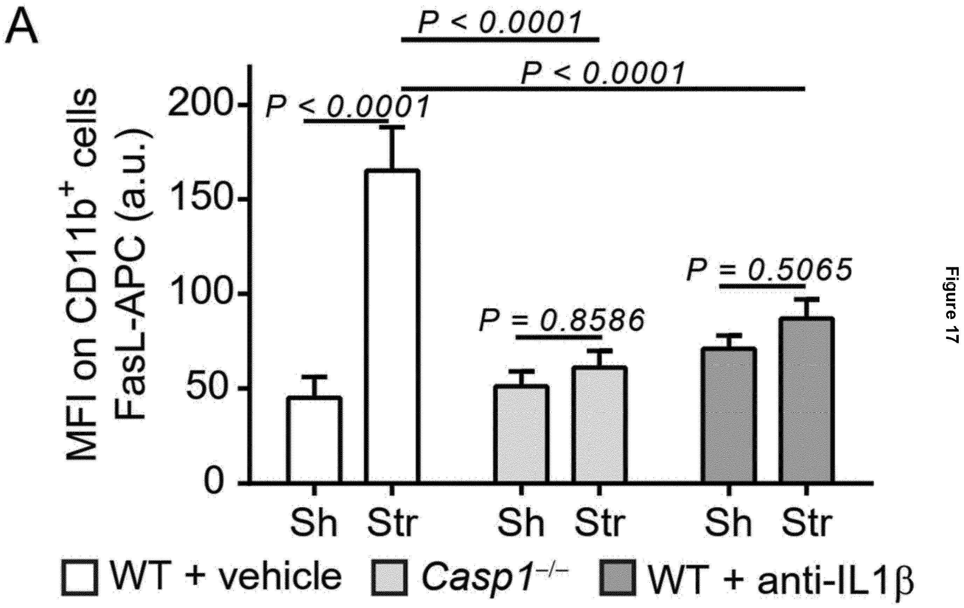

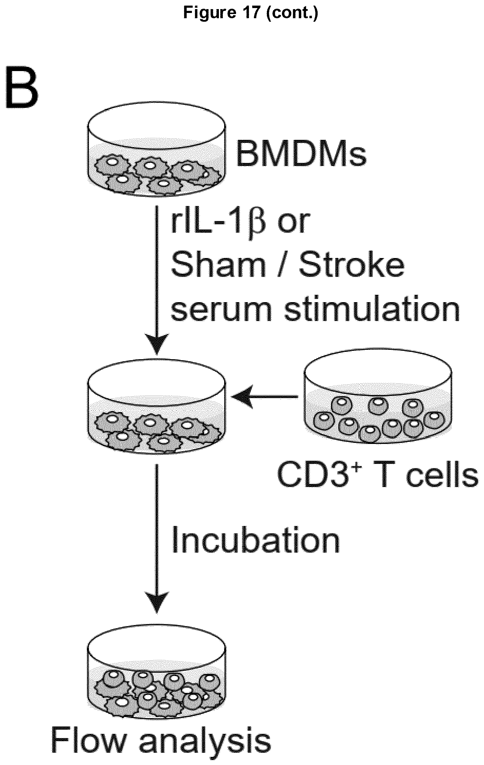

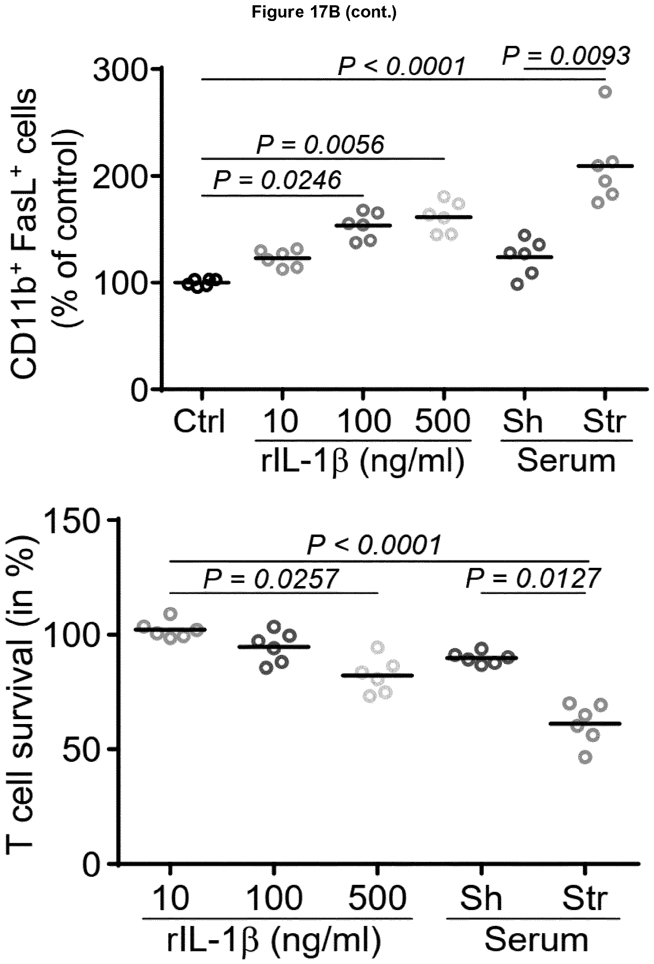

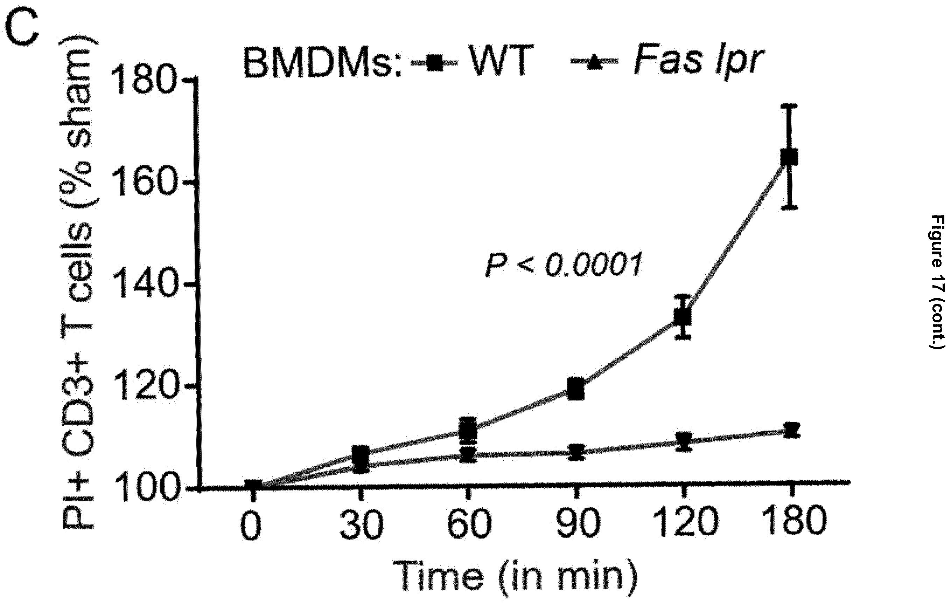

**Figure 17** shows that IL-1β increases FasL expression on monocytes. **Figure 17A** shows the analysis of the mean fluorescence intensity (MFI) for FasL (geometric mean of APC fluorescence) on CD11b⁺ monocytes of WT mice (anti-IL-1β or isotyp control-treated) and Casp1 mice 18 h after sham or stroke surgery. The IL-1β neutralization as well as caspase-1 deficiency significantly decreased the FasL MFI on monocytes after stroke to levels of sham-operated mice (n = 7-8 per group; H-test). **Figure 17B** shows that BMDMs were stimulated with either recombinant IL-1β (rIL-1β) in increasing doses or serum of sham or stroke mice. After changing the medium, T cells were added in fresh medium (allowing no direct contact of T cells to rIL-1β or serum) and 180 minutes later flow cytometry was performed for quantifying FasL expression on monocytes and survival of T cells. High concentrations of rIL-1β and stroke serum both increased the ratio of FasL⁺CD11b⁺ monocytes as well as increased T cell death (n = 6 per group, H-test). **Figure 17C** shows that BMDMs were stimulated with stroke or sham serum, the medium was changed to remove the serum and T cells were added in medium containing propidium iodide (PI). PI uptake in dying T cells was dynamically analyzed by live imaging of WT or *Fas Ipr* T cells in co-culture with WT BMDMs, showing completely blocked cell death for *Fas Ipr* T cells in response to BMDM stimulation. Results are presented as percentage of PI⁺ T cells in stroke serum normalized to sham serum-treated BMDM culture conditions for both T cell genotypes (n = 6 per group, 2-way ANOVA). Shown p value is for difference in T cell genotype.

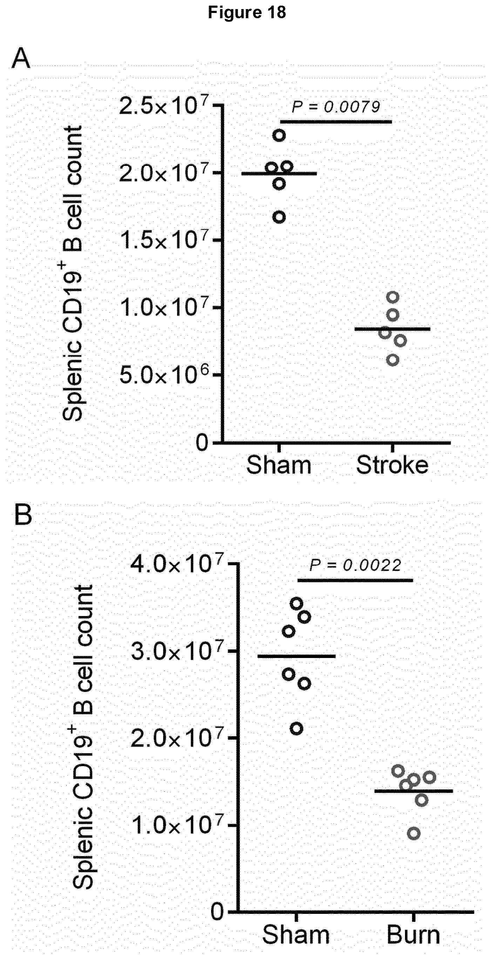

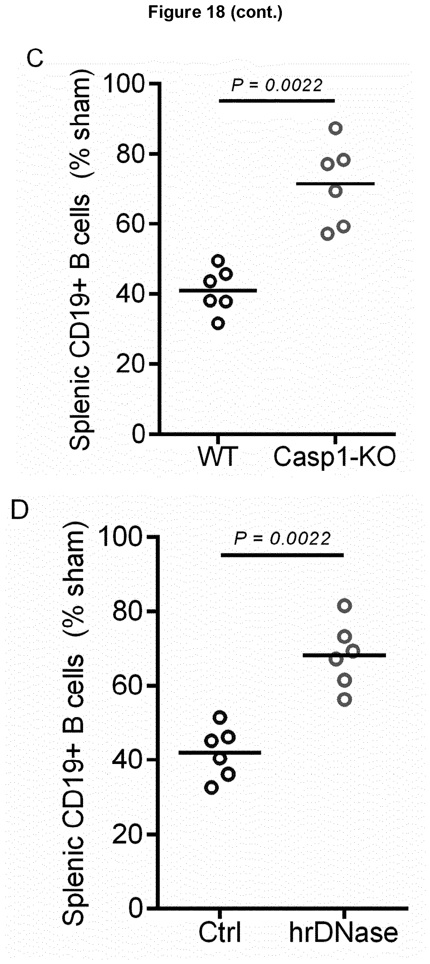

**Figures 18A** and **18B** show flow cytometric (FACS) quantification of splenic B cells at 18 h after experimental stroke (see **Figure 18A**) and 18 h after burn injury (**Figure 18B**) (n = 5-6 per group; U-test). **Figure 18C** shows splenic B cell counts, which were analyzed in caspase-1 deficient (*Casp1*^{-/-}) and wildtype (WT) mice 18 h after sham and stroke surgery (n = 6 per group; U-test), revealing significantly improved B cell survival in *Casp1*^{-/-} mice. **Figure 18D** shows FACS quantification of splenic B cells 18 h after a stroke in control- or hrDNase-treated WT mice. hrDNase treatment significantly reduced monocyte inflammasome activation and increased splenic T cell counts (n = 6 per group; U-test). Data for the intervention groups in Figure 18A to D was normalized to the respective sham group.

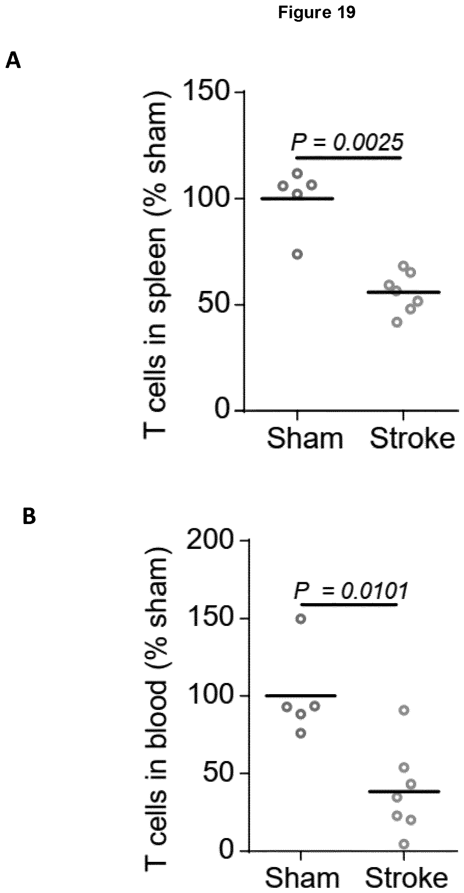

**Figure 19A** shows GF mice undergoing sham or stroke surgery and that they were sacrificed 18h after operation. CD3+ T cells from spleen were quantified by FACS (n=5-7 per group; U-test) **Figure19B** shows GF mice undergoing sham or stroke surgery and that they were sacrificed 18h after operation. CD3+ T cells from blood were quantified by FACS (n=5-7 per group; U-test).

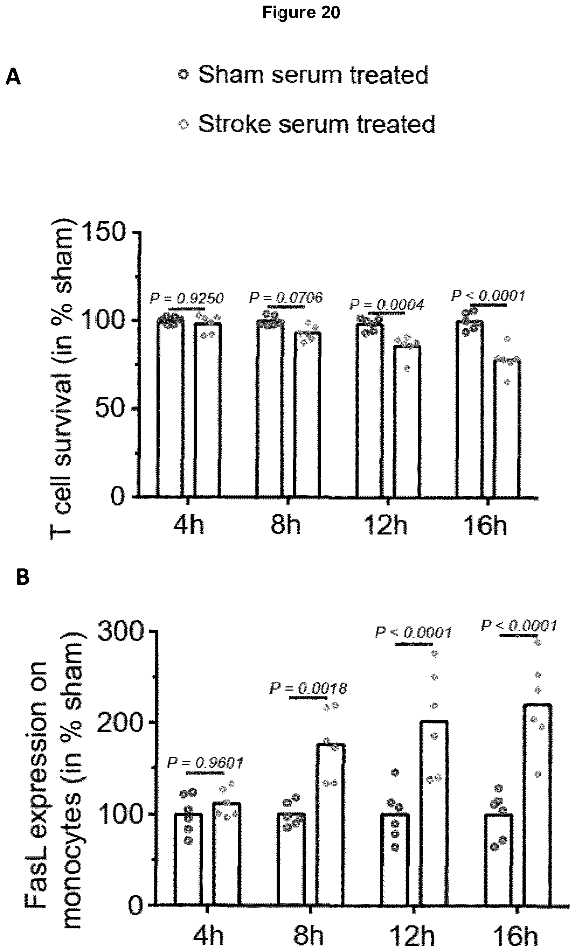

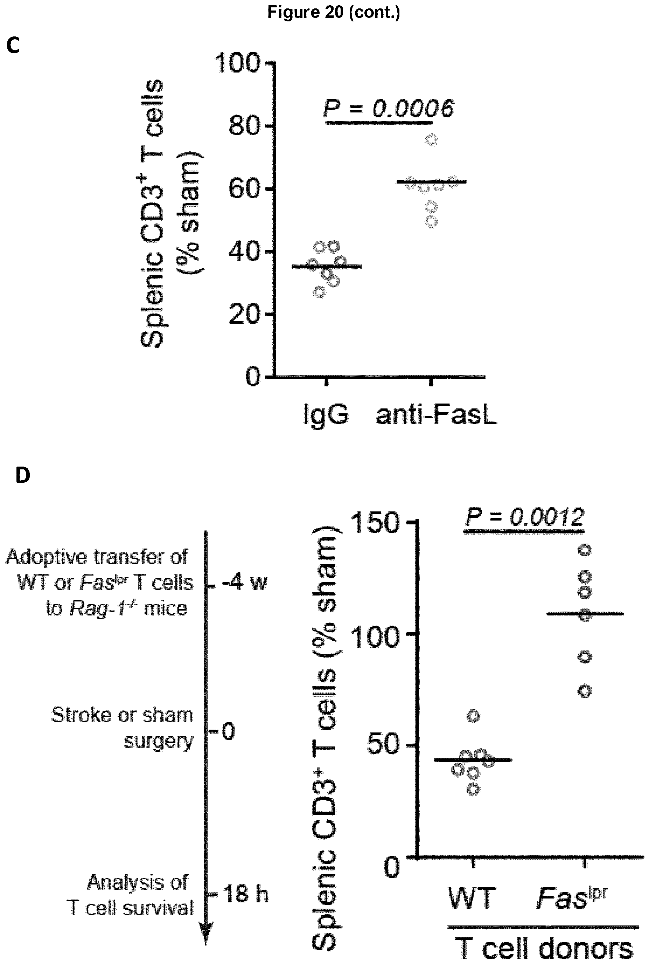

**Figure 20A** shows that whole splenocytes were cultured and treated with sham or stroke serum. For every time point after start of in vitro serum stimulation (4-16h) T cells were analyzed by FACS (n=6 per group; H-test). **Figure20B** shows that whole splenocytes were cultured and treated with sham or stroke serum. For every time point after start of in vitro serum stimulation (4-16h) FasL expression on CD11b+ splenocytes. **Figure 20C** shows FACS analysis of splenic CD3+ T cells in WT mice treated with isotypecontrol (IgG) or FasL-specific neutralizing antibodies and sacrificed for analysis 18h after stroke or sham surgery (n= 7 per group; U test). **Figure 20D** shows *Rag-1*-/- mice received adoptive transfer of WT or *Fas*lpr T cells. 4 weeks later these mice underwent sham or stroke surgery and splenic T cell counts were analyzed 18h later (n= 6-7 per group; U-test).

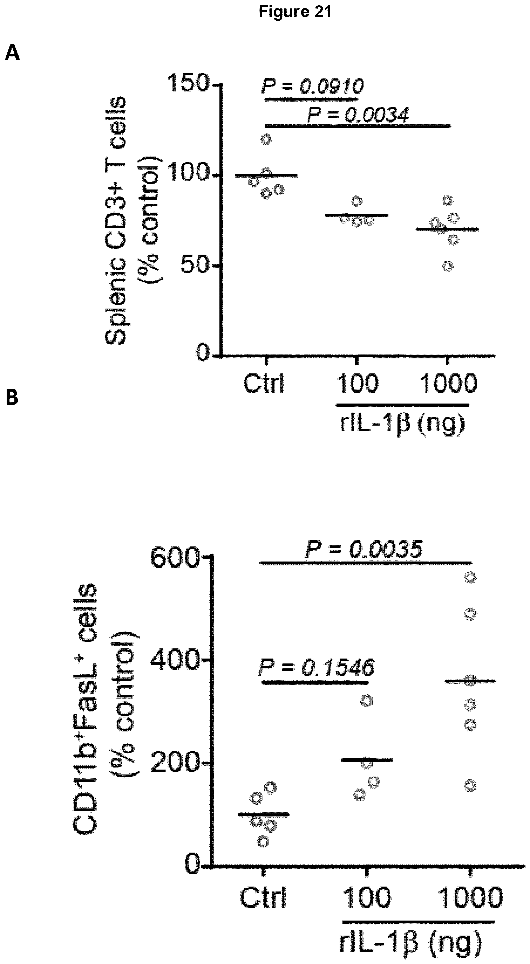

**Figure 21A** shows WT mice received rIL-1b (100 or 1000 ng) as a single i.p. injection and were sacrificed 6h later. FACS analysis revealed a dose-dependent increase in T cell death in the spleen (n= 4-6 per group; H-test). **Figure 21B** shows WT mice received rIL-1b (100 or 1000 ng) as a single i.p. injection and were sacrificed 6h later. FACS analysis revealed a dose-dependent increase in associated monocytic FasL expression in the spleen (n= 4-6 per group; H-test).

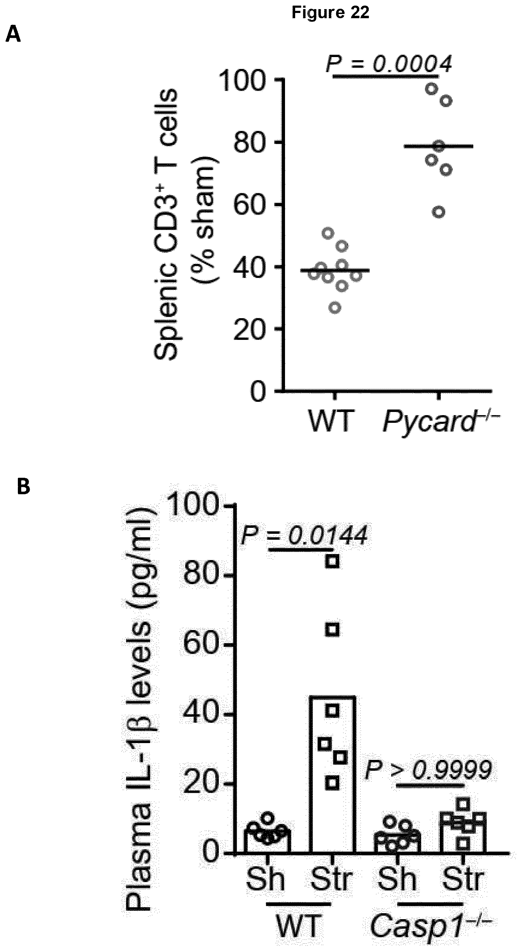

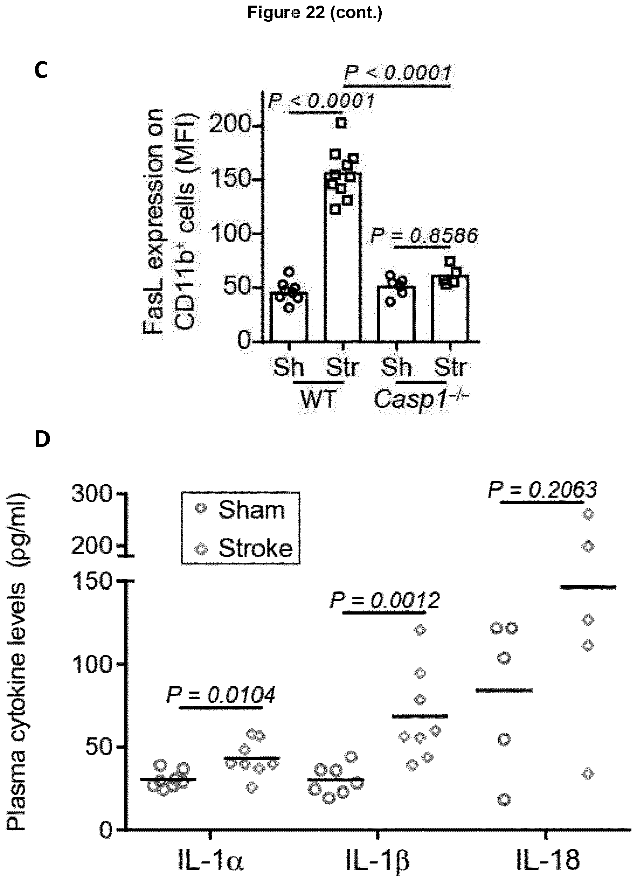

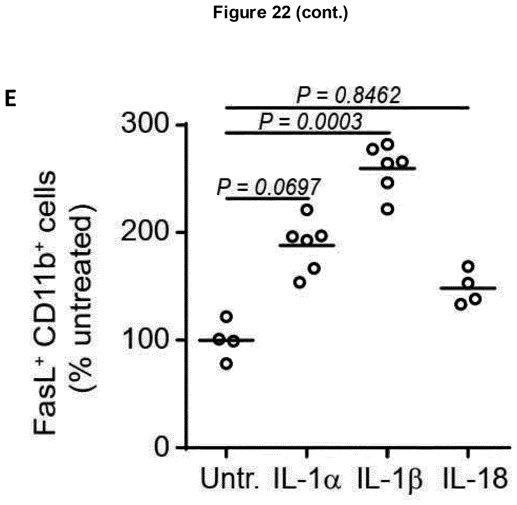

**Figure 22A** shows ASC-deficient (*Pycard-*/*-*) and WT mice undergoing a stroke or sham surgery and that they were euthanized 18h after surgical procedure. Splenic T cells were analyzed by FACS (n=5 per group; U-test). **Figure 22B** shows that plasma of WT and *Gasp1-*/*-* mice was collected 18h after sham or stroke surgery. IL-1β levels in the plasma were acquired by ELISA (n=6 per group; H-test). **Figure 22C** shows analysis of the mean fluorescent intensity (MFI) for FasL (geometric mean of APC fluorescence) on CD11b+ monocytes of WT and *Casp1-*/*-* mice 18h after sham or stroke surgery (n= 5-10 per group; H-test). **Figure 22D** shows that plasma of mice was collected 4h after sham or stroke surgery. IL-1a, IL-1b and IL-18 levels were acquired by ELISA (n= 5-8 per group, U-test per individual cytokine). **Figure 22E** shows that BMDMs were treated with 100 ng of either cytokine for 4h. FasL+ expression was acquired and normalized to the untreated (Untr.) control (n= 4-6 per group; H-test).

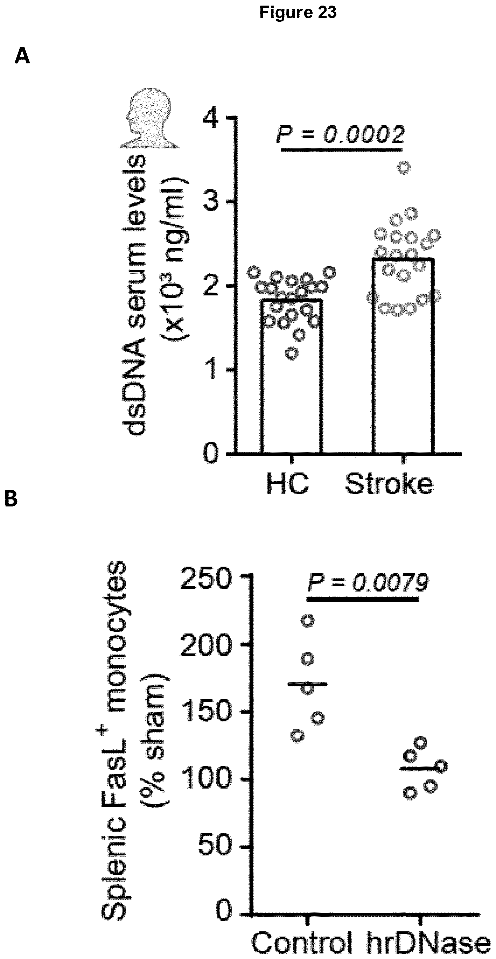

**Figure 23A** shows that dsDNA levels were analyzed in serum of stroke patients in comparison to age-matched healthy control patients (n=20 per group; U-test). **Figure 23B** shows that Cell-free dsDNA was therapeutically degraded by *i.p.* administration of hrDNase after stroke. hrDNase treatment significantly reduced monocyte inflammasome activation as measured by reduced FasL expression on monocytes (E) (n=5-7 per group; U-test).

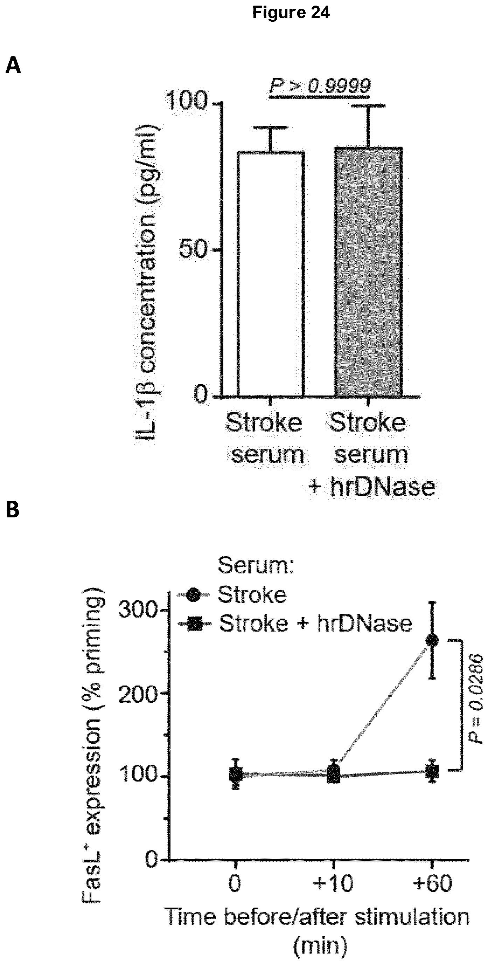

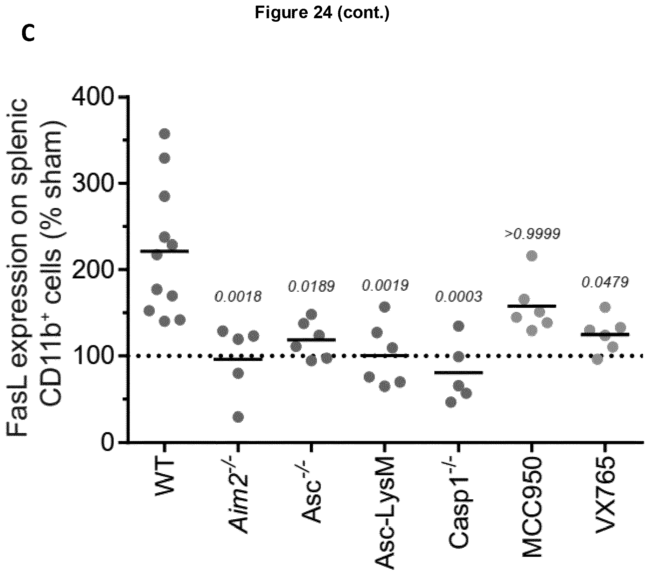

**Figure 24A** shows that BMDMs were stimulated with serum (± hrDNase) from stroke mice. IL-1β concentrations did not differ between the two stroke serum groups. **Figure 24B** shows that BMDMs were stimulated for 10 minutes with the post-stroke serum (± hrDNase) and FasL expression of the BMDMs was aquired before stimulation, 10 and 60 minutes after the stimulation by FACS (n=4 per group, U-test). **Figure 24C** shows that FasL expression on splenic CD11b+ cells were analyzed 18h after stroke in WT mice, the indicated genetic inflammasome knockout models and the pharmacological inflammasome inhibition using MCC950 (NLRP3 inhibitor) and VX765 (Caspase-1 inhibitor). H-test; p-values (post-hoc test) in comparison to WT group.

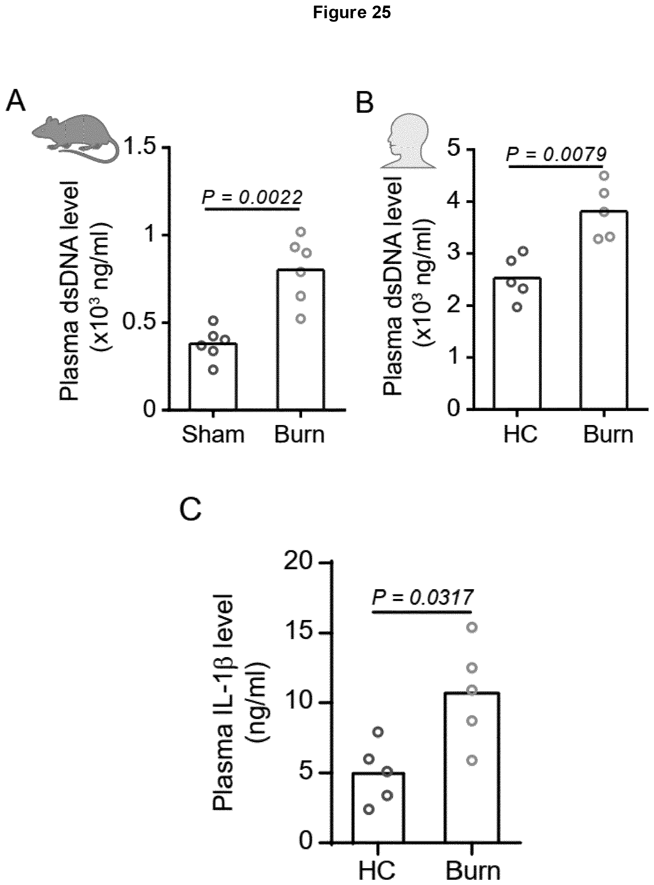

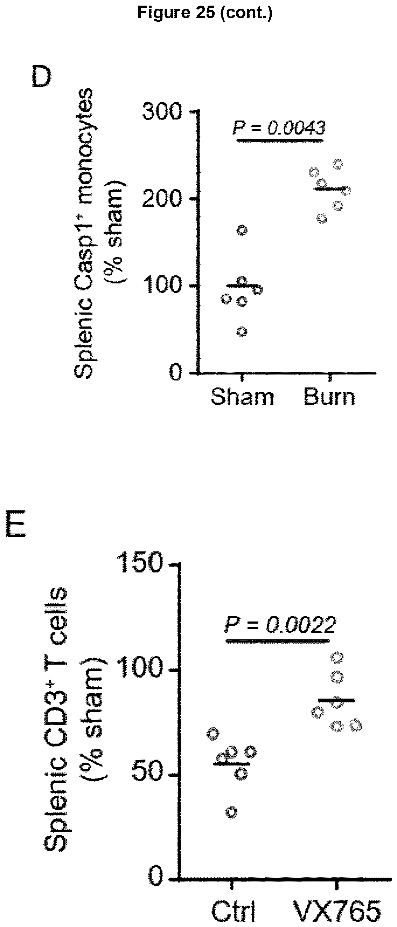

**Figure 25A** shows that plasma dsDNA levels of burn injury mice are increased compared to mice which underwent a control surgery (n=6 per group; U-test). **Figures 25B** and C show that plasma of burn injury patients show significantly increased dsDNA levels (**B**) and IL-1β (**C**) compared to age-matched healthy controls (HC) (n=5 per group; U-test). **Figures 25D** and E show that mice underwent an experimental burn injury or control surgery and were euthanized 18h after surgery. Splenic caspase-1 activity (**D**) and T cell numbers, from burn injury mice treated with control or VX765 treatment (**E**) were analyzed by FACS (n=6 per group; U-test).

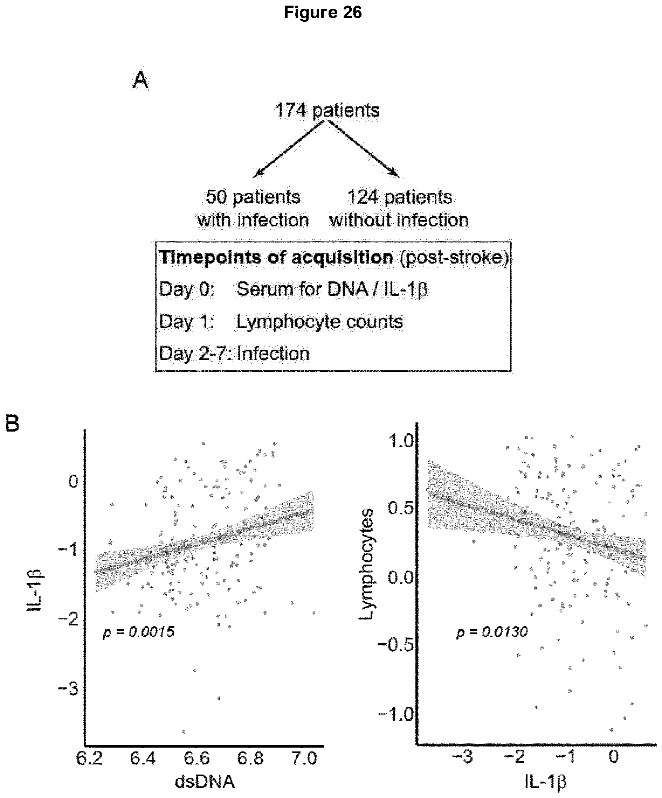

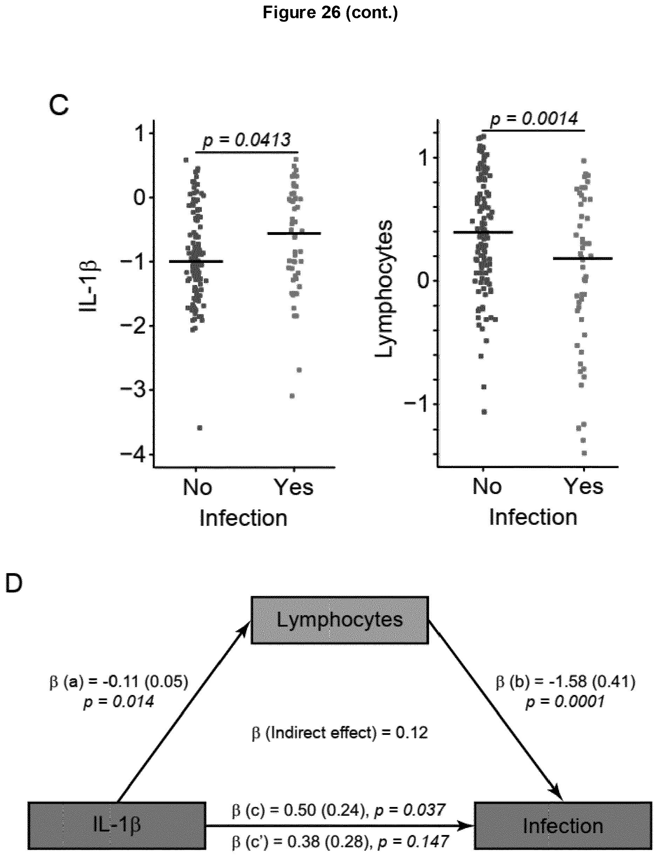

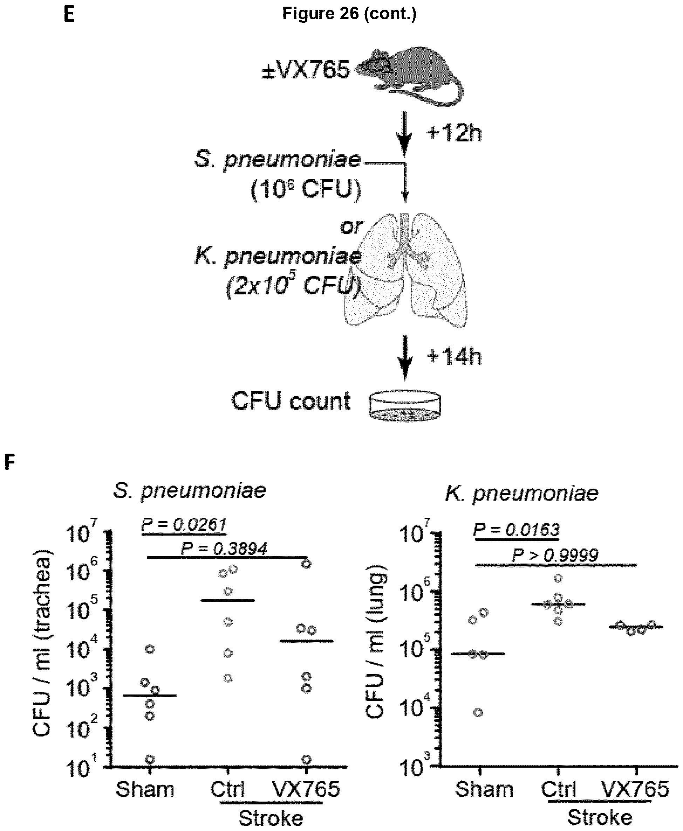

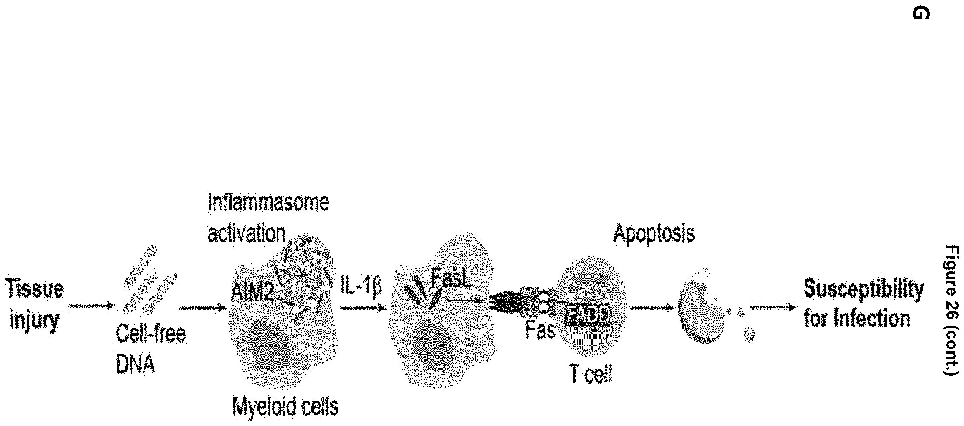

**Figure 26A** shows schematic description of patient characteristics and sequential analysis of acute mediators at d0 and d1 and subacute infections (d2-d7) after stroke. **Figure 26B** shows Left: Levels of dsDNA and IL-1b upon hospital admission were significantly associated in univariate linear regression analysis (R2=0.052). Right: Levels of IL-1b upon admission were negatively associated with lymphocyte counts at d1 in a multivariable linear regression model adjusting for age and sex (R2=0.16). The linear fit (dashed line) and 95% confidence intervals are shown in color. **Figure 26C** shows that IL-1b levels were significantly increased (left) and lymphocyte counts significantly decreased (right) in patients with subsequent infections (n=50) during the subacute phase (d2-7) after stroke compared to patients without infections (n=124). Multivariable linear regression model adjusting for age and sex. **Figure 26D** shows a path diagram of the mediation model including all 174 patients showing full mediation of IL-1b effects on infection via reduction of blood lymphocyte counts. C and c' indicate beta values for the direct effect without or with inclusion of lymphocyte counts in the model, respectively. **Figures 26E** and F show that mice received VX765 or control treatment and underwent stroke or sham surgery. 12h after the surgery they were intranasally inoculated with 106 CFU of *S. pneumoniae* or 2x105 *K. pneumoniae*, 14h later the CFU burden in the respiratory tract (*S.pneumoniae*: trachea; *K pneumoniae*: lung) was determined. **Figure 26G** shows a schematic overview of the proposed mechanism.

### DETAILED DESCRIPTION OF THE INVENTION

The present invention provides a composition comprising a DNA-degrading enzyme for use in a method of reducing the activation of a cell-free (cf) DNA sensing AIM2-inflammasome in myeloid cells of a subject in need thereof, wherein said DNA-degrading enzyme is DNAse !. Also disclosed herein is a composition comprising a DNA-degrading enzyme for use in a method for the treatment of post sterile traumatic immunosuppression.

As used within the context of the present invention, the term "DNA-degrading enzyme" means any enzyme that is able to degrade DNA into its individual nucleotide components. Various events may cause the degradation of DNA into nucleotides, e.g. DNA degradation is one of the final consequences of activation of the apoptotic cascade, and can be measured by quantification of free 3'-hydroxyl groups in tissue sections. If DNA is not properly degraded, this may cause various diseases.

The composition as further disclosed herein is for use in a method for the treatment of a wide variety of different diseases and disorders characterized by the post sterile traumatic immunosuppression. The invention envisages the composition to be for use in a method for the treatment of a subject in need thereof. The subject is typically a mammal, e.g., a human. In some embodiments the subject is a non-human animal that serves as a model for a disease or disorder that affects humans. The animal model may be used, e.g., in preclinical studies, e.g., to assess efficacy and/or to determine a suitable dose. In some embodiments, inventive compositions may be used prophylactically, e.g., may be used for a subject who does not exhibit signs or symptoms of the disease or disorder (but may be at increased risk of developing the immunosuppression or is expected to develop the immunosuppression). Thus, the term "for the treatment" as used herein may comprise "for the prevention" as well. An inventive composition as it is further disclosed herein is for use in a method of treatment of a subject who has developed one or more signs or symptoms of immunosuppression, e.g., the subject has been diagnosed as having immunosuppression. It is preferred that the composition for use of the present invention is administered to the subject in need thereof in a therapeutically effective amount. By "therapeutically effective amount" is meant an amount of the composition of the present invention that elicits a desired therapeutic effect. The exact amount dose will depend on the purpose of the treatment, and will be ascertainable by one skilled in the art using known techniques. As is known in the art and described above, adjustments for age, body weight, general health, sex, diet, drug interaction and the severity of the condition may be necessary, and will be ascertainable with routine experimentation by those skilled in the art. Further, it is also disclosed herein a method of treating post sterile traumatic immunosuppression as defined elsewhere herein, the method comprising administering a therapeutically effective amount of a composition comprising a DNA-degrading enzyme to a subject in need thereof as defined elsewhere herein. In addition, also disclosed herein is the use of a composition comprising a DNA-degrading enzyme for the manufacture of a medicament for the treatment of post sterile traumatic immunosuppression. Each definition made herein may also be applicable to the method of treatment and the Swiss type format.

The term "post sterile traumatic immunosuppression" refers to the medical condition which is known to a person skilled in the art (see f.e. Islam et al. Sterile post-traumatic immunosuppression", *Clin Transl Immunology* (2016)*)* Said term is encompassed by the term "immunosuppression after acute tissue injury" as it is also used herein. Thus, the definitions which apply to the term "immunosuppression after acute tissue injury" also apply to the term "post sterile traumatic immunosuppression" and *vice versa.* The term "immunosuppression after acute tissue injury", as used herein, may be used synonymously with the terms "immune suppression after acute tissue injury", "immune changes after acute tissue injury", "immune alterations after acute tissue injury", and "systemic immune consequences after acute tissue injury". These terms may also encompass "immunosuppression after sterile tissue damage / injury". Preferably, said term(s) encompass(es) the medical condition "post sterile traumatic immunosuppression". "Sterile trauma(tic)" refers to tissue damage / injury devoid of primary wound infection. Thus, "sterile trauma(tic)" may include "sterile tissue injury" or "sterile tissue damage" in the absence of microbial infection. Inflammation following sterile trauma without exposure to microbial pathogens is termed "sterile inflammation": Immunosuppression followed by this sterile inflammation is termed as "sterile immunosuppression". Thus, "post sterile traumatic immunosuppression" is an immunosuppression after an inflammation following sterile trauma, the latter being caused without exposure to microbial pathogens. Indeed, it is further disclosed that due to T cell apoptosis, an immunosuppression occurs. This predisposes patients with local tissue injuries / tissue damages to systemic infections as defined elsewhere herein, which may be a major cause of death after such injuries / damages. Without being bound by theory, it is assumed that DNA, e.g. nuclear DNA and/or mtDNA causes the native immune system to trigger apoptosis in T cells due to the interaction of cells of the native immune system with cells of the adaptive immune system, e.g. lymphocytes, preferably T cells via apoptosis-inducing receptor/ligand interactions. This has been proven by the present invention as described elsewhere herein. Accordingly, the term "post sterile traumatic immunosuppression" preferably encompasses "post sterile traumatic lymphopenia", more preferably "post sterile traumatic T cell cytopenia".

As used herein, the term "immunosuppression" means any form of a reduction of the activation or efficacy of the immune system. Thus, immunosuppression is the suppression of the endogenous defense system. It refers to a process of repressing the immunological activity of the humoral and/or cellular immune system. This can be an undesirable consequence of an effect from the inanimate environment, of an infection, of a malignant suffering, of a disease caused by another condition, of a mental or physical overload, or due to an undesired consequence of a medical diagnosis or a consequence of a medical treatment. Some portions of the immune system itself have immunosuppressive effects on other parts of the immune system, and immunosuppression may occur as an adverse reaction to treatment of other conditions. The immunosuppression may comprise decreased capacity to neutralize external organisms, which may result in repeated, more severe, or prolonged infections, as well as an increased susceptibility to cancer development. As used herein , an immunosuppression may be present, when one or more of the following cell types are suppressed with regard to their activity or reduced in their cell count, consisting of myeloid cells (including granulocytes, monocytes, macrophages, dendritic cells and mast cells) or lymphocytes (including T cells, B cells, Plasma cells, NK cells and NKT cells) or wherein the subject diagnosed with a suppressed immunosystem may develop infections by opportunistic pathogens (for example *Pneumocystis* or cytomegalovirus). The presence of the immunosuppression may be investigated in reference to a state which does not comprise an acute tissue injury / damage as defined herein or an immunosuppression of other cause.

"Acute tissue injury", (also called "acute tissue damage") as used within the context of the present invention, means an injury / damage with a sudden onset, for example being characterized by cell death concerning one or more organs in a certain time range, e.g. within 24 hours. Such an injury is not limited to an organ or any noxae. During such an acute tissue injury an organ or a part thereof can be affected, where cell function and integrity is lost within less than 24 h due to an insult. This insult can be ischemia (lack of blood flow) to the organ, a mechanical tissue trauma, the effect of a toxic agent or a thermal injury, for example. Examples for acute tissue injury are stroke, myocardial infarction, trauma, ischemia-reperfusion injuries to limbs or kidneys, burn injury or pharmacological toxicities such as acute liver failure due to various medication overuses. Acute tissue injuries include local, tissue-specific inflammatory and repair mechanisms that contribute to wound healing and scar formation. Besides these localized tissue-specific effects, acute tissue injuries also have a substantial and uniform impact on systemic immunity. The initial incidence of tissue damage acutely induces a pronounced local immune response and systemic proinflammatory activation, which is characterized by a rapid increase in circulating leukocytes pro-inflammatory cytokine levels (Offner *et al.*, 2006; Emsley *et al.*, 2003). After this early activation has resolved, a subsequent immune deficient phase follows. This immune deficiency is characterized by increased levels of circulating immature monocytes and systemic lymphopenia (Offner *et al.*, 2006; Howard *et al.,* 1974), which predisposes patients with local tissue injuries to systemic bacterial infections. In fact, infections are a major cause of death after acute tissue injuries such as stroke, trauma and burn injury.

The term "acute" as used within the term "acute tissue injury" as defined above, is a term, which may be understood in contrast to chronic diseases, leading to tissue injuries. Chronic diseases in general are slowly progressing, while the definition of "slowly progressing" depends on the specific disease entity, but may be generally over several weeks or months. For example, chronic vascular impairment may be in contrast to an acute ischemic injury to the brain (stroke) or the heart (myocardial infarction). An acute disease onset is clinically defined by the rapid onset of clinical symptoms. Acute diseases - in contrast to a chronic disease progression - are often more severe and require urgent medical attention. In some cases, an acute condition, e.g. a myocardial infarction or stroke, might lead to chronic conditions, such as chronic heart failure or immobility, respectively.

Also disclosed herein is the composition for such disclosed use, wherein an immunoactivation before the immunosuppression after acute tissue injury occurs. Immunoactivation in general comprises all forms of activation of the immune system and the subsequent immune response. As used herein, "immunoactivation", "immune activation" or "activation of the immune system" refers to an increase in the number and/or function of immune system cells, such as lymphocytes or myeloid cells, and/or an increase in humoral function of the immune system, involved in plasma cell and antibody production along with cytokine production. An immunoactivation may be, for example, present, when one or more of the following cell/ cells is/ are activated with regard to their activity/ activities, which is/ are selected from the group consisting of myeloid cells (including granulocytes, monocytes, macrophages, dendritic cells and mast cells) or lymphocytes (including T cells, B cells, plasma cells, NK cells and NKT cells) or wherein the subject diagnosed with an activated immunosystem may have the following clinical symptoms or parameters: increased blood cytokine levels, increased number of immune cells as specified above, increase in blood concentration of acute phase proteins (including C-reactive protein), fever and clinical signs of cytokine-induced sickness behavior (reduced appetite, apathy, sleeping disorder, reduced motivation and depressed mood). The presence of an immunoactivation may be investigated in reference to a healthy control population or the presence of the immunoactivation may be investigated in reference to a state which does not comprise an acute tissue injury as defined herein or an immunoactivation of other cause.

Also disclosed herein is the composition for such disclosed use, wherein the immunosuppression after acute tissue injury is characterized by lymphocyte death. "immunosuppression after acute tissue injury" can be used synonymously to "lymphocyte death". A lymphocyte is one of the subtypes of a white blood cell in a vertebrate's immune system. Lymphocytes include natural killer cells (which function in cell-mediated, cytotoxic innate immunity), T cells (for cell-mediated, cytotoxic adaptive immunity), and B cells (for humoral, antibody-driven adaptive immunity), which is well known to a person skilled in the art. They are the main type of cell found in lymph, which prompted the name "lymphocyte". The term "lymphocyte death", as used herein, means the drop in or reduction of the lymphocyte count, e.g. in the blood of a subject, which can be, for example, prompted by lymphocyte apoptosis. The "lymphocyte death" may also comprise "lymphopenia", which is the reduction in the numbers of lymphocytes in the blood. Lymphocyte death may also occur by both death receptor and mitochondrial-mediated apoptosis, so that there may be multiple triggers for lymphocyte death. Thus, also disclosed herein is the composition for such disclosed use, wherein the lymphocyte death is caused by apoptosis. Also disclosed herein is the composition, wherein the lymphocyte death comprises or is characterized by or is caused by T cell death. Also disclosed herein is the composition for such disclosed use, wherein the lymphocyte death comprises or is characterized by or is caused by T cell cytopenia. Cytopenia is a reduction in the number of mature blood cells. Also disclosed herein is the composition for such disclosed use, wherein the T cell cytopenia or the T cell death is caused by T cell apoptosis. "Apoptosis" is the programmed cell death that occurs in multicellular organisms, which is known by a person skilled in the art. Also disclosed herein is the composition, wherein the lymphocyte death comprises or is characterized by or is caused by B cell death. In this regard, it is referred to Figure 18 of the present application. Also disclosed herein is the composition for such disclosed use, wherein the lymphocyte death comprises or is characterized by or is caused by B cell cytopenia. Also disclosed herein is the composition for such disclosed use, wherein the B cell cytopenia or the B cell death is caused by B cell apoptosis.

Also disclosed herein is the composition for such disclosed use, wherein the immunosuppression after acute tissue injury is associated with systemic immune response syndrome (SIRS). "Systemic immune response syndrome" (SIRS) is an inflammatory state affecting the whole body. It is the body's response to an infectious or non-infectious insult. Although SIRS may refer to an "inflammatory" response, it actually has pro- and anti-inflammatory components. According to the foundings of the inventors, immunosuppression may contribute to SIRS development or accompanies the SIRS symptomes, while SIRS is characterized by the clinical parameters of dysregulated body temperature, elevated heart rate, tachypnea, a decreased or increased number of blood leukocytes and a high number of immature innate immune cells. For example, manifestations of SIRS for adults may include, but are not limited to, a body temperature less than 36 C or greater than 38 C, a heart rate greater than 90 beats per minute, a tachypnea (high respiratory rate) with greater than 20 breaths per minute or an arterial partial pressure of carbon dioxide less than 4.3 kPa and a white blood cell count less than 4000 cells/mm³ (4 × 10⁹ cells/L) or greater than 12,000 cells/mm³ (12 × 10⁹ cells/L) or the presence of greater than 10 % immature neutrophils. When two or more of these criteria are met with or without evidence of infection, patients may be diagnosed with "SIRS". Patients with SIRS and acute organ dysfunction may be termed "severe SIRS".

Also disclosed herein is the composition for such disclosed use, wherein the immunosuppression is triggered by acute tissue injury. The "acute tissue injury" is used in this embodiment as defined herein above. The terms "triggers", "triggered" or "triggering", as used herein, means to cause something to start leading to a specific outcome or condition. For example, the inventors of the present invention have found that a clinical condition like an acute tissue injury leads to or results in immunosuppression, which can be detected in the subject who has experienced the acute tissue injury.

According to one embodiment of the composition for use of the present invention, the acute tissue injury is triggered by a physical, chemical, or metabolic noxious stimulus. The stimulus is any kind of change in substances or in happenings, occuring in the surrounding of a living thing that bring about any kind of response from it. The term "physical stimulus", as used within the context of the present invention, means such a stimulus that directly affects one of the five senses. A chemical stimulus might be a stimulus that is caused by a chemical (liquid, gaseous, or solid) substance that is capable of evoking a response, e.g. in a subject exposed to said chemical stimulus. A noxious stimulus is an actually or potentially tissue damaging event. Noxious stimuli can either be mechanical (e.g. pinching or other tissue deformation), chemical (e.g. exposure to acid or irritant), or thermal (e.g. high or low temperatures).

In one specific embodiment of the composition for use of the present invention, the acute tissue injury is selected from stroke, myocardial infarction, haemorrhagic shock, ischemia, ischemia reperfusion injury, chronic inhalation of irritants (e.g. asbestos, silica), atherosclerosis, gout, pseudogout, trauma, non-penetrating polytrauma (multiple bone fractures), and thermal trauma.

Stroke is known to a person skilled in the art to be a medical condition with a sudden onset due to a vascular injury to the brain. There may be two main types of stroke: ischemic, due to lack of blood flow, and hemorrhagic, due to bleeding.

The term "myocardial infarction", as used within the context of the present invention, refers to tissue death (infarction) of the heart muscle (myocardium) caused by ischaemia, that is the lack of oxygen delivery to myocardial tissue. It is a type of acute coronary syndrome, which describes a sudden or short-term change in symptoms related to blood flow to the heart.

The term "haemorrhagic shock", as used within the context of the present invention, means a shock resulting from reduction of the volume of blood in the body due to hemorrhage. It is also known as a hypovolemic shock resulting from acute hemorrhage, characterized by hypotension, tachycardia, pale, cold, and clammy skin, and oliguria.

The term "ischemia" or "ischaemia", as used within the context of the present invention, is a restriction in blood supply to tissues, causing a shortage of oxygen that is needed for cellular metabolism (to keep tissue alive). Ischemia is generally caused by problems with blood vessels, with resultant damage to or dysfunction of tissue.

The term "ischemia reperfusion injury", also known as reperfusion injury or reoxygenation injury, refers to tissue damage caused when blood supply returns to tissue (re- + perfusion) after a period of ischemia or lack of oxygen (anoxia or hypoxia). The absence of oxygen and nutrients from blood during the ischemic period creates a condition, in which the restoration of circulation results in inflammation and oxidative damage through the induction of oxidative stress rather than (or along with) restoration of normal function.

The term "chronic inhalation of irritants", as used within the context of the present invention, means, for example, chronic exposure to asbestos or tobacco, which may result when being inhaled, into many airway diseases. Thus, many of the irritants can cause harm to the lungs or other parts of the airways, leading to a range of different inhalation disorders. However, possible is also that the chronic inhalation of irritants comprises irritant gas inhalation injuries. Irritant gases are those which, when being inhaled, dissolve in the water of the respiratory tract mucosa and cause an inflammatory response, usually due to the release of acidic or alkaline radicals. Irritant gas exposures predominantly affect the airways, causing tracheitis, bronchitis, and bronchiolitis. Other inhaled agents may be directly toxic (e.g., cyanide or carbon monoxide) or may cause harm simply by displacing oxygen and causing asphyxia (e.g., methane or carbon dioxide).

The term "atherosclerosis", as used within the context of the present invention, refers to a process of progressive thickening and hardening of the walls of medium-sized and large arteries as a result of fat deposits on their inner lining. Risk factors for atherosclerosis include high blood pressure (hypertension), smoking, diabetes and a genetic family history of atherosclerotic disease. Atherosclerosis can cause a heart attack if it completely blocks the blood flow in the heart (coronary) arteries. It can cause a stroke if it completely blocks the brain (carotid) arteries. Atherosclerosis can also occur in the arteries of the neck, kidneys, thighs, and arms, causing kidney failure or gangrene and amputation.

The term "gout", as used within the context of the present invention, refers to a metabolic disease marked by a painful inflammation of the joints, deposits of urates in and around the joints, and usually an excessive amount of uric acid in the blood. The tendency to develop gout and elevated blood uric acid level (hyperuricemia) is often inherited and can be promoted by obesity, weight gain, alcohol intake, high blood pressure, abnormal kidney function, and drugs. The most reliable diagnostic test for gout is the identification of crystals in joints, body fluids and tissues.

The term "pseudogout", as used within the context of the present invention, instead refers to an arthritic condition, which resembles gout, but is characterized by the deposition of crystalline salts other than urates in and around the joints. Specifically, it is characterized by an inflammation of the joints that is caused by deposits of calcium pyrophosphate crystals, resulting in arthritis, most commonly of the knees, wrists, shoulders, hips, and ankles. Pseudogout has sometimes been referred to as calcium pyrophosphate deposition disease or CPPD. Pseudogout is clearly related to aging as it is more common in the elderly and is associated with degenerative arthritis. Acute attacks of the arthritis of pseudogout can be caused by dehydration.

The term "trauma", as used within the context of the present invention, means any injury caused by a mechanical or physical agent. The term "non-penetrating polytrauma", as used within the context of the present invention, means there may be an impact, but the skin is not necessarily wounded. In contrast thereto, a penetrating polytrauma is an injury that occurs when an object enters a tissue of the body and creates an open wound. Conversely, the term "thermal trauma", as used within the context of the present invention, may include any burn-related injury as well as any cold/freeze-related skin injury that can potentially lead to serious outcomes. There are various causes of thermal trauma, including fire, radiant heat, radiation, chemical, or electrical contact that can affect a person in many ways based on factors from anatomical and physiological factors.

Also disclosed herein is the composition for such disclosed use, wherein the immunosuppression after acute tissue injury is associated with a secondary infectious disease. Such a secondary infectious disease is a disease that may occur as a result of another disease, herein, preferably, as a result of the acute tissue injury. Such may be, for example pneumonia (infection of the lung), urinary tract infections or sepsis. The infections may be caused by bacteria, viruses or fungi.

Also disclosed herein is the composition for such disclosed use, wherein the DNA-degrading enzyme is a nuclease. The term "nuclease", as used herein, refers to any of various enzymes that promote the hydrolysis of nucleic acids. Specifically, a nuclease (also archaically known as nucleodepolymerase or polynucleotidase) is an enzyme capable of cleaving the phosphodiester bonds between nucleotides of nucleic acids. Nucleases variously effect single and double stranded breaks in their target molecules. In living organisms, they are essential machineries for many aspects of DNA repair. Defects in certain nucleases can cause genetic instability or immunodeficiency.

Also disclosed herein is the composition for such disclosed use, wherein the nuclease is an exonuclease or endonuclease. Exonucleases are enzymes that work by cleaving nucleotides one at a time from the end (exo) of a polynucleotide chain. A hydrolyzing reaction that breaks phosphodiester bonds at either the 3'- or the 5'-end occurs. Eukaryotes and prokaryotes have three types of exonucleases involved in the normal turnover of mRNA: 1. 5' to 3'-exonuclease (Xrn1), which is a dependent decapping protein; 2. 3'- to 5'-exonuclease, an independent protein; and 3. poly(A)-specific 3'- to 5'-exonuclease. Endonucleases instead are enzymes that cleave the phosphodiester bond within a polynucleotide chain. Some, such as deoxyribonuclease I, cut DNA relatively non-specifically (without regard to sequence), while many, typically called restriction endonucleases or restriction enzymes, cleave only at very specific nucleotide sequences. Thus, endonucleases differ from exonucleases, which, as described above, cleave the ends of recognition sequences instead of the middle (endo) portion. Some enzymes known as "exo-endonucleases", however, are not limited to either nuclease function, displaying qualities that are both endo- and exo-like.

Also disclosed herein is the composition for such disclosed use, wherein the endonuclease is a deoxyribonuclease, preferably DNase I. According to the composition of the present invention for the use of the invention, the DNA-degrading enzyme is DNAse !. DNAse I is a nuclease that cleaves DNA preferentially at phosphodiester linkages adjacent to a pyrimidine nucleotide, yielding 5'-phosphate-terminated polynucleotides with a free hydroxyl group on position 3', on average producing tetra nucleotides. It acts on single-stranded DNA, double-stranded DNA, and chromatin.

Also disclosed herein is the composition for such disclosed use, wherein the nuclease is administered after the acute tissue injury and/or in the course of the treatment of the acute tissue injury. In one further embodiment of the composition for use of the present invention, the DNAse I is administered after the acute tissue injury and/or in the course of the treatment of the acute tissue injury.

Also disclosed herein is the composition for such disclosed use, wherein the nuclease is administered parenterally, preferably intravenously or by inhalation. According to one further embodiment of the composition for use of the present invention, the DNAse I is administered parenterally, preferably intravenously or by inhalation.

The term "parenterally", as used within the context of the present invention, refers to an administration route other than through the alimentary canal, such as by subcutaneous, intramuscular, intrasternal, or intravenous injection. Intravenously adminstration means an admistration of a fluid performed or occurred within or entering by way of a vein. By inhalation means the act or an instance of inhaling a substance.

It is noted that as used herein, the singular forms "a", "an" and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein. Additionally, for example, a reference to "a host cell" includes one or more of such host cells, respectively. Similarly, for example, a reference to "methods" or "host cells" includes "a host cell" or "a method", respectively.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

The term "and/or" wherever used herein includes the meaning of "and", "or" and "all or any other combination of the elements connected by said term". For example, A, B and/or C means A, B, C, A+B, A+C, B+C and A+B+C.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

The term "about" or "approximately" as used herein means within 20 %, preferably within 10 %, and more preferably within 5 % of a given value or range. It includes also the concrete number, e.g., about 20 includes 20.

Further, in describing representative embodiments of the present invention, the specification may have presented the method and/or process of the present invention as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims.

A better understanding of the present invention and of its advantages will be gained from the following examples, offered for illustrative purposes only. The examples are not intended to limit the scope of the present invention in any way.

### EXAMPLES OF THE INVENTION

Hereinafter, the present invention is explained in detail through examples. The following examples are intended merely to illustrate the present invention, to which the scope of the present invention is not restricted.

### Material and methods

### Experimental design of animal experiments

All animal experiments were performed in accordance with the guidelines for the use of experimental animals and were approved by the government committee of Upper Bavaria (Regierungspraesidium Oberbayern, #175-2013, Rhineland Palatinate Landesuntersuchungsamt Koblenz, #G-19-07-41). Wild type C57BL6/J mice, *Rag-1*^{-/-}(NOD.129S7(B6)-*Rag-1*^{*tm1Mom*/J}), *Fas*^{-/-} (MRL/MpJ-Fas*^{lpr}*/J), *Casp1*^{-/-} (B6N.129S2-*Casp1*^{<*tm1Flv*>}/J) were bred and housed at the animal core facility of the Center for Stroke and Dementia Research (Munich, Germany). The ASC-Citrine reporter mice (B6. Cg-Gt(ROSA)26Sor^{tm1.1(CAG-Pycard/mCitrine*,-CD2)Dtg}/J) and *Fas^{lpr}* (MRL/MpJ-Fas*^{lpr}*/J) were obtained from Jackson Laboratories (Bar Harbor, USA). Aim2^{-/-} mice (Aim2^{<tm1.2Arte>}) where bred at the Institute for Innate Immunity, University Bonn (Germany). *Asc*^{-/-} mice (B6.129S5-Pycard^{tm1Vmd}) and *Pycard*^{-/-} mice (B6.129S5-Pycard^{tm1Vmd}) were bred at the Gene Center of the LMU University Munich (Germany). Monocyte-specific ASC-knockout mice (LysM-Cre x *Asc*^{-/-}*; Lyz2-cre* x *Pycard*^{*fl*/*fl*}) mice were bred at the Institute for Clinical Chemistry and Pathobiochemistry (Technical University Munich, Germany). Cx3Cr1^{GFP/+} mice were purchased from Jackson Laboratory (Bar Harbor, USA) and bred at the animal core facility of Lanzhou University. All mice were housed with free access to food and water at a 12h dark-light cycle.

A priori sample size calculation was based upon the criteria of 1) variance and effect size from previous studies or 2) preliminary pilot experiments performed during the study. Data was excluded from all mice that died during surgery. Detailed exclusion criteria are described below. Animals were randomly assigned to treatment groups and all analyses were performed by investigators blinded to group allocation. All animal experiments were performed and reported in accordance with the ARRIVE guidelines (Kilkenny *et al.,* 2010).

### Transient ischemia-reperfusion stroke model

Mice were anaesthetized with isoflurane delivered in a mixture of 30% O₂ and 70% N₂O. An incision was made between the ear and the eye in order to expose the temporal bone. Mice were placed in supine position, and a laser Doppler probe was affixed to the skull above the middle cerebral artery (MCA) territory. The common carotid artery and left external carotid artery were exposed via midline incision and further isolated and ligated. A 2-mm silicon-coated filament (Doccol) was inserted into the internal carotid artery, advanced gently to the MCA until resistance was felt, and occlusion was confirmed by a corresponding decrease in blood flow (i.e., a decrease in the laser Doppler flow signal by ≥ 80 %. After 60 minutes of occlusion, the animals were re-anesthetized, and the filament was removed. After recovery, the mice were kept in their home cage with *ad libitum* access to water and food. Sham-operated mice received the same surgical procedure, but the filament was removed in lieu of being advanced to the MCA. Body temperature was maintained at 37 °C throughout surgery in all mice via feedback-controlled heating pad. The overall mortality rate of animals subjected to MCA occlusion was approximately 20 %. All animals in the sham group survived the procedure. Exclusion criteria: 1. Insufficient MCA occlusion (a reduction in blood flow to >20% of the baseline value). 2. Death during the surgery. 3. Lack of brain ischemia as quantified post-mortem by histological analysis.

### Germfree (GF) mouse handling

All surgeries, housing and post-operative animal handling were performed under sterile conditions as previously described (Singh *et al.*, 2018). In brief, stroke and sham surgeries have been performed under sterile conditions in a microbiological safety cabinet, animals received sterilized water and irradiated food and animals were kept in sterile gnotocage mini-isolators. All surgical procedures and post-surgical care were otherwise performed as stated above.

### Experimental thermal trauma model

Male C57BI/6J mice (Charles River, Freiburg, Germany), aged 7-8 weeks, received a 35 % total body surface area (TBSA) full thickness scald burn to the back through 10 seconds immersion in 98 °C water under deep anesthesia with 2 % isoflurane and analgesia with 0.1 mg kg⁻¹ buprenorphine. Immediately after burn injury, the mice were resuscitated with 2 ml of lactated Ringer's solution (Baxter, Unterschleißheim, Germany) via i.p. injection as previously described (Hundeshagen *et al.*, 2018; Bohannon *et al.*, 2008; Toliver-Kinsky *et al.*, 2005). Animals in the sham burn group were subjected to identical treatment except for water temperature during immersion being 36 C. Following burn injury or sham burn, mice were singly housed at room temperature (21°C).

### Transient hind limb ischemia-reperfusion injury

Mice were anaesthetized with isoflurane delivered in a mixture of 30 % O₂ and 70 % N₂O. An incision was made between the ear and the eye in order to expose the temporal bone. Mice were placed in supine position, and a laser Doppler probe was affixed to the skull above the middle cerebral artery (MCA) territory. The common carotid artery and left external carotid artery were exposed via midline incision and further isolated and ligated. A 2-mm silicon-coated filament (Doccol) was inserted into the internal carotid artery, advanced gently to the MCA until resistance was felt, and occlusion was confirmed by a corresponding decrease in blood flow (i.e., a decrease in the laser Doppler flow signal by ≥ 80 %. After 60 minutes of occlusion, the animals were re-anesthetized, and the filament was removed. After recovery, the mice were kept in their home cage with *ad libitum* access to water and food. Sham-operated mice received the same surgical procedure, but the filament was removed in lieu of being advanced to the MCA. Body temperature was maintained at 37 °C throughout surgery in all mice via feedback-controlled heating pad. The overall mortality rate of animals subjected to MCA occlusion was approximately 20 %. All animals in the sham group survived the procedure. Exclusion criteria: 1. Insufficient MCA occlusion (a reduction in blood flow to > 20 % of the baseline value). 2. Death during the surgery. 3. Lack of brain ischemia as quantified post-mortem by histological analysis.

### Parabiosis

Parabiosis experiments were performed at the Gansu Key Laboratory in Lanzhou, China. Pairs of weight-matched wild type C57BI6/J and heterozygous Cx3Cr1^{GFP/+} mice were subjected to parabiotic surgery (Wright *et al.*, 2001; Li *et al.*, 2013). Animals were anesthetized by intraperitoneal injection of 20 mg/ml ketamine and 2 mg/ml xylazine. The flanks were shaved and sterilized. An incision from behind the ear to the hip was made on the opposing sides of two mice. Opposing posterior muscles were joined with a 5-0 suture. The scapular region was conjoined then dorsal and ventral skin edges were sutured with a 4-0 suture. Mice were kept at 37 °C in a recovery box until completely recovered from anesthesia. During the first 7 days after surgery, Tylenol is mixed in the food for analgesic purposes. Food and water were provided *ad libitum.* The optimized procedure had a survival rate of ≥ 75 %.

### Intranasal bacterial infection

Pneumococcal infection experiments were performed at the Helmholtz Centre for Infection Research (HZI) in Braunschweig, Germany. Mice were anesthetized with isoflurane delivered in a mixture of 30% O₂ and 70% N₂O. The inoculum (10⁶ CFU of TIGR4, a serotype 4 *S. pneumoniae* strain (Tettelin et al., 2001) or 2×10⁵ CFU of *K.pneumoniae subsp. pneumoniae* (ATCC 43816) *(Wu et al., 2020)* in a total volume of 25µl PBS) was administered with a pipette onto the nostrils of the mice.

### Drug administration

Anti-IL1β: Mice received two injections of antagonizing anti-IL-1β in sterile saline (clone: B122, InVivoMab, BioXcell, US), 1 hour prior to and 1 hour after surgery. Anti-IL-1β or the corresponding IgG control (Armenian hamster IgG, InVivoMab, BioXcell, US) was injected i.p. at a dose of 4 mg kg⁻¹ body weight in a final volume of 200 µl.

Anti-FasL: Mice received two injections of antagonizing anti-FasL in sterile saline (clone: MFL3, InVivoMab, BioXcell, US), 1 hour prior to and 1 hour after surgery. Anti-FasL or the corresponding IgG control (Armenian hamster IgG, InVivoMab, BioXcell, US) was injected i.p. at a dose of 4 mg kg⁻¹ body weight in a final volume of 100 µl.

Human recombinant DNase (hrDNase): 1000 U of human recombinant DNase (Roche, Switzerland) dissolved in incubation 1x buffer (40 mM Tris-HCl, 10 mM NaCl, 6 mM MgCl₂, 1 mM CaCl₂, pH 7.9, diluted in PBS, Roche) was injected i.v. in the tail vein 1 hour after surgery in a final volume of 100 µl. The control group was administered vehicle injections at the same volume, route, and timing as experimental animals.

Caspase-1 inhibitor (VX-765): The caspase-1 inhibitor VX-765 in DMSO dissolved in PBS (Belnacasan, Invivogen, US) was injected i.p. 1 hour prior to surgery at a dose of 100 mg kg⁻¹ body weight at a final volume of 300 µl. The control group was administered vehicle injections at the same volume, route, and timing as experimental animals.

Caspase-8 inhibitor (Z-IETD-FMK) & Caspase-9 inhibitor (Z-LEHD-FMK): The apoptosis inhibitors Z-IETD-FMK and Z-LEHD-FMK (R&D systems, US) in DMSO dissolved in PBS and injected i.p. 30 minutes after surgery. Z-LEHD-FMK was injected at a dose of 0.8 µM kg⁻¹ body weight at a final volume of 200 µl. Z-IETD-FMK was injected at a dose of 0.8 mg kg⁻¹ body weight at a final volume of 100 µl. The control groups were administered vehicle injections at the same volume, route, and timing as experimental animals.

*Selective Beta2-adrenoreceptor inhibitor (ICI-118,551):* The β2-adrenoreceptor inhibitor ICI118,551 (Sigma, Germany) was dissolved in PBS and administered 1 hour prior to and 1 hour after surgery at a dose of 4 mg kg⁻¹ body weight at a final volume of 200 µl. The control group was administered vehicle injections at the same volume, route, and timing as experimental animals.

*Murine recombinant IL-1*β*:* Recombinant IL-1β (401-ML, R&D systems, US) was diluted in sterile PBS and administered intraperitoneally at a dose of 100 or 1000 ng per mouse in a total volume of 100 µl. The control group was administered vehicle injections at the same volume, route and timing as experimental animals.

### Adoptive T cell transfer in Rag-1^{-/-} recipient mice

Donor animals (C57BL6/J, *Casp1*^{-/-}*, Asc*^{-/-}) were euthanized and spleens were collected in Dulbecco's Modified Eagle Medium (DMEM+GlutaMax). Spleens were homogenized and filtered through 40 µm cell strainers. T cells were enriched using a negative selection kit for CD3⁺ T cells (*MagniSort*, Thermo Fisher). After washing and quantification, cells were injected i.p. into *Rag-1*^{-/-} recipient mice (4×10⁶ CD3⁺ T cells per mouse) in a total volume of 200 µl saline. Mice were maintained for 4 weeks in order to establish a functional T cell niche, and then assigned to the surgery groups.

### T cell isolation and culture

Round-bottom tissue culture-treated 96-well plates were coated with 100 µl of PBS containing a mixture of 0.5 mg/mL purified NA/LE hamster anti-mouse CD3e (clone: 145-2C11, BD Pharmingen) and 0.5 mg/mL anti-mouse CD28 (clone: 37.51, Invitrogen), and then incubated overnight at 37 °C with 5 % CO₂. Spleens (wild type, *Casp1*^{-/*-*}) isolated from mice were homogenized into single splenocyte suspensions by using a 40 µm cell strainer followed by erythrolysis as described above. T cells were purified from splenocytes using a negative selection kit (*MagniSort*, Thermo Fisher) according to the manufacturer's instructions. Purity was reliably ≥ 90 % as assessed by flow cytometry. Cells were resuspended in complete RPMI1640 (Gibco) and supplemented with 10 % FBS, 1 % penicillin/ streptomycin and 10 µM β-mercaptoethanol. T cells were seeded into the CD3/CD28 coated plates at a density of 300,000 cells per well in a total volume of 200 µl.

### Organ and tissue processing

Mice were deeply anaesthetized with ketamine (120 mg/kg) and xylazine (16 mg/kg) and blood was drawn via cardiac puncture in 50 mM EDTA (Sigma-Aldrich). Plasma was isolated by centrifugation at 3,000 g for 10 minutes and stored at -80 °C until further use. The blood pellet was resuspended in DMEM and erythrocytes were lysed using isotonic ammonium chloride buffer. Immediately following cardiac puncture, mice were transcardially perfused with normal saline for dissection of bone marrow and spleen. Spleen and bone marrow were transferred to tubes containing Hank's balanced salt solution (HBSS), homogenized and filtered through 40 µm cell strainers to obtain single cell suspensions. Homogenized spleens were subjected to erythrolysis using isotonic ammonium chloride buffer.

### Bacterial culture and CFU counts

*S. pneumoniae* TIGR4, an encapsulated strain of serotype 4, was grown overnight on Columbia blood agar plates (37°C, 5%CO₂), single colonies were cultured in Todd-Hewitt broth with 1% yeast extract to mid-logarithmic phase (OD₆₀₀ₙₘ: 0.35), washed, and diluted in sterile PBS to the desired concentration. *Klebsiella pneumoniae subsp. pneumoniae* was grown in Mueller Hinton broth to mid-logarithmic growth phase (OD₆₀₀ₙₘ: 0.7), washed and diluted in PBS. 14h post bacterial infection, mice were euthanized, tracheas and lungs were aseptically removed and mechanically homogenized in PBS. Serial dilutions of lung and tracheal tissue homogenates were plated onto blood agar plates and CFU were determined after 16h of incubation.

### Fluorescence-activated Cell Sorting (FACS) analysis

The anti-mouse antibodies listed below (see Table 1) were used for surface marker staining of CD45⁺ leukocytes, CD45⁺CD11b⁺ monocytes (+ FasL⁺ expression), CD3⁺ T cells, CD3⁺CD4⁺ Tₕₑₗₚₑᵣ cells, CD3⁺CD8⁺ T_{cytotox} cells and CD19⁺ B cells (for representative gating strategy, see Figure 10A). Fc blocking (Anti CD16/CD32, invitrogen) was performed on all samples prior to extracellular antibody staining. All stains were performed according to the manufacturer's protocols. Flow cytometric data was acquired using a BD FACSverse flow cytometer (BD Biosciences) and analyzed using FlowJo software (Treestar).

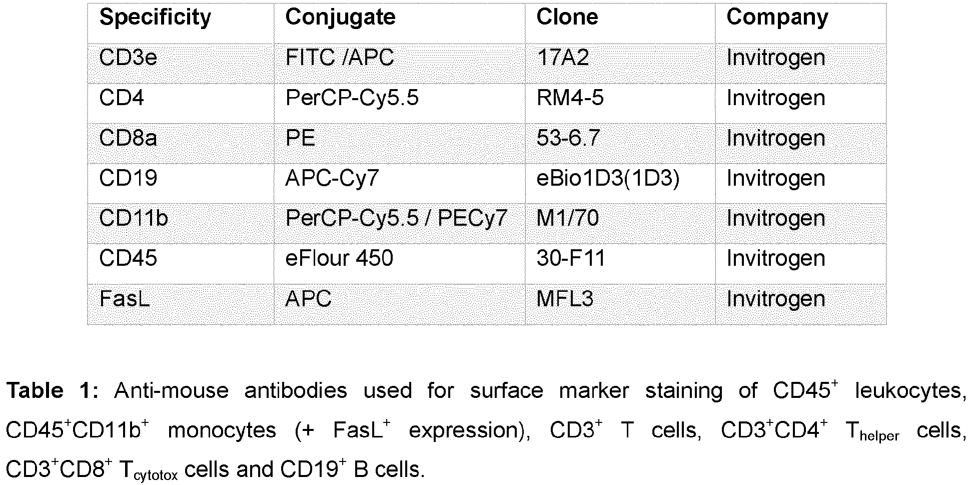

### Dimensionality reduction analysis for FACS data

FACS data acquired with FACSVerse was pre-analyzed with FlowJo software. To normalize the data, each sample was down-scaled ("DownSample" plugin FlowJo) to 3,000 CD45⁺ cells per individual mouse. After concatenating the individual samples into a batch, t-distributed stochastic neighboring embedding (t-SNE) analysis was conducted (Parameters: Iterations 550; Perplexity 30; Eta learning rate 200) using the "t-SNE plugin" of the FlowJo software (V10.6).

### FAM FLICA caspase-1 staining for FACS

To detect the active forms of caspase-1 in blood, spleen, and bone marrow samples, cell suspensions were stained with the fluorescent inhibitor probe FAM-YVAD-FMK (FAM FLICA, BioRad, Germany) for 30 minutes at 37 °C according to the manufacturer's instructions. After washing, the cells were stained for CD45⁺CD3⁺ T cells and CD45⁺Cd11b⁺ monocytes. The flow cytometry data was acquired on a BD FACSVerse (for representative gating strategy, see Figure 10B).

### FACS imaging of ASC-citrine reporter mice

Spleens from ASC-citrine reporter mice were dissected and single splenocyte suspensions were prepared using a 40 µm cell strainer, then subjected to erythrolysis as described above. Splenocytes were then stained with FACS antibodies against CD45, CD3 and CD11b as described above. Cells were resuspended at a concentration of 10⁷ cells/ml for FACS imaging using the Flowsight Imaging flow cytometer (Amnis). The results were analyzed using the IDEAS software (Amnis) (Tzeng *et al.*, 2016). For the speck analysis, cells were pre-gated for CD45⁺CD11b⁺ or CD45⁺CD3⁺ and then gated for citrine⁺. Citrine⁺ cells were randomly selected (50 CD45⁺ CD11b⁺ citrine⁺ cells per mouse) and the numbers of specks per cells was analyzed.

### FAM FLICA caspase-1 staining on fresh frozen spleen sections

Mice were deeply anesthetized and euthanized as described above. Spleens were immediately removed, embedded in cryotech solution (OCT, tissue-tek) and cryosectioned sagittally (20 µm thickness). FAM-YVAD-FMK (FAM FLICA, BioRad, Germany) solution was prepared as indicated in the manufacturer's instruction and sections were incubated for 1 hour at 37 C. Sections were then washed with PBS, stained with DAPI (1 : 5,000; Dako), and mounted (Aqueous mounting medium, Dako). Epifluorescence images were acquired at 20x magnification (Axio Imager 2, Carl Zeiss).

### Whole splenocyte culture

Spleens from naive wild type mice were dissected and single splenocyte suspensions were prepared using a 40 µm cell strainer, then subjected to erythrolysis as described above. Cells were washed three times with PBS, then cell number and viability was assessed using an automated cell counter (BioRad) and Trypan blue solution (Merck). Required viability threshold was ≥ 80 %. Cells were cultured (complete RPMI1640, 10 % heat-inactivated fetal bovine serum (FBS), 1 % penicillin/ streptomycin, 10 µM β-mercaptoethanol) overnight for 16 hours on a 96 well flat bottom (anti-CD3/ CD28 coated) plate at a density of 10⁵ cells per 100 µl in a final volume of 200 µl. Cells were then stimulated by 12-hour incubation with serum from either stroke or sham operated mice at a concentration of 25 % total well volume. After the stimulation, cell death and activation status were analyzed via FACS as described above.

### Bone Marrow-Derived Macrophages (BMDM) isolation and cell culture

BMDMs were generated from tibia and femur of transcardially perfused mice. After careful isolation and dissection of tibia and femur, bone marrow was flushed out of the bones through a 40 µm strainer using a plunger and 1 ml syringe filled with sterile 1x PBS. Strained bone marrow cells were washed with PBS, and resuspended in DMEM + GlutaMAX-1 (Gibco, US), supplemented with 10% FBS and 1% Gentamycin (Thermo Fisher Scientific) and counted. 5 × 10⁷ cells were plated onto 150 mm culture dishes. Cells were differentiated into BMDMs over the course of 8-10 days. For the first 3 days after isolation, cells were supplemented with 20 % L929 cell-conditioned media (LCM), as a source of M-CSF. Cultures were then maintained at 37 °C with 5 % CO₂ until ≥ 90 % confluency.

### BMDM - T cell co-culture assays

Differentiated BMDMs were cultured for 8-10 days, then harvested, washed, counted, and seeded in flat-bottom tissue-culture treated 96-well plates at a density of 100,000 cells per well in a total volume of 200 µl, and then cultured overnight for 16 h (see Figure 5). BMDMs were stimulated for 4 h with LPS (100 ng/ml) and by 10 minute incubation with serum from either stroke or sham operated wild type mice at a concentration of 25 % total volume. Control-treated BMDMs received only FBS-containing culture media. After stimulation, the culture medium was removed, and the cells were washed with sterile PBS to ensure no leftover serum in the medium. BMDM-T cell interaction was then assessed with two approaches (see Figure 5): **1**. Stimulation by secreted factors (left), and **2**. Cell-cell contact (right). **1**: Serum-free RPMI was added to the BMDMs, which were then incubated for 1 hour at 37 °C with 5 % CO₂. The BMDM-conditioned supernatant was then transferred onto purified, cultured T cells and incubated for 2 hours at 37 °C with 5 % CO₂. **2**: T cells were added to the serum-stimulated BMDMs at a density of 200,000 cells per well in a total volume of 200 µl complete RPMI medium (10 % FBS, 1 % penicillin/ streptomycin and 10 µM β-mercaptoethanol), and then incubated for 2 hours at 37 °C with 5 % CO₂. T cell counts and survival rate were assessed by flow cytometry.

For the kinetic analysis of T cell death, we used either eGFP-actin⁺ T cells (see Figure 14: co-culture with WT or *Aim2*^{-/-} BMDMs) or analyzed PI uptake of T cells after addition of PI in a final concentration of 1 µg/ml to the co-culture medium (see Figure 4D: WT or *Fas Ipr* T cells in co-culture with WT BMDMs). Microphotographs (10x magnification) of the cultured cells were acquired every 30 minutes for 180 minutes, starting after addition of T cells to the serum-stimulated BMDMs. Reduction in the number of eGFP-actin⁺ T cells or number of PI⁺ T cells, respectively, was quantified and normalized to the corresponding control group (sham serum treated).

### Western blotting

Spleens were harvested from deeply anaesthetized mice, and the whole organs were processed into single cell suspensions as described above. Single cell suspensions were lysed with RIPA lysis/extraction buffer with added protease/phosphatase inhibitor (Thermo Scientific, US). The total protein content of each sample was measured via bicinchoninic acid assay (Thermo Fisher Scientific, USA). Whole cell extracts were fractionated by SDS-PAGE and transferred onto a polyvinylidene difluoride membrane (BioRad, Germany). After blocking for 1 hour in TBS-T (TBS with 0.1 % Tween 20, pH 8.0) containing 4 % skim milk powder (Sigma), the membrane was washed with TBS-T and incubated with the primary antibodies against caspase-1 (1 : 1000; AdipoGen), IL-1β (1 : 500; R&D systems) and β-actin (1 : 1000; Sigma). Membranes were washed three times with TBS-T and incubated for 1 hour with HRP-conjugated anti-rabbit or anti-mouse secondary antibodies (1 : 5,000, Dako) at room temperature. Membranes were washed three times with TBS-T, developed using ECL substrate (Millipore) and acquired via the Vilber Fusion Fx7 imaging system.

### Clinical stroke study population

Ischemic stroke patients were recruited within 24 hours of symptom onset through the emergency department at the LMU University Hospital Munich (Germany), a tertiary level hospital. All patients had a final diagnosis of ischemic stroke as defined by 1) an acute focal neurological deficit in combination with a diffusion weighted imaging-positive lesion on magnetic resonance imaging, or 2) a new lesion on a delayed CT scan. Age-matched control patients were recruited in the neurological outpatient clinic. The study was approved by the local ethics committee and was conducted in accordance with the Declaration of Helsinki as well as institutional guidelines. Written and informed consent was obtained from all patients.

**Table 2 (shown as Mean (SD))**

| | Stroke | Control |
|---|---|---|
| Age | 74 (10) | 74 (10) |
| Sex (female) | 20 % (4) | 20 % (4) |
| Infarct volume | 126 ml (101 ml) | N/A |
| Time after stroke onset | 4.0 h (2.3 h) | N/A |

For analysis of secondary infections (see Figures 26A-D), 174 stroke patients were included. Secondary infections were defined as clinically diagnosed by the treating physician and additionally confirmed by either blood C-reactive protein (CRP) concentration >30 mg/l and/or radiographic (chest X-ray or CT) confirmation of pneumonia. The study was approved by the local ethics committee and was conducted in accordance with the Declaration of Helsinki as well as institutional guidelines. Written and informed consent was obtained from all patients.

**Table 3: Patient characteristics for analyses shown in Figures 26A-D.**

| | No infection (N=124) | Infection (N=50) | P |
|---|---|---|---|
| Age, median (IQR) [years] | 76 (66-82) | 80 (71-85) | 0.012 |
| Female, % (n) | 44 (55) | 54 (27) | 0.314 |
| Baseline NIHSS score, median (IQR) | 3 (1-8) | 15 (7-21) | <0.001 |
| Infarct volume, median (IQR) [ml] | 2 (0-10) | 29 (5-107) | <0.001 |

### Thermal injury patients

Patients with severe burn injury encompassing more than 40% of total body surface area (TBSA) were recruited through BG Trauma Center Ludwigshafen (Germany). TBSA was assessed on admission by the attending burn surgeon using Lund-Browder charts and serum samples were collected at 24 hours post burn. Age- and sex-matched control patients were recruited in the trauma center outpatient clinic. The study was approved by the local ethics committee and was conducted in accordance with the Declaration of Helsinki as well as institutional guidelines. Written and informed consent was obtained from all patients.

**Table 4 shown as Mean (SD))**

| Burn | | | | Control | | |
|---|---|---|---|---|---|---|
| No. | Age | Sex | TBSA % | No. | Age | Sex |
| 1 | 32 | m | 60 | 1 | 35 | m |
| 2 | 57 | m | 50 | 2 | 45 | m |
| 3 | 71 | m | 64 | 3 | 68 | m |
| 4 | 57 | m | 51 | 4 | 60 | m |
| 5 | 73 | m | 58 | 5 | 61 | m |

### Human monocyte culture stimulation with patient's serum

Human Monocytes cells (3 × 10⁵/well) were seeded in 96 flat bottom plates with 50 ng/ml recombinant human M-CSF in RPMI 1640 (Gibco) supplemented with 2.5 % (v/v) human serum (Sigma-Aldrich), Penicillin-Streptomycin (100 IU/ml, Thermo Fisher Scientific), Pyruvate (1 mM, Gibco) and HEPES (10 mM, Sigma-Aldrich) overnight to adjust the cells. Next day, cells were replaced with fresh medium (without M-CSF) in presence or absence of Pam3CSK4 (2.5 µg/ml) for 2 hours. Next, cells were stimulated with either control serum or stroke serum (1:4 dilution). After 2 hours, medium was gently removed and cells were washed once with PBS and replaced with fresh medium (150 µl each well) for 6 hours. Nigericin (Sigma) was used as positive control at final concentration 6.5 µM, stimulated for 6 hours. For each condition, 5 wells were stimulated. Supernatants were collected and 50 µl from each well was used for human IL-1β ELISA (BD) while remaining supernatants were combined and used for Western blot analysis after precipitating with methanol/chloroform. Cells were directly lysed in 1X SDS Laemmli buffer and lysates were combined from 5 wells for each condition. Samples were heated at 95 °C with 1100 rpm and loaded on SDS-PAGE gel (5 % stacking gel and 12 % separating gel; BioRad). Afterward, proteins were transferred on nitrocellulose membrane (GE healthcare) for 1 h. Membranes were blocked for another 60 min in 3 % milk in PBST (PBS containing 0.05 % Tween 20). All primary antibodies of caspase-1 (1 : 1000; AdipoGen), IL-1β (1 : 500; R&D systems) were incubated at least overnight in 1 % milk in PBST at 4 °C. Next day, membranes were incubated for at least 1 h in secondary antibody (Santa Cruz) and washed gently in PBST buffer for further 30-60 min. Loading control β-actin-HRP antibody was purchased from Santa Cruz (1 : 3000). Chemiluminescent signal was recorded with CCD camera in Fusion SL (PEQLAB). If needed, the whole image was contrast-enhanced in a linear fashion.

### Free nucleic acid quantification

Cell-free nucleic acids (RNA, single strand (ss) DNA and double strand (ds) DNA) levels in the plasma of mice and human patients was assessed with a Qubit 2.0 fluorometer (Invitrogen) using specific fluorescent dyes which bind either ssDNA (ssDNA Assay Kit, Thermo Fisher Scientific) or dsDNA (HS dsDNA Assay kit, Thermo Fisher Scientific). Dilutions and standards were generated following the manufacturer's instructions (Thermo Fisher scientific).

### Enzyme linked immunosorbent assay (ELISA)

Total IL-1β and caspase-1 levels from patient plasma samples (diluted 1 : 10 in sterile PBS) were obtained using commercial assay kits according to the manufacturer's instructions (Quantikine ELISA human IL-1β, Quantikine ELISA human caspase-1 R&D systems). Total IL-1β and IL-18 levels from murine plasma samples were measured using the Duoset ELISA IL-1β and the Duoset ELISA IL-18 kit according to the manufacturer's instructions (R&D systems).

### Quantitative RT-PCR

Total RNA was purified from naive splenic CD11b⁺ monocytes and CD3⁺ T cells using the RNeasy Mini Kit (Qiagen). RNA from each sample was used for cDNA synthesis using the High Capacity cDNA Reverse Transcription Kit (Applied Biosystems). The quantitative expression of different cytokines was measured by quantitative real-time PCR with the LightCycler 480 II (Roche) and RT² qPCR Primer Assays and SYBR Green ROX qPCR Mastermix (Qiagen).

### Multiplex mouse cytokine quantification

Plasma samples from mice were used to assess cytokine and chemokine levels (IL-1α, IL-1β, IL-2, IL-3, IL-4, IL-5, IL-6, IL-9, IL-10, IL-12(p40), IL-12(p70), IL-13, IL-17, Eotaxin, G-CSF, IFN-γ, KC, MCP-1, MIP-1α, MIP-1β, RANTES, Tnf-α) using a Luminex-100 system following the instructions in the manufacturer's manual (Bio-PIex23 Pro Mouse Cytokine Grp1, BioRad).

### Infarct volumetry

Mice were euthanized by overdose of ketamine-xylazine and perfused intracardially with normal saline. Brains were removed and immediately frozen in powdered dry ice. Frozen brains were fixed in cryotech solution (OCT, tissue-tek) and 20 µm coronal sections were collected at 400 µm intervals. Sections were stained with cresyl violet and scanned at a resolution of 600 dpi. Infarct area of each section was assessed by imaged software (NIH). The Swanson method was employed to measure the infarct area and to correct for cortical swelling: [ischemic area] = [area of the contralateral hemisphere] - [non-ischemic area of the ipsilateral hemisphere]. The total infarct volume was determined by integrating measured areas and distances between sections.

### Statistical analysis

Data were analyzed using GraphPad Prism version 6.0. All summary data are expressed as the mean ± standard deviation (s.d.). All data sets were tested for normality using the Shapiro-Wilk normality test. The groups containing normally distributed independent data were analyzed using a two-way Student's t-test (for 2 groups) or ANOVA (for > 2 groups). Normally distributed dependent data (i.e. *in vitro* co-culture kinetics) were analyzed using a 2-way ANOVA. The remaining data were analyzed using the Mann-Whitney *U* test (for 2 groups) or Kruskal-Wallis Test (H-test, for > 2 groups). Similar variance was assured for all groups, which were statistically compared. P-values were adjusted for comparison of multiple comparisons using Bonferroni correction or Dunn's multiple comparison tests. A p value < 0.05 was considered to be statistically significant.

For statistical analysis of human patient data (see **Figure 26**), values for dsDNA, IL-1β and lymphocyte counts were log10 transformed. The inventors applied linear regression analysis to assess associations of serum dsDNA concentrations, IL-1β concentrations, and lymphocyte counts and logistic regression analysis to assess association with secondary infections. Where indicated, adjustment was performed for age and sex. Mediation analysis was performed using the template described by Baron and Kenny and the method by Vanderweele and Vansteelandt (Baron and Kenny, 1986; Vanderweele and Vansteelandt, 2010). All pathways were assessed using multivariable logistic regression analyses adjusting for age, sex, and dsDNA concentrations. Statistical analyses were performed in R, version 3.5.1.

### Example 1: Sterile tissue injury induces severe T cell death

Experimental tissue injury of different etiologies and organs such as stroke (brain), burn injury (skin) or hindlimb ischemia (skeletal muscle) all result in subacute immunosuppression, characterized by a massive T cell death with approx. 50 % loss within less than 24 h after the injury (see Figures 1A-D and 6). The inventors tested the impact of soluble mediators on T cell death after tissue injury, utilizing a murine parabiosis model in which two mice share a common blood circulation (see **Figure 7**), but only one of the two parabionts received either a stroke or sham operation. The inventors observed a significant reduction of splenic T cells, not only in the operated animal after stroke but also in the non-operated parabiont, indicating a crucial role for soluble factors in the blood circulation for phenomenon (see **Figure 1E**). The inventors then used an in vitro model to fBacterial c cell culture with the serum from either sham- or stroke-operated mice. Serum of stroke mice significantly reduced T cell survival, confirming the release of a cytotoxic factor to the blood after stroke (see **Figure 1F**). Previous studies have consistently shown an early pro-inflammatory response after tissue injuries (Offner *et al.*, 2006; Osuka *et al.,* 2014), which is preceding the later lymphopenia. To further explore the main cytokines/ chemokines involved in the early pro-inflammatory response after tissue injury, the inventors performed a multiplex assay for 23 cytokines and chemokines in the serum of mice 6 h after sham or stroke surgery, which identified Interleukin-1β (IL-1β) as the most abundantly upregulated cytokine (see **Figure 1G**). Interestingly, a kinetic analysis comparing the time course of IL-1β serum concentration and T cell death after experimental stroke indicated an association of these two events (see **Figure 1H**). Indeed, neutralizing circulating IL-1β significantly improved T cell survival after stroke, demonstrating a causal relationship between IL-1β concentrations and T cell death (see **Figure 1I** and **Figure 8**).

### Example 2: Stroke leads to systemic inflammasome activation

IL-1β cleavage of the pro-form to the mature cytokine and its extracellular release are tightly regulated by caspase-1, the central effector enzyme of the inflammasome (Lopez-Castejon *et al*., 2011; Bauernfeind *et al.,* 2011). The inflammasome is a multi-protein complex which accumulates and orchestrates caspase-1 cleavage upon activation of a wide range of danger signals sensed by the inflammasome. The inventors were able to identify systemic inflammasome activation after local tissue injury in the brain by several lines of evidence: they observed an increase of both pro-caspase-1 as well as its active cleavage isoforms in spleens by western blot (see **Figure 2A**), confirmed caspase-1 cleavage histologically in spleens (see **Figure 2B**), and were able to visualize inflammasome formation in splenic monocytes using ASC-citrine reporter mice for flow imaging (see **Figure 2C**) (Tzeng *et al.*, 2016). Correspondingly, also in human stroke patients the inventors found not only increased IL-1β but also caspase-1 blood concentrations (see **Figure 9**). In order to test the effect of circulating blood factors in activating the inflammasome and IL-1β release in human cells, the inventors cultured human monocytes from 4 different healthy donors and treated the cells with serum from either stroke or healthy patients. The inventors detected a consistent increase in caspase-1 cleavage (see **Figure 2D**) and secretion of IL-1β (see **Figure 2E**) in the stroke compared to the control serum conditions. These findings from murine and human models clearly demonstrated that blood factors released after local tissue injury lead to systemic inflammasome activation and IL-1β release. The inventors next tested a causal role of the inflammasome in mediating post-injury T cell loss. Genetic caspase-1 deficiency substantially improved T cell survival (see **Figure 2F**) while it also reduced serum IL-1β concentrations and increased spleen cellularity (see **Figure 10**). In a series of *in vivo* experiments using different transgenic models of inflammasome-deficiency, we were able to confirm the concept of inflammasome-dependent IL-1L1-β secretion from splenic monocytes as the driver of FasL-mediated T cell death. This approach showed that global deficiency for caspase-1 and the adaptor molecule ASC (*Pycard*) ameliorated post-injury T cell death (see **Figure 22A**). Moreover, inflammasome deficiency completely prevented the increase in post-injury IL-1β secretion and FasL expression (see **Figures 22B** and C). Conditional inflammasome deficiency only in the myeloid cell compartment (using *Lyz2-cre x Pycard*^{*fl*/*fl*} mice) was sufficient to completely rescue post-injury T cell death (see **Figure 2I**), demonstrating the critical role of monocytic inflammasome activation for T cell death. The inventors further investigated the specificity of IL-1β in this phenomenon, specifically in comparison to the closely related IL-1α and to IL-18. While also IL-1α and IL-18 were increased in serum after stroke, FasL upregulation on myeloid cells was predominantly induced by IL-1β (see **Figures 22D** and E).

Likewise, pharmacological inhibition of caspase-1 using the small molecule inhibitor VX-765 improved T cell survival and spleen cellularity after stroke as well as burn injury in mice (see **Figure 11**). Notably, neither genetic nor pharmacological caspase-1 deficiency had a significant effect on the primary lesion size, highlighting the importance of the inflammasome in the secondary immunological events independent of modulating lesion severity (see **Figure 12**).

### Example 3: Inflammasome activation in monocytes drives cell death in T cells

Inflammasome subtypes are defined by the sensor molecule which determines the specificity for different activation signals, such as non-self proteins, ion flux or nucleic acids (Latz *et al.,* 2013; Hornung *et al.,* 2009). Most, but not all, inflammasome subtypes require the ASC adaptor protein for oligomerization and caspase-1 activation using its caspase activation and recruitment domain (CARD) (Hoss *et al.*, 2017). The inventors observed a significantly improved T cell survival after stroke in ASC-deficient mice, indicating an ASC-dependent inflammasome activation in T cell death after tissue injury (see **Figure 2G**). The inventors next looked at whether inflammasome activation and cell death is a T cell-autonomous process or is mediated by a different cell population. In order to test this, the inventors generated T cell-specific caspase-1 and ASC deficiency models by adoptive T cell transfer to lymphocyte-deficient *Rag-1*^{-/-} mice (see **Figure 2H** and **Figure 13A**). Neither caspase-1 nor ASC deficiency in T cells improved T cell survival, indicating that inflammasome-driven T cell death is non-autonomous. Furthermore, monocyte-specific ASC deficiency (*LysM-Asc*^{-/-} mice) resulted in substantially increased T cell survival after stroke, clearly demonstrating the importance of monocytic inflammasome activation for subsequent T cell death (see **Figure 2I**). Based on these findings, the inventors re-evaluated the cell type-specific inflammasome activation in splenic T cells and monocytes after tissue injury, detecting an increase in caspase-1 activity specifically in monocytes, but not in T cells after stroke (see **Figure 13B**). Correspondingly, the transcription of most inflammasome components, except for *Nlrc3*, was only detected in monocytes, but not in T cells (see Figure 13C), further supporting a non cell-autonomous inflammasome effect on T cells death. The inventors used an *in vitro* model to test this hypothesis via co-culture of either WT or *Casp1*^{-/-} monocytes (BMDMs) and T cells (see **Figure 2J**). Here, only WT but not the *Casp1*^{-/-} monocytes were able to induce T cell death. However, the T cell genotype was irrelevant for their survival. In summary, these findings unequivocally demonstrate systemic inflammasome activation in monocytes which is causative for non cell-autonomous T cell death after tissue injury.

### Example 4: Nucleic acids activate systemic inflammasome response after ischemia

The inventors identified in this Example the upstream mediator leading to systemic inflammasome activation. They detected a significant increase in cell free double strand DNA (cf-dsDNA) after stroke and burn lesions in mice as well as in patients (see Figure 3A-D and **Figure 23A**). Correspondingly, *in vivo* treatment of mice with 1000 U of human recombinant DNase (hrDNase) resulted in reduction of cf-dsDNA blood concentrations (see **Figure 14**), which substantially reduced inflammasome activation in splenic monocytes, prevented the expansion of FasL+ myeloid cell population and improved T cell survival after experimental stroke (see **Figure 3E** and **Figure 23B**). The inventors further validated the causal function of cf-dsDNA for the induction of FasL⁺ monocytes *in vitro*: ex vivo treatment of post-stroke mouse serum with hrDNAse did not affect the serum concentration of IL-1β but completely prevented the FasL upregulation on serum-stimulated monocytes (see **Figures 24A** and B), showing that cf-dsDNA as the initial stimulus upstream of the IL-1β-induced FasL upregulation. The inventors compared *Aim2*^{-/-} and WT mice for caspase-1 activation and T cell death after stroke and observed a significant reduction in caspase-1 activation in monocytes as well as drastically improved T cell death in *Aim2*^{-/-} compared to WT mice to levels of sham-operated mice (see **Figure 3F**), which was also reflected in increased overall splenic cellularity in *Aim2*^{-/-} mice after stroke (see **Figure 14B**). *In vitro* co-culture experiments (corresponding to **Figure 2J**) confirmed the *in vivo* observations, where *Aim2*^{-/-} BMDMs did not induce T cell death in contrast to WT BMDMs stimulated with serum from stroke mice (see **Figure 14C**).

Notably, while the used genetic and pharmacological models to block AIM2 inflammasome activation efficiently prevented myeloid FasL upregulation, the primary lesion size was unaffected by these approaches. These results underscore the notion that the inflammasome pathway impacts on the systemic, immunological events following stroke rather than modulating lesion severity (see **Figure 24C**). While the inventors identified this monocyte-T cell interaction in the prototypic tissue injury model of brain ischemia, they were able to replicate all key events of this pathway-cf-dsDNA release, inflammasome activation, and subsequent T cell apoptosis-in an independent model of burn injury (see Figures 25A to E).

Taken together, the inventors have observed that acute tissue injury increases cf-dsDNA blood concentrations and that cf-dsDNA is a potent and sufficient activator of the AIM2 inflammasome, leading to T cell death.

### Example 5: Cell-cell interaction is needed to induced T cell death

The inventors have also identified the mechanisms by which inflammasome activation in monocytes results in T cell death after tissue injury. First, the inventors tested whether cell-cell contact is necessary or soluble mediators released by monocytes are sufficient for this interaction. Therefore, the inventors established an *in vitro* co-culture model of BMDMs and T cells with or without cell contact (see **Figure 4A**). BMDM inflammasome activation by the serum from stroke induced T cell death only when cell-cell contact between BMDMs and T cells was enabled, while supernatant from activated BMDMs was not cytotoxic for T cells (see **Figure 4B**). To further analyze the mode of T cell apoptosis, mice were treated either with a caspase-8 inhibitor (Z-IETD-FMK) blocking the extrinsic or a caspase-9 inhibitor (Z-LEHD-FMK) blocking the intrinsic apoptosis pathways after sham or stroke surgery. T cell death was only reduced in mice treated with the caspase-8 but not the caspase-9 inhibitor (see **Figure 4C**), while neither affected caspase-1 activation in monocytes (see **Figure 15**). These findings demonstrate that inflammasome activation in monocytes induce the extrinsic cell death pathway in T cells, which is caspase-8 dependent and mediated via the Fas receptor and the intracellular Fas associated death domain (FADD) (Strasser *et al.,* 2009). Under physiological conditions, the Fas ligand (FasL) is upregulated by T cells during activation-induced cell death as well as by activated monocytes as an important regulatory mechanism for T cell homeostasis (Nagata *et al.,* 1999; Brown *et al.,* 1999). Multidimensional flow cytometric analysis revealed a FasL-positive subpopulation of tissue injury-induced monocytes (TIM) in the experimental stroke model (see **Figure 4D** and **Figure 16**). Further flow cytometric analyses revealed that induction of this population was completely blunted in caspase-1 deficient mice and after neutralization of IL-1β by monoclonal antibodies (see **Figure 4E** and **Figure 17A**). These findings indicate that inflammasome-activation and subsequent IL-1β secretion is the required upstream mechanism for FasL upregulation in monocytes in accordance with the initial observation that IL-1β reduced T cell death (see Figure 1J). Correspondingly, *in vitro* treatment of BMDMs with recombinant IL-1β resulted in increased FasL expression and T cell death but to a lesser degree than induced by the serum of stroke mice (see **Figure 17B**). In turn, the injection of recombinant IL-1β to mice in vivo dose-dependently induced T cell death and FasL upregulation on myeloid cells, closely resembling the stroke-induced phenotype (see **Figures 21A** and B). Taken together, these experiments reveal that the post-injury increase in IL-1β blood concentration drives the expansion of T cell-cytotoxic FasL⁺ myeloid cells. The inventors then tested the role of the Fas receptor for tissue-injury induced T cell death by comparison of WT and Fas-deficient (*Fas Ipr*) T cells in co-culture with WT BMDMs enabling cell-cell contact (corresponding to **Figure 4A**, right). Indeed, Fas-deficient T cells were protected from the cytotoxic effect of inflammasome-activated monocytes, induced by the stroke serum in comparison to serum from Sham-operated control mice (see **Figure 4F** and **Figure 17C**).

### Example 6: Acute tissue injury also causes B cell death

Additionally, the inventors found corresponding findings for the mechanism of B cell death as above for T cells. Experimental tissue injury (stroke and burn injury) results as well in a massive B cell death with approx. 40-50 % loss within less than 24 h after the injury (see **Figures 18A****, B**). As previously described above for T cells, genetic caspase-1 deficiency also substantially improved B cell survival (see **Figure 18C**). *In vivo* treatment of mice with 1000 U of human recombinant DNase (hrDNase), which showed to decrease cf-dsDNA concentration in plasma and inflammasome activation in monocytes (corresponding to **Figure 3E**), also improved B cell survival after experimental stroke.

### Example 7: T cell apoptosis occurs as bystander cell death following injury-induced FasL⁺ myeloid cells

The inventors aimed to test the hypothesis that soluble mediators released after injury are a potential cause for T cell apoptosis. First, the inventors confirmed a pronounced and general T cell death across subpopulations after experimental stroke which occurred even under sterile (germfree) conditions, hence, cannot be attributed to potential concomitant microbial infections (see **Figures 19A** and B).

Treatment of mixed splenocytes-which allows an unbiased ex vivo analysis of all splenic leukocyte subpopulations and their potential interactions-with stroke serum *in vitro* revealed a close temporal association between the monocytic FasL upregulation and T cell death (see **Figures 20** **A** and **B**). Correspondingly, treatment of mice with FasL-specific neutralizing antibodies significantly improved T cell survival post-injury (see **Figure 20C**). Hence, as already been shown the inventors also tested the role of the death receptor Fas in extrinsic T cell death by comparing WT and Fas-deficient (*Fas*^{lpr}) T cells first in an *in vitro* co-culture with serum-stimulated BMDMs. Indeed, Fas-deficient T cells were protected from the cytotoxic effect of stroke serum-stimulated monocytes (see **Figure 4F**). Next, the inventors aimed to validate this finding *in vivo* by adoptively transferring *Fas*^{lpr} or WT T cells to lymphocyte-deficient *Rag-1*^{-/-} mice. In contrast to WT T cells, post-stroke cell death was completely prevented in *Fas*^{/pr} T cells, demonstrating the critical role of Fas-signaling for post-stroke T cell death (see **Figure 20D**). In summary, these experiments reveal a previously unrecognized cause of post-injury lymphopenia: extrinsic T cell apoptosis as bystanders to an injury-induced FasL⁺ myeloid population.

### Example 8: Inflammasome-driven lymphocyte death predisposes to bacterial infections

Patients with severe tissue injuries after stroke, trauma, or burn have a high susceptibility to infections, which contribute substantially to secondary mortality. Therefore, after identifying the mechanism of T cell death by a bystander mechanism to inflammasome activation in monocytes, the inventors aimed to test the relevance of this pathway for post-injury infections. They analyzed 174 patients with ischemic stroke for which complete information was available for serum concentrations of dsDNA and IL-1β at hospital admission (d0; mean time after symptom onset: 4.9 hours), their blood lymphocyte counts on the subsequent day (d1) and the occurrence of infections (requiring antibiotic treatment and CRP >30 mg/l and/or radiographic confirmation) between days 2-7 after stroke onset (see **Figure 26A**). The inventors detected a significant association between serum dsDNA and IL-1β concentrations as well as a significant negative association between IL-1β concentration on admission and blood lymphocyte counts on the following day (see **Figure 26B**). Patients with secondary infections after stroke showed significantly increased IL-1β concentrations on admission and reduced lymphocyte counts on d1 (see **Figure 26C**). Therefore, the inventors performed a mediation analysis to test whether lymphocyte counts mediate the effect of IL-1β on the incidence of secondary infections. Supporting the hypothesis, acute IL-1β concentrations were significantly associated with subacute infections, where this effect was mediated via a reduction of lymphocyte counts. This effect was considered a full mediation because the direct effect of IL-1β concentrations on infections (p=0.037) was no longer statistically significant after inclusion of the mediator in the regression model (p=0.12) (see **Figure 26D**). Next, the inventors aimed to test the therapeutic targeting of this pathway for immunocompetence during post-injury infections. Therefore, mice were treated with the pharmacological inflammasome inhibitor VX765 after stroke. Then, VX765 or control-treated animals received an experimental respiratory tract infection with either *Streptococcus pneumoniae* or *Klebsiella pneumoniae* 12h after sham or stroke surgery and the bacterial load in the respiratory tract was analyzed another 14h later (see **Figure 26E**). Control-treated mice showed increased bacterial loads after stroke compared to sham surgery in both experimental pneumonia models, while inflammasome inhibition by VX765 reduced post-injury bacterial load comparable to sham-operated group (see **Figure 26F**). These findings reveal that inhibition of the inflammasome after stroke functionally increases immunocompetence by rescuing post-stroke T cell death instead of inhibiting the direct anti-microbial functions of inflammasome activation.

**Summary:** Dysregulation of systemic immune homeostasis is a common consequence of local tissue injuries. The inventors have identified a surprising mechanism by which systemic activation of the AIM2 inflammasome links an immediate pro-inflammatory response with subsequent immunosuppression after various types of acute injuries in mice and human patients (see **Figure 4G**). The biphasic systemic immune response after tissue injury - early activation and subsequent immunosuppression - is of major clinical relevance for patients with acute tissue injuries. The pro-inflammatory systemic immune response after acute tissue injury has been associated with the development of depressive-like behaviour due to the psychotropic functions of pro-inflammatory cytokines (Dantzer *et al.*, 2008), which increases morbidity and impairs the rehabilitation from acute injuries such as trauma or stroke (Robinson *et al.*, 2016). Besides the neuropsychiatric complications, acute systemic inflammation to sterile tissue injury can also lead to multiple organ dysfunction by cardiac and renal dysfunction and vascular leakage (Lord *et al.*, 2014; Hundeshagen *et al.*, 2017). In the subacute phase, immunodepression and lymphopenia equally account for morbidity and mortality after tissue injury: pneumonia, the most common infection across stroke, burn injury or polytrauma patients accounts for 12 - 34 % of in-hospital deaths in these patient cohorts (Koennecke *et al.*, 2011, Lachiewicz *et al.,* 2017; Sauaia *et al.*, 2017; Cook *et al.,* 2011). Interestingly, lymphopenia has been consistently demonstrated as a predictor of bacterial infections in patients with acute tissue injury (Haeusler *et al.*, 2012; Prass *et al.,* 2003; Barlow *et al.*, 1994; Patenaude *et al.,* 2005). Yet, prophylactic antibiotic treatment did not proof to be an efficient therapeutic strategy after acute tissue injury, most likely due to off-target effects (Westendorp *et al.*, 2015; Ramos *et al.*, 2017).

Therefore, in addition to a detailed understanding of the immunological mechanisms, the present invention also provides several novel therapeutic targets to ameliorate the diverse immunological consequences of tissue injuries. The identified pathway along the events of increased cf-dsDNA concentrations, inflammasome activation, IL-1β secretion and Fas-mediated T cell death provides several druggable, therapeutic targets - for which available drugs could even be repurposed. The most promising therapeutic target seems to be the pathological initiator of this immunological cascade, the increase in circulating cf-dsDNA. The inventors have shown the efficient degradation of cf-dsDNA and reduction of the immunological consequences by use of human recombinant DNAse. Inhaled hrDNAse is already in clinical use for patients with cystic fibrosis, however, its systemic administration and immunological effects in tissue injury have so far not been tested. Additionally, the key effector molecule of the inflammasome, the IL-1β cytokine, represents another promising drug target. The inventors have identified IL-1β secretion to be important in mediating the downstream cell-cell contact-dependent T cell death after tissue injury. Hence, neutralization of circulating IL-1β by monoclonal antibodies might paradoxically improve systemic immunocompetence after tissue injury by preventing T cell death despite being currently used as an immunosuppressive drug. Indeed, while IL-1β blockade has initially been developed for rare autoimmune disorders, this approach has recently been tested also for patients with myocardial infarction. IL-1β blockade significantly lowered recurrent local cardiovascular events in a large clinical trial (Ridker *et al.*, 2017) and its local anti-inflammatory effects might reduce development of heart failure (Panahi *et al.,* 2018).

Taken together, the present invention identified a surprising systemic activation of the inflammasome as the linking mechanisms between a systemic immune response and subsequent immunosuppression after various local tissue injuries. Inhibiting the inflammasome-IL-1β-Fas pathway is therefore important for preventing secondary immunosuppression in patients with acute tissue injury.

### REFERENCES

Barlow, Y. T lymphocytes and immunosuppression in the burned patient: a review. Burns 20, 487-490, doi:10.1016/0305-4179(94)90002-7 (1994).
Baron, R.M., and Kenny, D.A. The moderator-mediator variable distinction in social psychological research: conceptual, strategic, and statistical considerations. J Pers Soc Psychol 51, 1173-1182 (1986).
Bauernfeind, F. et al. Inflammasomes: current understanding and open questions. Cell Mol Life Sci 68, 765-783, doi:10.1007/s00018-010-0567-4 (2011).
Bohannon, J. et al. Prophylactic treatment with fms-like tyrosine kinase-3 ligand after burn injury enhances global immune responses to infection. J Immunol 180, 3038-3048, doi:10.4049/jimmunol.180.5.3038 (2008).
Brown, S. B. & Savill, J. Phagocytosis triggers macrophage release of Fas ligand and induces apoptosis of bystander leukocytes. J Immunol 162, 480-485 (1999).
Cook, A., Norwood, S. & Berne, J. Ventilator-associated pneumonia is more common and of less consequence in trauma patients compared with other critically ill patients. J Trauma 69, 1083-1091, doi:10.1097/TA.0b013e3181f9fb51 (2010).
Dantzer, R., O'Connor, J. C., Freund, G. G., Johnson, R. W. & Kelley, K. W. From inflammation to sickness and depression: when the immune system subjugates the brain. Nat Rev Neurosci 9, 46-56, doi:10.1038/nrn2297 (2008).
D'Avignon, L. C. et al. Contribution of bacterial and viral infections to attributable mortality in patients with severe burns: an autopsy series. Burns 36, 773-779, doi:10.1016/j.burns.2009.11.007 (2010).
Emsley, H. C. et al. An early and sustained peripheral inflammatory response in acute ischaemic stroke: relationships with infection and atherosclerosis. J Neuroimmunol 139, 93-101, doi:10.1016/s0165-5728(03)00134-6 (2003).
Haeusler, K. G. et al. Immune responses after acute ischemic stroke or myocardial infarction. Int J Cardiol 155, 372-377, doi:10.1016/j.ijcard.2010.10.053 (2012).
Hazeldine, J., Hampson, P., Opoku, F. A., Foster, M. & Lord, J. M. N-Formyl peptides drive mitochondrial damage associated molecular pattern induced neutrophil activation through ERK1/2 and P38 MAP kinase signalling pathways. Injury 46, 975-984, doi:10.1016/j.injury.2015.03.028 (2015).
Hoss, F., Rodriguez-Alcazar, J. F. & Latz, E. Assembly and regulation of ASC specks. Cell Mol Life Sci 74, 1211-1229, doi:10.1007/s00018-016-2396-6 (2017).
Howard, R. J. & Simmons, R. L. Acquired immunologic deficiencies after trauma and surgical procedures. Surg Gynecol Obstet 139, 771-782 (1974).
Hundeshagen, G. et al. Fms-Like Tyrosine Kinase-3 Ligand Attenuates Local and Systemic Infection in a Model of Post-Burn Pneumonia. Shock 49, 721-727, doi:10.1097/SHK.0000000000000964 (2018).
Hundeshagen, G. et al. The occurrence of single and multiple organ dysfunction in pediatric electrical versus other thermal burns. J Trauma Acute Care Surg 82, 946-951, doi: 1 0.1097 IT A. 000000000000 1356 (2017).
Kilkenny, C., Browne, W. J., Cuthill, I. C., Emerson, M. & Altman, D. G. Improving bioscience research reporting: the ARRIVE guidelines for reporting animal research. PLoS Biol 8, e1000412, doi:10.1371/journal.pbio.1000412 (2010).
Kohsaka, S. et al. Systemic inflammatory response syndrome after acute myocardial infarction complicated by cardiogenic shock. Arch Intern Med 165, 1643-1650, doi:10.1001/archinte.165.14.1643 (2005).
Lachiewicz, A. M., Hauck, C. G., Weber, D. J., Cairns, B. A. & van Duin, D. Bacterial Infections After Burn Injuries: Impact of Multidrug Resistance. Clin Infect Dis 65, 2130-2136, doi:10.1093/cid/cix682 (2017).
Latz, E., Xiao, T. S. & Stutz, A. Activation and regulation of the inflammasomes. Nat Rev Immunol 13, 397-411, doi:10.1038/nri3452 (2013).
Li, T. et al. Proliferation of parenchymal microglia is the main source of microgliosis after ischaemic stroke. Brain 136, 3578-3588, doi:10.1093/brain/awt287 (2013).
Liliensiek, B. et al. Receptor for advanced glycation end products (RAGE) regulates sepsis but not the adaptive immune response. J Clin Invest 113, 1641-1650, doi:10.1172/JCI18704 (2004).
Lo, H. R. & Chao, Y. C. Rapid titer determination of baculovirus by quantitative real-time polymerase chain reaction. Biotechnol Prog 20, 354-360, doi:10.1021/bp034132i (2004).
Lopez-Castejon, G. & Brough, D. Understanding the mechanism of IL-1beta secretion. Cytokine Growth Factor Rev 22, 189-195, doi:10.1016/j.cytogfr.2011.10.001 (2011).
Lord, J. M. et al. The systemic immune response to trauma: an overview of pathophysiology and treatment. Lancet 384, 1455-1465, doi:10.1016/S0140-6736(14)60687-5 (2014).
Manson, J., Thiemermann, C. & Brohi, K. Trauma alarmins as activators of damage-induced inflammation. Br J Surg 99 Suppl 1, 12-20, doi:10.1002/bjs.7717 (2012).
Nagata, S. Fas ligand-induced apoptosis. Annu Rev Genet 33, 29-55, doi:10.1146/annurev.genet.33.1.29 (1999).
Offner, H. et al. Experimental stroke induces massive, rapid activation of the peripheral immune system. J Cereb Blood Flow Metab 26, 654-665, doi:10.1038/sj.jcbfm.9600217 (2006).
Offner, H. et al. Splenic atrophy in experimental stroke is accompanied by increased regulatory T cells and circulating macrophages. J Immunol 176, 6523-6531, doi:10.4049/jimmunol.176.11.6523 (2006).
Osuka, A., Ogura, H., Ueyama, M., Shimazu, T. & Lederer, J. A. Immune response to traumatic injury: harmony and discordance of immune system homeostasis. Acute Med Surg 1, 63-69, doi:10.1002/ams2.17 (2014).
Panahi, M. et al. Immunomodulatory interventions in myocardial infarction and heart failure: a systematic review of clinical trials and meta-analysis of IL-1 inhibition. Cardiovasc Res 114, 1445-1461, doi:10.1093/cvr/cvy145 (2018).
Patenaude, J., D'Elia, M., Hamelin, C., Garrel, D. & Bernier, J. Burn injury induces a change in T cell homeostasis affecting preferentially CD4+ T cells. J Leukoc Biol 77, 141-150, doi:10.1189/jlb.0703314 (2005).
Prass, K. et al. Stroke-induced immunodeficiency promotes spontaneous bacterial infections and is mediated by sympathetic activation reversal by poststroke T helper cell type 1-like immunostimulation. J Exp Med 198, 725-736, doi:10.1084/jem.20021098 (2003).
Ramos, G., Cornistein, W., Cerino, G. T. & Nacif, G. Systemic antimicrobial prophylaxis in burn patients: systematic review. J Hosp Infect 97, 105-114, doi:10.1016/j.jhin.2017.06.015 (2017).
Ridker, P. M. et al. Antiinflammatory Therapy with Canakinumab for Atherosclerotic Disease. N Engl J Med 377, 1119-1131, doi:10.1056/NEJMoa1707914 (2017).
Robinson, R. G. & Jorge, R. E. Post-Stroke Depression: A Review. Am J Psychiatry 173, 221-231, doi:10.1176/appi.ajp.2015.15030363 (2016).
Roth, S. et al. Brain-released alarmins and stress response synergize in accelerating atherosclerosis progression after stroke. Sci Transl Med 10, doi:10.1126/scitranslmed.aao1313 (2018).
Sauaia, A., Moore, F. A. & Moore, E. E. Postinjury Inflammation and Organ Dysfunction. Crit Care Clin 33, 167-191, doi:10.1016/j.ccc.2016.08.006 (2017).
Singh, V., Sadler, R., Heindl, S., Llovera, G., Roth, S., Benakis, C., and Liesz, A. The gut microbiome primes a cerebroprotective immune response after stroke. J Cereb Blood Flow Metab 38, 1293-1298 (2018).
Strasser, A., Jost, P. J. & Nagata, S. The many roles of FAS receptor signaling in the immune system. Immunity 30, 180-192, doi:10.1016/j.immuni.2009.01.001 (2009).
Tettelin, H., Nelson, K.E., Paulsen, I.T., Eisen, J.A., Read, T.D., Peterson, S., Heidelberg, J., DeBoy, R.T., Haft, D.H., Dodson, R.J., et al. Complete genome sequence of a virulent isolate of Streptococcus pneumoniae. Science 293, 498-506 2001).
Toliver-Kinsky, T. E., Cui, W., Murphey, E. D., Lin, C. & Sherwood, E. R. Enhancement of dendritic cell production by fms-like tyrosine kinase-3 ligand increases the resistance of mice to a burn wound infection. J Immunol 174, 404-410, doi:10.4049/jimmunol.174.1.404 (2005).
Tzeng, T. C. et al. A Fluorescent Reporter Mouse for Inflammasome Assembly Demonstrates an Important Role for Cell-Bound and Free ASC Specks during In Vivo Infection. Cell Rep 16, 571-582, doi:10.1016/j.celrep.2016.06.011 (2016).
Tzeng, T. C. et al. A Fluorescent Reporter Mouse for Inflammasome Assembly Demonstrates an Important Role for Cell-Bound and Free ASC Specks during In Vivo Infection. Cell Rep 16, 571-582, doi:10.1016/j.celrep.2016.06.011 (2016).
Vanderweele, T.J., and Vansteelandt, S. Odds ratios for mediation analysis for a dichotomous outcome. Am J Epidemiol 172, 1339-1348 (2010).
Vermeij, F. H. et al. Stroke-associated infection is an independent risk factor for poor outcome after acute ischemic stroke: data from the Netherlands Stroke Survey. Cerebrovasc Dis 27, 465-471, doi:10.1159/000210093 (2009).
Vogelgesang, A. et al. Analysis of lymphocyte subsets in patients with stroke and their influence on infection after stroke. Stroke 39, 237-241, doi:10.1161/STROKEAHA.107.493635 (2008).
Westendorp, W. F. et al. The Preventive Antibiotics in Stroke Study (PASS): a pragmatic randomised open-label masked endpoint clinical trial. Lancet 385, 1519-1526, doi:10.1016/S0140-6736(14)62456-9 (2015).
Wright, D. E., Wagers, A. J., Gulati, A. P., Johnson, F. L. & Weissman, I. L. Physiological migration of hematopoietic stem and progenitor cells. Science 294, 1933-1936, doi:10.1126/science.1064081 (2001).
Wu, T., Xu, F., Su, C., Li, H., Lv, N., Liu, Y., Gao, Y., Lan, Y., and Li, J. Alterations in the Gut Microbiome and Cecal Metabolome During Klebsiella pneumoniae-Induced Pneumosepsis. Front Immunol 11, 1331 (2020).
Xu, Y. C., Luo, C. Q. & Li, X. Systemic inflammatory response syndrome following burns is mediated by brain natriuretic peptide/natriuretic peptide A receptor-induced shock factor 1 signaling pathway. Clin Exp Pharmacol Physiol 43, 921-929, doi:10.1111/1440-1681.12620 (2016).

## Claims

1. A composition comprising a DNA-degrading enzyme for use in a method of reducing the activation of a cell-free (cf) DNA sensing AIM2-inflammasome in myeloid cells of a subject in need thereof, wherein said DNA-degrading enzyme is DNAse I, and wherein the method comprises administering of DNAse I after an acute tissue injury which releases said cf DNA, wherein the acute tissue injury is an acute ischemic injury to the brain or to the heart.

2. The composition for the use of claim 1, wherein the DNAse I is administered parenterally.

3. The composition for the use of claim 1 or 2, wherein the DNAse I is administered intravenously or by inhalation.

4. The composition for the use of any one of claims 1-3, wherein the cf DNA is cf-double-stranded (ds) DNA.

5. The composition for the use of any one of claims 1-4, wherein the acute ischemic injury to the brain is stroke.

6. The composition for the use of any one of claims 1-4, wherein the acute ischemic injury to the heart is myocardial infarction.

## Patentansprüche

1. Zusammensetzung umfassend ein DNA-abbauendes Enzym zur Verwendung in einem Verfahren zum Verringern der Aktivierung eines zellfreien (zf) DNAerkennenden AIM2-Inflammasoms in myeloischen Zellen eines Subjekts, das dessen bedarf, wobei das DNA-abbauende Enzym DNAse I ist, und wobei das Verfahren das Verabreichen von DNAse I nach einer akuten Gewebeverletzung umfasst, die die zf-DNA freisetzt, wobei die akute Gewebeverletzung eine akute ischämische Verletzung des Gehirns oder des Herzens ist.

2. Zusammensetzung zur Verwendung nach Anspruch 1, wobei die DNAse I parenteral verabreicht wird.

3. Zusammensetzung zur Verwendung nach Anspruch 1 oder 2, wobei die DNAse I intravenös oder durch Inhalation verabreicht wird.

4. Zusammensetzung zur Verwendung nach einem der Ansprüche 1-3, wobei die zf-DNA zf-doppelsträngige (ds) DNA ist.

5. Zusammensetzung zur Verwendung nach einem der Ansprüche 1-4, wobei die akute ischämische Verletzung des Gehirns ein Schlaganfall ist.

6. Zusammensetzung zur Verwendung nach einem der Ansprüche 1-4, wobei die akute ischämische Verletzung des Herzens ein Myokardinfarkt ist.

## Revendications

1. Composition comprenant une enzyme dégradant l'ADN à utiliser dans un procédé de réduction de l'activation d'un inflammasome AIM2 détectant l'ADN acellulaire (cf) dans les cellules myéloïdes d'un sujet en ayant besoin, dans lequel ladite enzyme dégradant l'ADN est l'ADN I, et dans lequel le procédé comprend l'administration de DNAse I après une lésion tissulaire aiguë qui libère ledit ADN cf, dans laquelle la lésion tissulaire aiguë est une lésion ischémique aiguë au cerveau ou au coeur.

2. Composition pour l'utilisation de la revendication 1, dans laquelle DNAse I est administrée par voie parentérale.

3. Composition pour l'utilisation selon la revendication 1 ou 2, dans laquelle DNAse I est administrée par voie intraveineuse ou par inhalation.

4. Composition pour l'utilisation de l'une quelconque des revendications 1-3, dans laquelle l'ADN cf est de l'ADN cf-double brin (ds).

5. Composition pour l'utilisation de l'une quelconque des revendications 1-4, dans laquelle le La lésion ischémique aiguë du cerveau est un accident vasculaire cérébral.

6. Composition pour l'utilisation de l'une quelconque des revendications 1-4, dans laquelle le La lésion ischémique aiguë du coeur est un infarctus du myocarde.
